# EUROPEAN PATENT APPLICATION

(11) **EP 3 730 617 A1**
(43) Date of publication of application: **28.10.2020**
(21) Application number: 19171455.9
(22) Date of filing: 26.04.2019
(51) Int. Cl.: C12N 15/113

(54) **NUCLEIC ACIDS FOR INHIBITING EXPRESSION OF C3 IN A CELL**

(71) Applicant: Silence Therapeutics GmbH, 13125 Berlin (DE)
(72) Inventor: Verena, Aumiller, 10247 Berlin (DE); Dames, Sibylle, 12435 Berlin (DE); Schubert, Steffen, 14199 Berlin (DE); Hauptmann, Judith, 13125 Berlin (DE); Frauendorf, Christian, 13127 Berlin (DE); Wikström Linhholm, Marie, 211 45 Malmö (SE); Weingärtner, Adrien, 10318 Berlin (DE); Bethge, Lucas, 14482 Potsdam (DE)
(74) Representative: Mewburn Ellis LLP

(57) **Abstract**

The invention relates to nucleic acid products that interfere with complement component C3 gene expression or inhibit its expression. The nucleic acids are preferably for use as treatment, prevention or reduction of risk of suffering from complement component C3 associated diseases, disorders or syndromes, particularly C3 Glomerulopathy (C3G), Paroxysmal Nocturnal Hemoglobinuria (PNH), atypical Hemolytic Uremic Syndrome (aHUS), Lupus nephritis, IgA nephropathy (IgAN), Cold Agglutinin Disease (CAD), Myasthenia gravis (MG), and Primary Membranous Nephropathy.

SEQUENCE LISTING

<110>  Silence Therapeutics GmbH

<120>  Nucleic acids for inhibiting expression of complement component
       C3

<130>  S114EP3

<160>  361

<170>  PatentIn version 3.5

<210>  1
<211>  19
<212>  RNA
<213>  Artificial Sequence

<220>
<223>  siRNA

<400>  1
uuucauagua ggcucggau                                                    19


<210>  2
<211>  19
<212>  RNA
<213>  Artificial Sequence

<220>
<223>  siRNA

<400>  2
auccgagccu acuaugaaa                                                    19


<210>  3
<211>  19
<212>  RNA
<213>  Artificial Sequence

<220>
<223>  siRNA

<400>  3
uuucucugua ggcuccacu                                                    19


<210>  4
<211>  19
<212>  RNA
<213>  Artificial Sequence

<220>
<223>  siRNA

<400>  4
aguggagccu acagagaaa                                                    19


<210>  5
<211>  19

<212>  RNA
<213>  Artificial Sequence

<220>
<223>  siRNA

<400>  5
uuauagaugu aguagaauu                                                    19


<210>  6
<211>  19
<212>  RNA
<213>  Artificial Sequence

<220>
<223>  siRNA

<400>  6
aauucuacua caucuauaa                                                    19


<210>  7
<211>  19
<212>  RNA
<213>  Artificial Sequence

<220>
<223>  siRNA

<400>  7
augacaaagg caguucccu                                                    19


<210>  8
<211>  19
<212>  RNA
<213>  Artificial Sequence

<220>
<223>  siRNA

<400>  8
agggaacugc cuuugucau                                                    19


<210>  9
<211>  19
<212>  RNA
<213>  Artificial Sequence

<220>
<223>  siRNA

<400>  9
aucugguagg gagagguca                                                    19


<210>  10
<211>  19
<212>  RNA
<213>  Artificial Sequence

<220>
<223>  siRNA

<400>  10
ugaccucucc cuaccagau                                                    19


<210>  11
<211>  19
<212>  RNA
<213>  Artificial Sequence

<220>
<223>  siRNA

<400>  11
uguguguuga ugcugaguu                                                    19


<210>  12
<211>  19
<212>  RNA
<213>  Artificial Sequence

<220>
<223>  siRNA

<400>  12
aacucagcau caacacaca                                                    19


<210>  13
<211>  19
<212>  RNA
<213>  Artificial Sequence

<220>
<223>  siRNA

<400>  13
uaauuguugg aguugccca                                                    19


<210>  14
<211>  19
<212>  RNA
<213>  Artificial Sequence

<220>
<223>  siRNA

<400>  14
ugggcaacuc caacaauua                                                    19


<210>  15
<211>  19
<212>  RNA
<213>  Artificial Sequence

<220>
<223>  siRNA

<400>  15
aggaaguuga cguugaggg                                                    19


<210>  16
<211>  19
<212>  RNA
<213>  Artificial Sequence

<220>
<223>  siRNA

<400>  16
cccucaacgu caacuuccu                                                    19


<210>  17
<211>  19
<212>  RNA
<213>  Artificial Sequence

<220>
<223>  siRNA

<400>  17
augaagucgg uggugaugg                                                    19


<210>  18
<211>  19
<212>  RNA
<213>  Artificial Sequence

<220>
<223>  siRNA

<400>  18
ccaucaccac cgacuucau                                                    19


<210>  19
<211>  19
<212>  RNA
<213>  Artificial Sequence

<220>
<223>  siRNA

<400>  19
uuuaccacca gcgagccca                                                    19


<210>  20
<211>  19
<212>  RNA
<213>  Artificial Sequence

<220>
<223>  siRNA

<400>  20
ugggcucgcu ggugguaaa                                                    19

<210>  21
<211>  19
<212>  RNA
<213>  Artificial Sequence

<220>
<223>  siRNA

<400>  21
ucuaucuuca gggucaucu                                                    19


<210>  22
<211>  19
<212>  RNA
<213>  Artificial Sequence

<220>
<223>  siRNA

<400>  22
agaugacccu gaagauaga                                                    19


<210>  23
<211>  19
<212>  RNA
<213>  Artificial Sequence

<220>
<223>  siRNA

<400>  23
auguaguugc agcagucca                                                    19


<210>  24
<211>  19
<212>  RNA
<213>  Artificial Sequence

<220>
<223>  siRNA

<400>  24
uggacugcug caacuacau                                                    19


<210>  25
<211>  19
<212>  RNA
<213>  Artificial Sequence

<220>
<223>  siRNA

<400>  25
aauauauuca ugagcuucg                                                    19


<210>  26
<211>  19

<212>  RNA
<213>  Artificial Sequence

<220>
<223>  siRNA

<400>  26
cgaagcucau gaauauauu                                                    19


<210>  27
<211>  19
<212>  RNA
<213>  Artificial Sequence

<220>
<223>  siRNA

<400>  27
aaauauauuc augagcuuc                                                    19


<210>  28
<211>  19
<212>  RNA
<213>  Artificial Sequence

<220>
<223>  siRNA

<400>  28
gaagcucaug aauauauuu                                                    19


<210>  29
<211>  19
<212>  RNA
<213>  Artificial Sequence

<220>
<223>  siRNA

<400>  29
uccugcauua cugugaccu                                                    19


<210>  30
<211>  19
<212>  RNA
<213>  Artificial Sequence

<220>
<223>  siRNA

<400>  30
aggucacagu aaugcagga                                                    19


<210>  31
<211>  19
<212>  RNA
<213>  Artificial Sequence

<220>
<223>  siRNA

<400>  31
caacagagua ggguagccg                                                    19


<210>  32
<211>  19
<212>  RNA
<213>  Artificial Sequence

<220>
<223>  siRNA

<400>  32
cggcuacccu acucuguug                                                    19


<210>  33
<211>  19
<212>  RNA
<213>  Artificial Sequence

<220>
<223>  siRNA

<400>  33
ucgaacaaca gaguagggu                                                    19


<210>  34
<211>  19
<212>  RNA
<213>  Artificial Sequence

<220>
<223>  siRNA

<400>  34
acccuacucu guuguucga                                                    19


<210>  35
<211>  19
<212>  RNA
<213>  Artificial Sequence

<220>
<223>  siRNA

<400>  35
uuucgaacaa cagaguagg                                                    19


<210>  36
<211>  19
<212>  RNA
<213>  Artificial Sequence

<220>
<223>  siRNA

<400>  36
ccuacucugu uguucgaaa                                                    19


<210>  37
<211>  19
<212>  RNA
<213>  Artificial Sequence

<220>
<223>  siRNA

<400>  37
guuucaucca gguaaugca                                                    19


<210>  38
<211>  19
<212>  RNA
<213>  Artificial Sequence

<220>
<223>  siRNA

<400>  38
ugcauuaccu ggaugaaac                                                    19


<210>  39
<211>  19
<212>  RNA
<213>  Artificial Sequence

<220>
<223>  siRNA

<400>  39
uuaucuuugg cugugguca                                                    19


<210>  40
<211>  19
<212>  RNA
<213>  Artificial Sequence

<220>
<223>  siRNA

<400>  40
ugaccacagc caaagauaa                                                    19


<210>  41
<211>  19
<212>  RNA
<213>  Artificial Sequence

<220>
<223>  siRNA

<400>  41
uguucauuga gccaacgca                                                    19

<210>  42
<211>  19
<212>  RNA
<213>  Artificial Sequence

<220>
<223>  siRNA

<400>  42
ugcguuggcu caaugaaca                                                    19


<210>  43
<211>  19
<212>  RNA
<213>  Artificial Sequence

<220>
<223>  siRNA

<400>  43
uugguauuga gccaaggcu                                                    19


<210>  44
<211>  19
<212>  RNA
<213>  Artificial Sequence

<220>
<223>  siRNA

<400>  44
agccuuggcu caauaccaa                                                    19


<210>  45
<211>  19
<212>  RNA
<213>  Artificial Sequence

<220>
<223>  siRNA

<400>  45
uuuauuacag gugaguuga                                                    19


<210>  46
<211>  19
<212>  RNA
<213>  Artificial Sequence

<220>
<223>  siRNA

<400>  46
ucaacucacc uguaauaaa                                                    19


<210>  47
<211>  19

<212>  RNA
<213>  Artificial Sequence

<220>
<223>  siRNA

<400>  47
uuucuguuuc cggugcugg                                                    19


<210>  48
<211>  19
<212>  RNA
<213>  Artificial Sequence

<220>
<223>  siRNA

<400>  48
ccagcaccgg aaacagaaa                                                    19


<210>  49
<211>  19
<212>  RNA
<213>  Artificial Sequence

<220>
<223>  siRNA

<400>  49
ucaaggauca uaguguucu                                                    19


<210>  50
<211>  19
<212>  RNA
<213>  Artificial Sequence

<220>
<223>  siRNA

<400>  50
agaacacuau gauccuuga                                                    19


<210>  51
<211>  19
<212>  RNA
<213>  Artificial Sequence

<220>
<223>  siRNA

<400>  51
aucucaagga ucauagugu                                                    19


<210>  52
<211>  19
<212>  RNA
<213>  Artificial Sequence

<220>
<223>  siRNA

<400>  52
acacuaugau ccuugagau                                                    19


<210>  53
<211>  19
<212>  RNA
<213>  Artificial Sequence

<220>
<223>  siRNA

<400>  53
ucauacuugg agauguauc                                                    19


<210>  54
<211>  19
<212>  RNA
<213>  Artificial Sequence

<220>
<223>  siRNA

<400>  54
gauacaucuc caaguauga                                                    19


<210>  55
<211>  19
<212>  RNA
<213>  Artificial Sequence

<220>
<223>  siRNA

<400>  55
uaucggagaa ggcuuuguc                                                    19


<210>  56
<211>  19
<212>  RNA
<213>  Artificial Sequence

<220>
<223>  siRNA

<400>  56
gacaaagccu ucuccgaua                                                    19


<210>  57
<211>  19
<212>  RNA
<213>  Artificial Sequence

<220>
<223>  siRNA

<400>  57
augaugaggg uguuccuau                                                    19


<210>  58
<211>  19
<212>  RNA
<213>  Artificial Sequence

<220>
<223>  siRNA

<400>  58
auaggaacac ccucaucau                                                    19


<210>  59
<211>  19
<212>  RNA
<213>  Artificial Sequence

<220>
<223>  siRNA

<400>  59
uauuggugaa cuuugaaag                                                    19


<210>  60
<211>  19
<212>  RNA
<213>  Artificial Sequence

<220>
<223>  siRNA

<400>  60
cuuucaaagu ucaccaaua                                                    19


<210>  61
<211>  19
<212>  RNA
<213>  Artificial Sequence

<220>
<223>  siRNA

<400>  61
agcuuguuca gcuuuccau                                                    19


<210>  62
<211>  19
<212>  RNA
<213>  Artificial Sequence

<220>
<223>  siRNA

<400>  62
auggaaagcu gaacaagcu                                                    19

<210>  63
<211>  19
<212>  RNA
<213>  Artificial Sequence

<220>
<223>  siRNA

<400>  63
uuuguaugaa gcaauucuc                                                    19


<210>  64
<211>  19
<212>  RNA
<213>  Artificial Sequence

<220>
<223>  siRNA

<400>  64
gagaauugcu ucauacaaa                                                    19


<210>  65
<211>  19
<212>  RNA
<213>  Artificial Sequence

<220>
<223>  siRNA

<400>  65
gucuuguaca cauagucca                                                    19


<210>  66
<211>  19
<212>  RNA
<213>  Artificial Sequence

<220>
<223>  siRNA

<400>  66
uggacuaugu guacaagac                                                    19


<210>  67
<211>  19
<212>  RNA
<213>  Artificial Sequence

<220>
<223>  siRNA

<400>  67
ugcucaaugg ccaugaugu                                                    19


<210>  68
<211>  19

<212>  RNA
<213>  Artificial Sequence

<220>
<223>  siRNA

<400>  68
acaucauggc cauugagca                                                    19


<210>  69
<211>  19
<212>  RNA
<213>  Artificial Sequence

<220>
<223>  siRNA

<400>  69
uuggcauucg uccuccucg                                                    19


<210>  70
<211>  19
<212>  RNA
<213>  Artificial Sequence

<220>
<223>  siRNA

<400>  70
cgaggaggac gaaugccaa                                                    19


<210>  71
<211>  19
<212>  RNA
<213>  Artificial Sequence

<220>
<223>  siRNA

<400>  71
gucuggauga agagguacc                                                    19


<210>  72
<211>  19
<212>  RNA
<213>  Artificial Sequence

<220>
<223>  siRNA

<400>  72
gguaccucuu cauccagac                                                    19


<210>  73
<211>  19
<212>  RNA
<213>  Artificial Sequence

<220>
<223>  siRNA

<400>  73
ugucuggaug aagagguac                                                    19


<210>  74
<211>  19
<212>  RNA
<213>  Artificial Sequence

<220>
<223>  siRNA

<400>  74
guaccucuuc auccagaca                                                    19


<210>  75
<211>  19
<212>  RNA
<213>  Artificial Sequence

<220>
<223>  siRNA

<400>  75
ucugucugga ugaagaggu                                                    19


<210>  76
<211>  19
<212>  RNA
<213>  Artificial Sequence

<220>
<223>  siRNA

<400>  76
accucuucau ccagacaga                                                    19


<210>  77
<211>  19
<212>  RNA
<213>  Artificial Sequence

<220>
<223>  siRNA

<400>  77
cuugucuguc uggaugaag                                                    19


<210>  78
<211>  19
<212>  RNA
<213>  Artificial Sequence

<220>
<223>  siRNA

<400>  78
cuucauccag acagacaag                                                    19


<210>  79
<211>  19
<212>  RNA
<213>  Artificial Sequence

<220>
<223>  siRNA

<400>  79
auggucuugu cugucugga                                                    19


<210>  80
<211>  19
<212>  RNA
<213>  Artificial Sequence

<220>
<223>  siRNA

<400>  80
uccagacaga caagaccau                                                    19


<210>  81
<211>  19
<212>  RNA
<213>  Artificial Sequence

<220>
<223>  siRNA

<400>  81
agauggucuu gucugucug                                                    19


<210>  82
<211>  19
<212>  RNA
<213>  Artificial Sequence

<220>
<223>  siRNA

<400>  82
cagacagaca agaccaucu                                                    19


<210>  83
<211>  19
<212>  RNA
<213>  Artificial Sequence

<220>
<223>  siRNA

<400>  83
guagaugguc uugucuguc                                                    19

<210>  84
<211>  19
<212>  RNA
<213>  Artificial Sequence

<220>
<223>  siRNA

<400>  84
gacagacaag accaucuac                                                    19


<210>  85
<211>  19
<212>  RNA
<213>  Artificial Sequence

<220>
<223>  siRNA

<400>  85
guguagaugg ucuugucug                                                    19


<210>  86
<211>  19
<212>  RNA
<213>  Artificial Sequence

<220>
<223>  siRNA

<400>  86
cagacaagac caucuacac                                                    19


<210>  87
<211>  19
<212>  RNA
<213>  Artificial Sequence

<220>
<223>  siRNA

<400>  87
gggguguaga uggucuugu                                                    19


<210>  88
<211>  19
<212>  RNA
<213>  Artificial Sequence

<220>
<223>  siRNA

<400>  88
acaagaccau cuacacccc                                                    19


<210>  89
<211>  19

<212>  RNA
<213>  Artificial Sequence

<220>
<223>  siRNA

<400>  89
uaggcucgga ucuuccacu                                                    19


<210>  90
<211>  19
<212>  RNA
<213>  Artificial Sequence

<220>
<223>  siRNA

<400>  90
aguggaagau ccgagccua                                                    19


<210>  91
<211>  19
<212>  RNA
<213>  Artificial Sequence

<220>
<223>  siRNA

<400>  91
cucgaaacug ggcagcacg                                                    19


<210>  92
<211>  19
<212>  RNA
<213>  Artificial Sequence

<220>
<223>  siRNA

<400>  92
cgugcugccc aguuucgag                                                    19


<210>  93
<211>  19
<212>  RNA
<213>  Artificial Sequence

<220>
<223>  siRNA

<400>  93
uuggugaagu ggaucuggu                                                    19


<210>  94
<211>  19
<212>  RNA
<213>  Artificial Sequence

<220>
<223>  siRNA

<400>  94
accagaucca cuucaccaa                                                    19


<210>  95
<211>  19
<212>  RNA
<213>  Artificial Sequence

<220>
<223>  siRNA

<400>  95
ucuuggugaa guggaucug                                                    19


<210>  96
<211>  19
<212>  RNA
<213>  Artificial Sequence

<220>
<223>  siRNA

<400>  96
cagauccacu ucaccaaga                                                    19


<210>  97
<211>  19
<212>  RNA
<213>  Artificial Sequence

<220>
<223>  siRNA

<400>  97
gucuugguga aguggaucu                                                    19


<210>  98
<211>  19
<212>  RNA
<213>  Artificial Sequence

<220>
<223>  siRNA

<400>  98
agauccacuu caccaagac                                                    19


<210>  99
<211>  19
<212>  RNA
<213>  Artificial Sequence

<220>
<223>  siRNA

<400>  99
ggugucuugg ugaagugga                                                    19


<210>  100
<211>  19
<212>  RNA
<213>  Artificial Sequence

<220>
<223>  siRNA

<400>  100
uccacuucac caagacacc                                                    19


<210>  101
<211>  19
<212>  RNA
<213>  Artificial Sequence

<220>
<223>  siRNA

<400>  101
aacaccauga ggucaaagg                                                    19


<210>  102
<211>  19
<212>  RNA
<213>  Artificial Sequence

<220>
<223>  siRNA

<400>  102
   ccuuugaccu cauggiguu                                                    19


<210>  103
<211>  19
<212>  RNA
<213>  Artificial Sequence

<220>
<223>  siRNA

<400>  103
gaacaccaug aggucaaag                                                    19


<210>  104
<211>  19
<212>  RNA
<213>  Artificial Sequence

<220>
<223>  siRNA

<400>  104
cuuugaccuc augguguuc                                                    19

<210>  105
<211>  19
<212>  RNA
<213>  Artificial Sequence

<220>
<223>  siRNA

<400>  105
gucacgaaca ccaugaggu                                                    19


<210>  106
<211>  19
<212>  RNA
<213>  Artificial Sequence

<220>
<223>  siRNA

<400>  106
accucauggu guucgugac                                                    19


<210>  107
<211>  19
<212>  RNA
<213>  Artificial Sequence

<220>
<223>  siRNA

<400>  107
acacagaucc cuuucuugu                                                    19


<210>  108
<211>  19
<212>  RNA
<213>  Artificial Sequence

<220>
<223>  siRNA

<400>  108
acaagaaagg gaucugugu                                                    19


<210>  109
<211>  19
<212>  RNA
<213>  Artificial Sequence

<220>
<223>  siRNA

<400>  109
ccuugcagga gaauucugg                                                    19


<210>  110
<211>  19

<212>  RNA
<213>  Artificial Sequence

<220>
<223>  siRNA

<400>  110
ccagaauucu ccugcaagg                                                    19


<210>  111
<211>  19
<212>  RNA
<213>  Artificial Sequence

<220>
<223>  siRNA

<400>  111
agagagaaga ccuugacca                                                    19


<210>  112
<211>  19
<212>  RNA
<213>  Artificial Sequence

<220>
<223>  siRNA

<400>  112
uggucaaggu cuucucucu                                                    19


<210>  113
<211>  19
<212>  RNA
<213>  Artificial Sequence

<220>
<223>  siRNA

<400>  113
gagucgaugg cgaugaggu                                                    19


<210>  114
<211>  19
<212>  RNA
<213>  Artificial Sequence

<220>
<223>  siRNA

<400>  114
accucaucgc caucgacuc                                                    19


<210>  115
<211>  19
<212>  RNA
<213>  Artificial Sequence

<220>
<223>  siRNA

<400>  115
gcuuggaaca ccaugaagg                                                    19


<210>  116
<211>  19
<212>  RNA
<213>  Artificial Sequence

<220>
<223>  siRNA

<400>  116
ccuucauggu guuccaagc                                                    19


<210>  117
<211>  19
<212>  RNA
<213>  Artificial Sequence

<220>
<223>  siRNA

<400>  117
ucauuuuccu uggucucuu                                                    19


<210>  118
<211>  19
<212>  RNA
<213>  Artificial Sequence

<220>
<223>  siRNA

<400>  118
aagagaccaa ggaaaauga                                                    19


<210>  119
<211>  19
<212>  RNA
<213>  Artificial Sequence

<220>
<223>  siRNA

<400>  119
ugugucugga gcaaagcca                                                    19


<210>  120
<211>  19
<212>  RNA
<213>  Artificial Sequence

<220>
<223>  siRNA

<400>  120
uggcuuugcu ccagacaca                                                    19


<210>  121
<211>  19
<212>  RNA
<213>  Artificial Sequence

<220>
<223>  siRNA

<400>  121
agguagauga ugagggugu                                                    19


<210>  122
<211>  19
<212>  RNA
<213>  Artificial Sequence

<220>
<223>  siRNA

<400>  122
acacccucau caucuaccu                                                    19


<210>  123
<211>  19
<212>  RNA
<213>  Artificial Sequence

<220>
<223>  siRNA

<400>  123
uuguaauagg cguagaccu                                                    19


<210>  124
<211>  19
<212>  RNA
<213>  Artificial Sequence

<220>
<223>  siRNA

<400>  124
aggucuacgc cuauuacaa                                                    19


<210>  125
<211>  19
<212>  RNA
<213>  Artificial Sequence

<220>
<223>  siRNA

<400>  125
guuguaauag gcguagacc                                                    19

<210>  126
<211>  19
<212>  RNA
<213>  Artificial Sequence

<220>
<223>  siRNA

<400>  126
ggucuacgcc uauuacaac                                                    19


<210>  127
<211>  19
<212>  RNA
<213>  Artificial Sequence

<220>
<223>  siRNA

<400>  127
gggucuugua cacauaguc                                                    19


<210>  128
<211>  19
<212>  RNA
<213>  Artificial Sequence

<220>
<223>  siRNA

<400>  128
gacuaugugu acaagaccc                                                    19


<210>  129
<211>  19
<212>  RNA
<213>  Artificial Sequence

<220>
<223>  siRNA

<400>  129
cacuugaugg ggcugauga                                                    19


<210>  130
<211>  19
<212>  RNA
<213>  Artificial Sequence

<220>
<223>  siRNA

<400>  130
ucaucagccc caucaagug                                                    19


<210>  131
<211>  19

<212>  RNA
<213>  Artificial Sequence

<220>
<223>  siRNA

<400>  131
auuguaauag gcguagacc                                                    19


<210>  132
<211>  19
<212>  RNA
<213>  Artificial Sequence

<220>
<223>  siRNA

<400>  132
ggucuacgcc uauuacaau                                                    19


<210>  133
<211>  19
<212>  RNA
<213>  Artificial Sequence

<220>
<223>  siRNA

<400>  133
auguagaugg ucuugucug                                                    19


<210>  134
<211>  19
<212>  RNA
<213>  Artificial Sequence

<220>
<223>  siRNA

<400>  134
cagacaagac caucuacau                                                    19


<210>  135
<211>  19
<212>  RNA
<213>  Artificial Sequence

<220>
<223>  siRNA

<400>  135
gguaccucuu cauccagaa                                                    19


<210>  136
<211>  19
<212>  RNA
<213>  Artificial Sequence

<220>
<223>  siRNA

<400>  136
cuucauccag acagacaaa                                                    19


<210>  137
<211>  19
<212>  RNA
<213>  Artificial Sequence

<220>
<223>  siRNA

<400>  137
uccagacaga caagaccaa                                                    19


<210>  138
<211>  19
<212>  RNA
<213>  Artificial Sequence

<220>
<223>  siRNA

<400>  138
cagacagaca agaccauca                                                    19


<210>  139
<211>  19
<212>  RNA
<213>  Artificial Sequence

<220>
<223>  siRNA

<400>  139
gacagacaag accaucuaa                                                    19


<210>  140
<211>  19
<212>  RNA
<213>  Artificial Sequence

<220>
<223>  siRNA

<400>  140
cagacaagac caucuacaa                                                    19


<210>  141
<211>  19
<212>  RNA
<213>  Artificial Sequence

<220>
<223>  siRNA

<400>  141
acaagaccau cuacaccca                                                    19


<210>  142
<211>  19
<212>  RNA
<213>  Artificial Sequence

<220>
<223>  siRNA

<400>  142
cgugcugccc aguuucgaa                                                    19


<210>  143
<211>  19
<212>  RNA
<213>  Artificial Sequence

<220>
<223>  siRNA

<400>  143
agauccacuu caccaagaa                                                    19


<210>  144
<211>  19
<212>  RNA
<213>  Artificial Sequence

<220>
<223>  siRNA

<400>  144
uccacuucac caagacaca                                                    19


<210>  145
<211>  19
<212>  RNA
<213>  Artificial Sequence

<220>
<223>  siRNA

<400>  145
ccuuugaccu cauggugua                                                    19


<210>  146
<211>  19
<212>  RNA
<213>  Artificial Sequence

<220>
<223>  siRNA

<400>  146
cuuugaccuc augguguua                                                    19

<210>  147
<211>  19
<212>  RNA
<213>  Artificial Sequence

<220>
<223>  siRNA

<400>  147
accucauggu guucgugaa                                                    19


<210>  148
<211>  19
<212>  RNA
<213>  Artificial Sequence

<220>
<223>  siRNA

<400>  148
acaagaaagg gaucuguga                                                    19


<210>  149
<211>  19
<212>  RNA
<213>  Artificial Sequence

<220>
<223>  siRNA

<400>  149
agggaucugu guggcagaa                                                    19


<210>  150
<211>  19
<212>  RNA
<213>  Artificial Sequence

<220>
<223>  siRNA

<400>  150
aaugcaggac uucuucaua                                                    19


<210>  151
<211>  19
<212>  RNA
<213>  Artificial Sequence

<220>
<223>  siRNA

<400>  151
ccagaauucu ccugcaaga                                                    19


<210>  152
<211>  19

<212>  RNA
<213>  Artificial Sequence

<220>
<223>  siRNA

<400>  152
uggucaaggu cuucucuca                                                    19


<210>  153
<211>  19
<212>  RNA
<213>  Artificial Sequence

<220>
<223>  siRNA

<400>  153
accucaucgc caucgacua                                                    19


<210>  154
<211>  19
<212>  RNA
<213>  Artificial Sequence

<220>
<223>  siRNA

<400>  154
ccuucauggu guuccaaga                                                    19


<210>  155
<211>  19
<212>  RNA
<213>  Artificial Sequence

<220>
<223>  siRNA

<400>  155
acacccucau caucuacca                                                    19


<210>  156
<211>  19
<212>  RNA
<213>  Artificial Sequence

<220>
<223>  siRNA

<400>  156
ggucuacgcc uauuacaaa                                                    19


<210>  157
<211>  19
<212>  RNA
<213>  Artificial Sequence

<220>
<223>  siRNA

<400>  157
gacuaugugu acaagacca                                                    19


<210>  158
<211>  19
<212>  RNA
<213>  Artificial Sequence

<220>
<223>  siRNA

<400>  158
ucaucagccc caucaagua                                                    19


<210>  159
<211>  19
<212>  RNA
<213>  Artificial Sequence

<220>
<223>  siRNA - modified_base, modified as per summary sequence table at
       the end of the description

<400>  159
uuucauagua ggcucggau                                                    19


<210>  160
<211>  19
<212>  RNA
<213>  Artificial Sequence

<220>
<223>  siRNA - modified_base, modified as per summary sequence table at
       the end of the description

<400>  160
auccgagccu acuaugaaa                                                    19


<210>  161
<211>  19
<212>  RNA
<213>  Artificial Sequence

<220>
<223>  siRNA - modified_base, modified as per summary sequence table at
       the end of the description

<400>  161
uuucucugua ggcuccacu                                                    19


<210>  162
<211>  19
<212>  RNA
<213>  Artificial Sequence

<220>
<223>  siRNA - modified_base, modified as per summary sequence table at
      the end of the description

<400>  162
aguggagccu acagagaaa                                                    19


<210>  163
<211>  19
<212>  RNA
<213>  Artificial Sequence

<220>
<223>  siRNA - modified_base, modified as per summary sequence table at
      the end of the description

<400>  163
uuauagaugu aguagaauu                                                    19


<210>  164
<211>  19
<212>  RNA
<213>  Artificial Sequence

<220>
<223>  siRNA - modified_base, modified as per summary sequence table at
      the end of the description

<400>  164
aauucuacua caucuauaa                                                    19


<210>  165
<211>  19
<212>  RNA
<213>  Artificial Sequence

<220>
<223>  siRNA - modified_base, modified as per summary sequence table at
      the end of the description

<400>  165
augacaaagg caguucccu                                                    19


<210>  166
<211>  19
<212>  RNA
<213>  Artificial Sequence

<220>
<223>  siRNA - modified_base, modified as per summary sequence table at
      the end of the description

<400>  166
agggaacugc cuuugucau                                                    19


<210>  167
<211>  19
<212>  RNA

<213>  Artificial Sequence

<220>
<223>  siRNA - modified_base, modified as per summary sequence table at
       the end of the description

<400>  167
aucugguagg gagagguca                                                    19


<210>  168
<211>  19
<212>  RNA
<213>  Artificial Sequence

<220>
<223>  siRNA - modified_base, modified as per summary sequence table at
       the end of the description

<400>  168
ugaccucucc cuaccagau                                                    19


<210>  169
<211>  19
<212>  RNA
<213>  Artificial Sequence

<220>
<223>  siRNA - modified_base, modified as per summary sequence table at
       the end of the description

<400>  169
uguguguuga ugcugaguu                                                    19


<210>  170
<211>  19
<212>  RNA
<213>  Artificial Sequence

<220>
<223>  siRNA - modified_base, modified as per summary sequence table at
       the end of the description

<400>  170
aacucagcau caacacaca                                                    19


<210>  171
<211>  19
<212>  RNA
<213>  Artificial Sequence

<220>
<223>  siRNA - modified_base, modified as per summary sequence table at
       the end of the description

<400>  171
uaauuguugg aguugccca                                                    19


<210>  172

<211>  19
<212>  RNA
<213>  Artificial Sequence

<220>
<223>  siRNA - modified_base, modified as per summary sequence table at
       the end of the description

<400>  172
ugggcaacuc caacaauua                                                    19


<210>  173
<211>  19
<212>  RNA
<213>  Artificial Sequence

<220>
<223>  siRNA - modified_base, modified as per summary sequence table at
       the end of the description

<400>  173
aggaaguuga cguugaggg                                                    19


<210>  174
<211>  19
<212>  RNA
<213>  Artificial Sequence

<220>
<223>  siRNA - modified_base, modified as per summary sequence table at
       the end of the description

<400>  174
cccucaacgu caacuuccu                                                    19


<210>  175
<211>  19
<212>  RNA
<213>  Artificial Sequence

<220>
<223>  siRNA - modified_base, modified as per summary sequence table at
       the end of the description

<400>  175
augaagucgg uggugaugg                                                    19


<210>  176
<211>  19
<212>  RNA
<213>  Artificial Sequence

<220>
<223>  siRNA - modified_base, modified as per summary sequence table at
       the end of the description

<400>  176
ccaucaccac cgacuucau                                                    19

<210>  177
<211>  19
<212>  RNA
<213>  Artificial Sequence

<220>
<223>  siRNA - modified_base, modified as per summary sequence table at
       the end of the description

<400>  177
uuuaccacca gcgagccca                                                    19


<210>  178
<211>  19
<212>  RNA
<213>  Artificial Sequence

<220>
<223>  siRNA - modified_base, modified as per summary sequence table at
       the end of the description

<400>  178
ugggcucgcu ggugguaaa                                                    19


<210>  179
<211>  19
<212>  RNA
<213>  Artificial Sequence

<220>
<223>  siRNA - modified_base, modified as per summary sequence table at
       the end of the description

<400>  179
ucuaucuuca gggucaucu                                                    19


<210>  180
<211>  19
<212>  RNA
<213>  Artificial Sequence

<220>
<223>  siRNA - modified_base, modified as per summary sequence table at
       the end of the description

<400>  180
agaugacccu gaagauaga                                                    19


<210>  181
<211>  19
<212>  RNA
<213>  Artificial Sequence

<220>
<223>  siRNA - modified_base, modified as per summary sequence table at
       the end of the description

<400>  181

auguaguugc agcagucca                                                    19


<210>  182
<211>  19
<212>  RNA
<213>  Artificial Sequence

<220>
<223>  siRNA - modified_base, modified as per summary sequence table at
       the end of the description

<400>  182
uggacugcug caacuacau                                                    19


<210>  183
<211>  19
<212>  RNA
<213>  Artificial Sequence

<220>
<223>  siRNA - modified_base, modified as per summary sequence table at
       the end of the description

<400>  183
aauauauuca ugagcuucg                                                    19


<210>  184
<211>  19
<212>  RNA
<213>  Artificial Sequence

<220>
<223>  siRNA - modified_base, modified as per summary sequence table at
       the end of the description

<400>  184
cgaagcucau gaauauauu                                                    19


<210>  185
<211>  19
<212>  RNA
<213>  Artificial Sequence

<220>
<223>  siRNA - modified_base, modified as per summary sequence table at
       the end of the description

<400>  185
aaauauauuc augagcuuc                                                    19


<210>  186
<211>  19
<212>  RNA
<213>  Artificial Sequence

<220>
<223>  siRNA - modified_base, modified as per summary sequence table at
       the end of the description

<400>  186
gaagcucaug aauauauuu                                                    19


<210>  187
<211>  19
<212>  RNA
<213>  Artificial Sequence

<220>
<223>  siRNA - modified_base, modified as per summary sequence table at
       the end of the description

<400>  187
uccugcauua cugugaccu                                                    19


<210>  188
<211>  19
<212>  RNA
<213>  Artificial Sequence

<220>
<223>  siRNA - modified_base, modified as per summary sequence table at
       the end of the description

<400>  188
aggucacagu aaugcagga                                                    19


<210>  189
<211>  19
<212>  RNA
<213>  Artificial Sequence

<220>
<223>  siRNA - modified_base, modified as per summary sequence table at
       the end of the description

<400>  189
caacagagua ggguagccg                                                    19


<210>  190
<211>  19
<212>  RNA
<213>  Artificial Sequence

<220>
<223>  siRNA - modified_base, modified as per summary sequence table at
       the end of the description

<400>  190
cggcuacccu acucuguug                                                    19


<210>  191
<211>  19
<212>  RNA
<213>  Artificial Sequence

<220>

<223>  siRNA - modified_base, modified as per summary sequence table at
       the end of the description

<400>  191
ucgaacaaca gaguagggu                                                    19


<210>  192
<211>  19
<212>  RNA
<213>  Artificial Sequence

<220>
<223>  siRNA - modified_base, modified as per summary sequence table at
       the end of the description

<400>  192
acccuacucu guuguucga                                                    19


<210>  193
<211>  19
<212>  RNA
<213>  Artificial Sequence

<220>
<223>  siRNA - modified_base, modified as per summary sequence table at
       the end of the description

<400>  193
uuucgaacaa cagaguagg                                                    19


<210>  194
<211>  19
<212>  RNA
<213>  Artificial Sequence

<220>
<223>  siRNA - modified_base, modified as per summary sequence table at
       the end of the description

<400>  194
ccuacucugu uguucgaaa                                                    19


<210>  195
<211>  19
<212>  RNA
<213>  Artificial Sequence

<220>
<223>  siRNA - modified_base, modified as per summary sequence table at
       the end of the description

<400>  195
guuucaucca gguaaugca                                                    19


<210>  196
<211>  19
<212>  RNA
<213>  Artificial Sequence

<220>
<223>  siRNA - modified_base, modified as per summary sequence table at
      the end of the description

<400>  196
ugcauuaccu ggaugaaac                                                    19


<210>  197
<211>  19
<212>  RNA
<213>  Artificial Sequence

<220>
<223>  siRNA - modified_base, modified as per summary sequence table at
      the end of the description

<400>  197
uuaucuuugg cugugguca                                                    19


<210>  198
<211>  19
<212>  RNA
<213>  Artificial Sequence

<220>
<223>  siRNA - modified_base, modified as per summary sequence table at
      the end of the description

<400>  198
ugaccacagc caaagauaa                                                    19


<210>  199
<211>  19
<212>  RNA
<213>  Artificial Sequence

<220>
<223>  siRNA - modified_base, modified as per summary sequence table at
      the end of the description

<400>  199
uguucauuga gccaacgca                                                    19


<210>  200
<211>  19
<212>  RNA
<213>  Artificial Sequence

<220>
<223>  siRNA - modified_base, modified as per summary sequence table at
      the end of the description

<400>  200
ugcguuggcu caaugaaca                                                    19


<210>  201
<211>  19

<212>  RNA
<213>  Artificial Sequence

<220>
<223>  siRNA - modified_base, modified as per summary sequence table at
       the end of the description

<400>  201
aacaccauga agguggccu                                                    19


<210>  202
<211>  19
<212>  RNA
<213>  Artificial Sequence

<220>
<223>  siRNA - modified_base, modified as per summary sequence table at
       the end of the description

<400>  202
aggccaccuu cauggguguu                                                   19


<210>  203
<211>  19
<212>  RNA
<213>  Artificial Sequence

<220>
<223>  siRNA - modified_base, modified as per summary sequence table at
       the end of the description

<400>  203
uugguauuga gccaaggcu                                                    19


<210>  204
<211>  19
<212>  RNA
<213>  Artificial Sequence

<220>
<223>  siRNA - modified_base, modified as per summary sequence table at
       the end of the description

<400>  204
agccuuggcu caauaccaa                                                    19


<210>  205
<211>  19
<212>  RNA
<213>  Artificial Sequence

<220>
<223>  siRNA - modified_base, modified as per summary sequence table at
       the end of the description

<400>  205
uuuauuacag gugaguuga                                                    19

<210>  206
<211>  19
<212>  RNA
<213>  Artificial Sequence

<220>
<223>  siRNA - modified_base, modified as per summary sequence table at
       the end of the description

<400>  206
ucaacucacc uguaauaaa                                                    19


<210>  207
<211>  19
<212>  RNA
<213>  Artificial Sequence

<220>
<223>  siRNA - modified_base, modified as per summary sequence table at
       the end of the description

<400>  207
uuucuguuuc cggugcugg                                                    19


<210>  208
<211>  19
<212>  RNA
<213>  Artificial Sequence

<220>
<223>  siRNA - modified_base, modified as per summary sequence table at
       the end of the description

<400>  208
ccagcaccgg aaacagaaa                                                    19


<210>  209
<211>  19
<212>  RNA
<213>  Artificial Sequence

<220>
<223>  siRNA - modified_base, modified as per summary sequence table at
       the end of the description

<400>  209
ucaaggauca uaguguucu                                                    19


<210>  210
<211>  19
<212>  RNA
<213>  Artificial Sequence

<220>
<223>  siRNA - modified_base, modified as per summary sequence table at
       the end of the description

<400>  210
agaacacuau gauccuuga                                                    19

<210>  211
<211>  19
<212>  RNA
<213>  Artificial Sequence

<220>
<223>  siRNA - modified_base, modified as per summary sequence table at
       the end of the description

<400>  211
aucucaagga ucauagugu                                                    19


<210>  212
<211>  19
<212>  RNA
<213>  Artificial Sequence

<220>
<223>  siRNA - modified_base, modified as per summary sequence table at
       the end of the description

<400>  212
acacuaugau ccuugagau                                                    19


<210>  213
<211>  19
<212>  RNA
<213>  Artificial Sequence

<220>
<223>  siRNA - modified_base, modified as per summary sequence table at
       the end of the description

<400>  213
ucauacuugg agauguauc                                                    19


<210>  214
<211>  19
<212>  RNA
<213>  Artificial Sequence

<220>
<223>  siRNA - modified_base, modified as per summary sequence table at
       the end of the description

<400>  214
gauacaucuc caaguauga                                                    19


<210>  215
<211>  19
<212>  RNA
<213>  Artificial Sequence

<220>
<223>  siRNA - modified_base, modified as per summary sequence table at
       the end of the description

<400>  215
uaucggagaa ggcuuuguc                                                    19


<210>  216
<211>  19
<212>  RNA
<213>  Artificial Sequence

<220>
<223>  siRNA - modified_base, modified as per summary sequence table at
       the end of the description

<400>  216
gacaaagccu ucuccgaua                                                    19


<210>  217
<211>  19
<212>  RNA
<213>  Artificial Sequence

<220>
<223>  siRNA - modified_base, modified as per summary sequence table at
       the end of the description

<400>  217
augaugaggg uguuccuau                                                    19


<210>  218
<211>  19
<212>  RNA
<213>  Artificial Sequence

<220>
<223>  siRNA - modified_base, modified as per summary sequence table at
       the end of the description

<400>  218
auaggaacac ccucaucau                                                    19


<210>  219
<211>  19
<212>  RNA
<213>  Artificial Sequence

<220>
<223>  siRNA - modified_base, modified as per summary sequence table at
       the end of the description

<400>  219
uauuggugaa cuuugaaag                                                    19


<210>  220
<211>  19
<212>  RNA
<213>  Artificial Sequence

<220>
<223>  siRNA - modified_base, modified as per summary sequence table at

the end of the description

<400>  220
cuuucaaagu ucaccaaua                                                    19


<210>  221
<211>  19
<212>  RNA
<213>  Artificial Sequence

<220>
<223>  siRNA - modified_base, modified as per summary sequence table at
       the end of the description

<400>  221
agcuuguuca gcuuuccau                                                    19


<210>  222
<211>  19
<212>  RNA
<213>  Artificial Sequence

<220>
<223>  siRNA - modified_base, modified as per summary sequence table at
       the end of the description

<400>  222
auggaaagcu gaacaagcu                                                    19


<210>  223
<211>  19
<212>  RNA
<213>  Artificial Sequence

<220>
<223>  siRNA - modified_base, modified as per summary sequence table at
       the end of the description

<400>  223
uuuguaugaa gcaauucuc                                                    19


<210>  224
<211>  19
<212>  RNA
<213>  Artificial Sequence

<220>
<223>  siRNA - modified_base, modified as per summary sequence table at
       the end of the description

<400>  224
gagaauugcu ucauacaaa                                                    19


<210>  225
<211>  19
<212>  RNA
<213>  Artificial Sequence

<220>
<223>  siRNA - modified_base, modified as per summary sequence table at
      the end of the description

<400>  225
gucuuguaca cauagucca                                                    19


<210>  226
<211>  19
<212>  RNA
<213>  Artificial Sequence

<220>
<223>  siRNA - modified_base, modified as per summary sequence table at
      the end of the description

<400>  226
uggacuaugu guacaagac                                                    19


<210>  227
<211>  19
<212>  RNA
<213>  Artificial Sequence

<220>
<223>  siRNA - modified_base, modified as per summary sequence table at
      the end of the description

<400>  227
ugcucaaugg ccaugaugu                                                    19


<210>  228
<211>  19
<212>  RNA
<213>  Artificial Sequence

<220>
<223>  siRNA - modified_base, modified as per summary sequence table at
      the end of the description

<400>  228
acaucauggc cauugagca                                                    19


<210>  229
<211>  19
<212>  RNA
<213>  Artificial Sequence

<220>
<223>  siRNA - modified_base, modified as per summary sequence table at
      the end of the description

<400>  229
uuggcauucg uccuccucg                                                    19


<210>  230
<211>  19
<212>  RNA

<213>  Artificial Sequence

<220>
<223>  siRNA - modified_base, modified as per summary sequence table at
       the end of the description

<400>  230
cgaggaggac gaaugccaa                                                    19


<210>  231
<211>  19
<212>  RNA
<213>  Artificial Sequence

<220>
<223>  siRNA - modified_base, modified as per summary sequence table at
       the end of the description

<400>  231
gucuggauga agagguacc                                                    19


<210>  232
<211>  19
<212>  RNA
<213>  Artificial Sequence

<220>
<223>  siRNA - modified_base, modified as per summary sequence table at
       the end of the description

<400>  232
gguaccucuu cauccagac                                                    19


<210>  233
<211>  19
<212>  RNA
<213>  Artificial Sequence

<220>
<223>  siRNA - modified_base, modified as per summary sequence table at
       the end of the description

<400>  233
ugucuggaug aagagguac                                                    19


<210>  234
<211>  19
<212>  RNA
<213>  Artificial Sequence

<220>
<223>  siRNA - modified_base, modified as per summary sequence table at
       the end of the description

<400>  234
guaccucuuc auccagaca                                                    19


<210>  235

<211>  19
<212>  RNA
<213>  Artificial Sequence

<220>
<223>  siRNA - modified_base, modified as per summary sequence table at
       the end of the description

<400>  235
ucugucugga ugaagaggu                                                    19


<210>  236
<211>  19
<212>  RNA
<213>  Artificial Sequence

<220>
<223>  siRNA - modified_base, modified as per summary sequence table at
       the end of the description

<400>  236
accucuucau ccagacaga                                                    19


<210>  237
<211>  19
<212>  RNA
<213>  Artificial Sequence

<220>
<223>  siRNA - modified_base, modified as per summary sequence table at
       the end of the description

<400>  237
cuugucuguc uggaugaag                                                    19


<210>  238
<211>  19
<212>  RNA
<213>  Artificial Sequence

<220>
<223>  siRNA - modified_base, modified as per summary sequence table at
       the end of the description

<400>  238
cuucauccag acagacaag                                                    19


<210>  239
<211>  19
<212>  RNA
<213>  Artificial Sequence

<220>
<223>  siRNA - modified_base, modified as per summary sequence table at
       the end of the description

<400>  239
auggucuugu cugucugga                                                    19

<210>  240
<211>  19
<212>  RNA
<213>  Artificial Sequence

<220>
<223>  siRNA - modified_base, modified as per summary sequence table at
       the end of the description

<400>  240
uccagacaga caagaccau                                                    19


<210>  241
<211>  19
<212>  RNA
<213>  Artificial Sequence

<220>
<223>  siRNA - modified_base, modified as per summary sequence table at
       the end of the description

<400>  241
agauggucuu gucugucug                                                    19


<210>  242
<211>  19
<212>  RNA
<213>  Artificial Sequence

<220>
<223>  siRNA - modified_base, modified as per summary sequence table at
       the end of the description

<400>  242
cagacagaca agaccaucu                                                    19


<210>  243
<211>  19
<212>  RNA
<213>  Artificial Sequence

<220>
<223>  siRNA - modified_base, modified as per summary sequence table at
       the end of the description

<400>  243
guagaugguc uugucuguc                                                    19


<210>  244
<211>  19
<212>  RNA
<213>  Artificial Sequence

<220>
<223>  siRNA - modified_base, modified as per summary sequence table at
       the end of the description

<400>  244

gacagacaag accaucuac                                                    19

<210>  245
<211>  19
<212>  RNA
<213>  Artificial Sequence

<220>
<223>  siRNA - modified_base, modified as per summary sequence table at
       the end of the description

<400>  245
guguagaugg ucuugucug                                                    19

<210>  246
<211>  19
<212>  RNA
<213>  Artificial Sequence

<220>
<223>  siRNA - modified_base, modified as per summary sequence table at
       the end of the description

<400>  246
cagacaagac caucuacac                                                    19

<210>  247
<211>  19
<212>  RNA
<213>  Artificial Sequence

<220>
<223>  siRNA - modified_base, modified as per summary sequence table at
       the end of the description

<400>  247
gggguguaga uggucuugu                                                    19

<210>  248
<211>  19
<212>  RNA
<213>  Artificial Sequence

<220>
<223>  siRNA - modified_base, modified as per summary sequence table at
       the end of the description

<400>  248
acaagaccau cuacacccc                                                    19

<210>  249
<211>  19
<212>  RNA
<213>  Artificial Sequence

<220>
<223>  siRNA - modified_base, modified as per summary sequence table at
       the end of the description

<400>  249
uaggcucgga ucuuccacu                                                    19


<210>  250
<211>  19
<212>  RNA
<213>  Artificial Sequence

<220>
<223>  siRNA - modified_base, modified as per summary sequence table at
       the end of the description

<400>  250
aguggaagau ccgagccua                                                    19


<210>  251
<211>  19
<212>  RNA
<213>  Artificial Sequence

<220>
<223>  siRNA - modified_base, modified as per summary sequence table at
       the end of the description

<400>  251
cucgaaacug ggcagcacg                                                    19


<210>  252
<211>  19
<212>  RNA
<213>  Artificial Sequence

<220>
<223>  siRNA - modified_base, modified as per summary sequence table at
       the end of the description

<400>  252
cgugcugccc aguuucgag                                                    19


<210>  253
<211>  19
<212>  RNA
<213>  Artificial Sequence

<220>
<223>  siRNA - modified_base, modified as per summary sequence table at
       the end of the description

<400>  253
uuggugaagu ggaucuggu                                                    19


<210>  254
<211>  19
<212>  RNA
<213>  Artificial Sequence

<220>

<223>  siRNA - modified_base, modified as per summary sequence table at
       the end of the description

<400>  254
accagaucca cuucaccaa                                                    19


<210>  255
<211>  19
<212>  RNA
<213>  Artificial Sequence

<220>
<223>  siRNA - modified_base, modified as per summary sequence table at
       the end of the description

<400>  255
ucuuggugaa guggaucug                                                    19


<210>  256
<211>  19
<212>  RNA
<213>  Artificial Sequence

<220>
<223>  siRNA - modified_base, modified as per summary sequence table at
       the end of the description

<400>  256
cagauccacu ucaccaaga                                                    19


<210>  257
<211>  19
<212>  RNA
<213>  Artificial Sequence

<220>
<223>  siRNA - modified_base, modified as per summary sequence table at
       the end of the description

<400>  257
gucuugguga aguggaucu                                                    19


<210>  258
<211>  19
<212>  RNA
<213>  Artificial Sequence

<220>
<223>  siRNA - modified_base, modified as per summary sequence table at
       the end of the description

<400>  258
agauccacuu caccaagac                                                    19


<210>  259
<211>  19
<212>  RNA
<213>  Artificial Sequence

<220>
<223>  siRNA - modified_base, modified as per summary sequence table at
      the end of the description

<400>  259
ggugucuugg ugaagugga                                                    19


<210>  260
<211>  19
<212>  RNA
<213>  Artificial Sequence

<220>
<223>  siRNA - modified_base, modified as per summary sequence table at
      the end of the description

<400>  260
uccacuucac caagacacc                                                    19


<210>  261
<211>  19
<212>  RNA
<213>  Artificial Sequence

<220>
<223>  siRNA - modified_base, modified as per summary sequence table at
      the end of the description

<400>  261
aacaccauga ggucaaagg                                                    19


<210>  262
<211>  19
<212>  RNA
<213>  Artificial Sequence

<220>
<223>  siRNA - modified_base, modified as per summary sequence table at
      the end of the description

<400>  262
ccuuugaccu cauggguguu                                                    19


<210>  263
<211>  19
<212>  RNA
<213>  Artificial Sequence

<220>
<223>  siRNA - modified_base, modified as per summary sequence table at
      the end of the description

<400>  263
gaacaccaug aggucaaag                                                    19


<210>  264
<211>  19

<212>  RNA
<213>  Artificial Sequence

<220>
<223>  siRNA - modified_base, modified as per summary sequence table at
       the end of the description

<400>  264
cuuugaccuc augguguuc                                                    19


<210>  265
<211>  19
<212>  RNA
<213>  Artificial Sequence

<220>
<223>  siRNA - modified_base, modified as per summary sequence table at
       the end of the description

<400>  265
gucacgaaca ccaugaggu                                                    19


<210>  266
<211>  19
<212>  RNA
<213>  Artificial Sequence

<220>
<223>  siRNA - modified_base, modified as per summary sequence table at
       the end of the description

<400>  266
accucauggu guucgugac                                                    19


<210>  267
<211>  19
<212>  RNA
<213>  Artificial Sequence

<220>
<223>  siRNA - modified_base, modified as per summary sequence table at
       the end of the description

<400>  267
acacagaucc cuuucuugu                                                    19


<210>  268
<211>  19
<212>  RNA
<213>  Artificial Sequence

<220>
<223>  siRNA - modified_base, modified as per summary sequence table at
       the end of the description

<400>  268
acaagaaagg gaucugugu                                                    19

<210>  269
<211>  19
<212>  RNA
<213>  Artificial Sequence

<220>
<223>  siRNA - modified_base, modified as per summary sequence table at
       the end of the description

<400>  269
gucugccaca cagaucccu                                                    19


<210>  270
<211>  19
<212>  RNA
<213>  Artificial Sequence

<220>
<223>  siRNA - modified_base, modified as per summary sequence table at
       the end of the description

<400>  270
agggaucugu guggcagac                                                    19


<210>  271
<211>  19
<212>  RNA
<213>  Artificial Sequence

<220>
<223>  siRNA - modified_base, modified as per summary sequence table at
       the end of the description

<400>  271
gaugaagaag uccugcauu                                                    19


<210>  272
<211>  19
<212>  RNA
<213>  Artificial Sequence

<220>
<223>  siRNA - modified_base, modified as per summary sequence table at
       the end of the description

<400>  272
aaugcaggac uucuucauc                                                    19


<210>  273
<211>  19
<212>  RNA
<213>  Artificial Sequence

<220>
<223>  siRNA - modified_base, modified as per summary sequence table at
       the end of the description

<400>  273
ccuugcagga gaauucugg                                                    19

<210>  274
<211>  19
<212>  RNA
<213>  Artificial Sequence

<220>
<223>  siRNA - modified_base, modified as per summary sequence table at
       the end of the description

<400>  274
ccagaauucu ccugcaagg                                                    19


<210>  275
<211>  19
<212>  RNA
<213>  Artificial Sequence

<220>
<223>  siRNA - modified_base, modified as per summary sequence table at
       the end of the description

<400>  275
agagagaaga ccuugacca                                                    19


<210>  276
<211>  19
<212>  RNA
<213>  Artificial Sequence

<220>
<223>  siRNA - modified_base, modified as per summary sequence table at
       the end of the description

<400>  276
uggucaaggu cuucucucu                                                    19


<210>  277
<211>  19
<212>  RNA
<213>  Artificial Sequence

<220>
<223>  siRNA - modified_base, modified as per summary sequence table at
       the end of the description

<400>  277
gagucgaugg cgaugaggu                                                    19


<210>  278
<211>  19
<212>  RNA
<213>  Artificial Sequence

<220>
<223>  siRNA - modified_base, modified as per summary sequence table at
       the end of the description

<400>  278
accucaucgc caucgacuc                                                    19


<210>  279
<211>  19
<212>  RNA
<213>  Artificial Sequence

<220>
<223>  siRNA - modified_base, modified as per summary sequence table at
       the end of the description

<400>  279
gcuuggaaca ccaugaagg                                                    19


<210>  280
<211>  19
<212>  RNA
<213>  Artificial Sequence

<220>
<223>  siRNA - modified_base, modified as per summary sequence table at
       the end of the description

<400>  280
ccuucauggu guuccaagc                                                    19


<210>  281
<211>  19
<212>  RNA
<213>  Artificial Sequence

<220>
<223>  siRNA - modified_base, modified as per summary sequence table at
       the end of the description

<400>  281
ucauuuuccu uggucucuu                                                    19


<210>  282
<211>  19
<212>  RNA
<213>  Artificial Sequence

<220>
<223>  siRNA - modified_base, modified as per summary sequence table at
       the end of the description

<400>  282
aagagaccaa ggaaaauga                                                    19


<210>  283
<211>  19
<212>  RNA
<213>  Artificial Sequence

<220>
<223>  siRNA - modified_base, modified as per summary sequence table at

the end of the description

<400>  283
ugugucugga gcaaagcca                                                    19


<210>  284
<211>  19
<212>  RNA
<213>  Artificial Sequence

<220>
<223>  siRNA - modified_base, modified as per summary sequence table at
       the end of the description

<400>  284
uggcuuugcu ccagacaca                                                    19


<210>  285
<211>  19
<212>  RNA
<213>  Artificial Sequence

<220>
<223>  siRNA - modified_base, modified as per summary sequence table at
       the end of the description

<400>  285
agguagauga ugagggugu                                                    19


<210>  286
<211>  19
<212>  RNA
<213>  Artificial Sequence

<220>
<223>  siRNA - modified_base, modified as per summary sequence table at
       the end of the description

<400>  286
acacccucau caucuaccu                                                    19


<210>  287
<211>  19
<212>  RNA
<213>  Artificial Sequence

<220>
<223>  siRNA - modified_base, modified as per summary sequence table at
       the end of the description

<400>  287
uuguaauagg cguagaccu                                                    19


<210>  288
<211>  19
<212>  RNA
<213>  Artificial Sequence

<220>
<223>  siRNA - modified_base, modified as per summary sequence table at
      the end of the description

<400>  288
aggucuacgc cuauuacaa                                                    19


<210>  289
<211>  19
<212>  RNA
<213>  Artificial Sequence

<220>
<223>  siRNA - modified_base, modified as per summary sequence table at
      the end of the description

<400>  289
guuguaauag gcguagacc                                                    19


<210>  290
<211>  19
<212>  RNA
<213>  Artificial Sequence

<220>
<223>  siRNA - modified_base, modified as per summary sequence table at
      the end of the description

<400>  290
ggucuacgcc uauuacaac                                                    19


<210>  291
<211>  19
<212>  RNA
<213>  Artificial Sequence

<220>
<223>  siRNA - modified_base, modified as per summary sequence table at
      the end of the description

<400>  291
gggucuugua cacauaguc                                                    19


<210>  292
<211>  19
<212>  RNA
<213>  Artificial Sequence

<220>
<223>  siRNA - modified_base, modified as per summary sequence table at
      the end of the description

<400>  292
gacuaugugu acaagaccc                                                    19


<210>  293
<211>  19
<212>  RNA

<213>  Artificial Sequence

<220>
<223>  siRNA - modified_base, modified as per summary sequence table at
       the end of the description

<400>  293
cacuugaugg ggcugauga                                                    19


<210>  294
<211>  19
<212>  RNA
<213>  Artificial Sequence

<220>
<223>  siRNA - modified_base, modified as per summary sequence table at
       the end of the description

<400>  294
ucaucagccc caucaagug                                                    19


<210>  295
<211>  19
<212>  RNA
<213>  Artificial Sequence

<220>
<223>  siRNA - modified_base, modified as per summary sequence table at
       the end of the description

<400>  295
gguaccucuu cauccagaa                                                    19


<210>  296
<211>  19
<212>  RNA
<213>  Artificial Sequence

<220>
<223>  siRNA - modified_base, modified as per summary sequence table at
       the end of the description

<400>  296
guaccucuuc auccagaca                                                    19


<210>  297
<211>  19
<212>  RNA
<213>  Artificial Sequence

<220>
<223>  siRNA - modified_base, modified as per summary sequence table at
       the end of the description

<400>  297
accucuucau ccagacaga                                                    19


<210>  298

<211>  19
<212>  RNA
<213>  Artificial Sequence

<220>
<223>  siRNA - modified_base, modified as per summary sequence table at
       the end of the description

<400>  298
cuucauccag acagacaaa                                                    19


<210>  299
<211>  19
<212>  RNA
<213>  Artificial Sequence

<220>
<223>  siRNA - modified_base, modified as per summary sequence table at
       the end of the description

<400>  299
uccagacaga caagaccaa                                                    19


<210>  300
<211>  19
<212>  RNA
<213>  Artificial Sequence

<220>
<223>  siRNA - modified_base, modified as per summary sequence table at
       the end of the description

<400>  300
cagacagaca agaccauca                                                    19


<210>  301
<211>  19
<212>  RNA
<213>  Artificial Sequence

<220>
<223>  siRNA - modified_base, modified as per summary sequence table at
       the end of the description

<400>  301
gacagacaag accaucuaa                                                    19


<210>  302
<211>  19
<212>  RNA
<213>  Artificial Sequence

<220>
<223>  siRNA - modified_base, modified as per summary sequence table at
       the end of the description

<400>  302
cagacaagac caucuacaa                                                    19

<210>  303
<211>  19
<212>  RNA
<213>  Artificial Sequence

<220>
<223>  siRNA - modified_base, modified as per summary sequence table at
       the end of the description

<400>  303
acaagaccau cuacaccca                                                    19


<210>  304
<211>  19
<212>  RNA
<213>  Artificial Sequence

<220>
<223>  siRNA - modified_base, modified as per summary sequence table at
       the end of the description

<400>  304
aguggaagau ccgagccua                                                    19


<210>  305
<211>  19
<212>  RNA
<213>  Artificial Sequence

<220>
<223>  siRNA - modified_base, modified as per summary sequence table at
       the end of the description

<400>  305
cgugcugccc aguuucgaa                                                    19


<210>  306
<211>  19
<212>  RNA
<213>  Artificial Sequence

<220>
<223>  siRNA - modified_base, modified as per summary sequence table at
       the end of the description

<400>  306
accagaucca cuucaccaa                                                    19


<210>  307
<211>  19
<212>  RNA
<213>  Artificial Sequence

<220>
<223>  siRNA - modified_base, modified as per summary sequence table at
       the end of the description

<400>  307

cagauccacu ucaccaaga                                                    19

<210>  308
<211>  19
<212>  RNA
<213>  Artificial Sequence

<220>
<223>  siRNA - modified_base, modified as per summary sequence table at
       the end of the description

<400>  308
agauccacuu caccaagaa                                                    19

<210>  309
<211>  19
<212>  RNA
<213>  Artificial Sequence

<220>
<223>  siRNA - modified_base, modified as per summary sequence table at
       the end of the description

<400>  309
uccacuucac caagacaca                                                    19

<210>  310
<211>  19
<212>  RNA
<213>  Artificial Sequence

<220>
<223>  siRNA - modified_base, modified as per summary sequence table at
       the end of the description

<400>  310
ccuuugaccu cauggugua                                                    19

<210>  311
<211>  19
<212>  RNA
<213>  Artificial Sequence

<220>
<223>  siRNA - modified_base, modified as per summary sequence table at
       the end of the description

<400>  311
cuuugaccuc augguguua                                                    19

<210>  312
<211>  19
<212>  RNA
<213>  Artificial Sequence

<220>
<223>  siRNA - modified_base, modified as per summary sequence table at
       the end of the description

<400>  312
accucauggu guucgugaa                                                    19


<210>  313
<211>  19
<212>  RNA
<213>  Artificial Sequence

<220>
<223>  siRNA - modified_base, modified as per summary sequence table at
       the end of the description

<400>  313
acaagaaagg gaucuguga                                                    19


<210>  314
<211>  19
<212>  RNA
<213>  Artificial Sequence

<220>
<223>  siRNA - modified_base, modified as per summary sequence table at
       the end of the description

<400>  314
agggaucugu guggcagaa                                                    19


<210>  315
<211>  19
<212>  RNA
<213>  Artificial Sequence

<220>
<223>  siRNA - modified_base, modified as per summary sequence table at
       the end of the description

<400>  315
aaugcaggac uucuucaua                                                    19


<210>  316
<211>  19
<212>  RNA
<213>  Artificial Sequence

<220>
<223>  siRNA - modified_base, modified as per summary sequence table at
       the end of the description

<400>  316
ccagaauucu ccugcaaga                                                    19


<210>  317
<211>  19
<212>  RNA
<213>  Artificial Sequence

<220>

<223>  siRNA - modified_base, modified as per summary sequence table at
       the end of the description

<400>  317
uggucaaggu cuucucuca                                                    19


<210>  318
<211>  19
<212>  RNA
<213>  Artificial Sequence

<220>
<223>  siRNA - modified_base, modified as per summary sequence table at
       the end of the description

<400>  318
accucaucgc caucgacua                                                    19


<210>  319
<211>  19
<212>  RNA
<213>  Artificial Sequence

<220>
<223>  siRNA - modified_base, modified as per summary sequence table at
       the end of the description

<400>  319
ccuucauggu guuccaaga                                                    19


<210>  320
<211>  19
<212>  RNA
<213>  Artificial Sequence

<220>
<223>  siRNA - modified_base, modified as per summary sequence table at
       the end of the description

<400>  320
aagagaccaa ggaaaauga                                                    19


<210>  321
<211>  19
<212>  RNA
<213>  Artificial Sequence

<220>
<223>  siRNA - modified_base, modified as per summary sequence table at
       the end of the description

<400>  321
uggcuuugcu ccagacaca                                                    19


<210>  322
<211>  19
<212>  RNA
<213>  Artificial Sequence

<220>
<223>  siRNA - modified_base, modified as per summary sequence table at
      the end of the description

<400>  322
acacccucau caucuacca                                                    19


<210>  323
<211>  19
<212>  RNA
<213>  Artificial Sequence

<220>
<223>  siRNA - modified_base, modified as per summary sequence table at
      the end of the description

<400>  323
aggucuacgc cuauuacaa                                                    19


<210>  324
<211>  19
<212>  RNA
<213>  Artificial Sequence

<220>
<223>  siRNA - modified_base, modified as per summary sequence table at
      the end of the description

<400>  324
ggucuacgcc uauuacaaa                                                    19


<210>  325
<211>  19
<212>  RNA
<213>  Artificial Sequence

<220>
<223>  siRNA - modified_base, modified as per summary sequence table at
      the end of the description

<400>  325
gacuaugugu acaagacca                                                    19


<210>  326
<211>  19
<212>  RNA
<213>  Artificial Sequence

<220>
<223>  siRNA - modified_base, modified as per summary sequence table at
      the end of the description

<400>  326
ucaucagccc caucaagua                                                    19


<210>  327
<211>  19

<212>  RNA
<213>  Artificial Sequence

<220>
<223>  siRNA - modified_base, modified as per summary sequence table at
       the end of the description

<400>  327
aggaaguuga cguugaggg                                                    19


<210>  328
<211>  19
<212>  RNA
<213>  Artificial Sequence

<220>
<223>  siRNA - modified_base, modified as per summary sequence table at
       the end of the description

<400>  328
cccucaacgu caacuuccu                                                    19


<210>  329
<211>  19
<212>  RNA
<213>  Artificial Sequence

<220>
<223>  siRNA - modified_base, modified as per summary sequence table at
       the end of the description

<400>  329
aauauauuca ugagcuucg                                                    19


<210>  330
<211>  19
<212>  RNA
<213>  Artificial Sequence

<220>
<223>  siRNA - modified_base, modified as per summary sequence table at
       the end of the description

<400>  330
cgaagcucau gaauauauu                                                    19


<210>  331
<211>  19
<212>  RNA
<213>  Artificial Sequence

<220>
<223>  siRNA - modified_base, modified as per summary sequence table at
       the end of the description

<400>  331
augaugaggg uguuccuau                                                    19

<210>  332
<211>  19
<212>  RNA
<213>  Artificial Sequence

<220>
<223>  siRNA - modified_base, modified as per summary sequence table at
       the end of the description

<400>  332
auaggaacac ccucaucau                                                    19


<210>  333
<211>  19
<212>  RNA
<213>  Artificial Sequence

<220>
<223>  siRNA - modified_base, modified as per summary sequence table at
       the end of the description

<400>  333
ucugucugga ugaagaggu                                                    19


<210>  334
<211>  19
<212>  RNA
<213>  Artificial Sequence

<220>
<223>  siRNA - modified_base, modified as per summary sequence table at
       the end of the description

<400>  334
accucuucau ccagacaga                                                    19


<210>  335
<211>  19
<212>  RNA
<213>  Artificial Sequence

<220>
<223>  siRNA - modified_base, modified as per summary sequence table at
       the end of the description

<400>  335
guagaugguc uugucuguc                                                    19


<210>  336
<211>  19
<212>  RNA
<213>  Artificial Sequence

<220>
<223>  siRNA - modified_base, modified as per summary sequence table at
       the end of the description

<400>  336
gacagacaag accaucuac                                                    19

<210>  337
<211>  19
<212>  RNA
<213>  Artificial Sequence

<220>
<223>  siRNA - modified_base, modified as per summary sequence table at
       the end of the description

<400>  337
gaacaccaug aggucaaag                                                    19


<210>  338
<211>  19
<212>  RNA
<213>  Artificial Sequence

<220>
<223>  siRNA - modified_base, modified as per summary sequence table at
       the end of the description

<400>  338
cuuugaccuc augguguuc                                                    19


<210>  339
<211>  19
<212>  RNA
<213>  Artificial Sequence

<220>
<223>  siRNA - modified_base, modified as per summary sequence table at
       the end of the description

<400>  339
agagagaaga ccuugacca                                                    19


<210>  340
<211>  19
<212>  RNA
<213>  Artificial Sequence

<220>
<223>  siRNA - modified_base, modified as per summary sequence table at
       the end of the description

<400>  340
uggucaaggu cuucucucu                                                    19


<210>  341
<211>  19
<212>  RNA
<213>  Artificial Sequence

<220>
<223>  siRNA - modified_base, modified as per summary sequence table at
       the end of the description

<400>  341
cuugucuguc uggaugaag                                                    19


<210>  342
<211>  19
<212>  RNA
<213>  Artificial Sequence

<220>
<223>  siRNA - modified_base, modified as per summary sequence table at
       the end of the description

<400>  342
cuucauccag acagacaaa                                                    19


<210>  343
<211>  19
<212>  RNA
<213>  Artificial Sequence

<220>
<223>  siRNA - modified_base, modified as per summary sequence table at
       the end of the description

<400>  343
guagaugguc uugucuguc                                                    19


<210>  344
<211>  19
<212>  RNA
<213>  Artificial Sequence

<220>
<223>  siRNA - modified_base, modified as per summary sequence table at
       the end of the description

<400>  344
gacagacaag accaucuaa                                                    19


<210>  345
<211>  19
<212>  RNA
<213>  Artificial Sequence

<220>
<223>  siRNA - modified_base, modified as per summary sequence table at
       the end of the description

<400>  345
ucuuggugaa guggaucug                                                    19


<210>  346
<211>  19
<212>  RNA
<213>  Artificial Sequence

<220>
<223>  siRNA - modified_base, modified as per summary sequence table at

the end of the description

<400>  346
cagauccacu ucaccaaga                                                    19


<210>  347
<211>  19
<212>  RNA
<213>  Artificial Sequence

<220>
<223>  siRNA - modified_base, modified as per summary sequence table at
       the end of the description

<400>  347
guuguaauag gcguagacc                                                    19


<210>  348
<211>  19
<212>  RNA
<213>  Artificial Sequence

<220>
<223>  siRNA - modified_base, modified as per summary sequence table at
       the end of the description

<400>  348
ggucuacgcc uauuacaaa                                                    19


<210>  349
<211>  19
<212>  RNA
<213>  Artificial Sequence

<220>
<223>  siRNA - modified_base, modified as per summary sequence table at
       the end of the description

<400>  349
auuguaauag gcguagacc                                                    19


<210>  350
<211>  19
<212>  RNA
<213>  Artificial Sequence

<220>
<223>  siRNA - modified_base, modified as per summary sequence table at
       the end of the description

<400>  350
ggucuacgcc uauuacaau                                                    19


<210>  351
<211>  19
<212>  RNA
<213>  Artificial Sequence

<220>
<223>  siRNA - modified_base, modified as per summary sequence table at
      the end of the description

<400>  351
auguagaugg ucuugucug                                                    19


<210>  352
<211>  19
<212>  RNA
<213>  Artificial Sequence

<220>
<223>  siRNA - modified_base, modified as per summary sequence table at
      the end of the description

<400>  352
cagacaagac caucuacau                                                    19


<210>  353
<211>  19
<212>  RNA
<213>  Artificial Sequence

<220>
<223>  siRNA - modified_base, modified as per summary sequence table at
      the end of the description

<400>  353
ugagagaaga ccuugacca                                                    19


<210>  354
<211>  19
<212>  RNA
<213>  Artificial Sequence

<220>
<223>  siRNA - modified_base, modified as per summary sequence table at
      the end of the description

<400>  354
uggucaaggu cuucucucu                                                    19


<210>  355
<211>  19
<212>  RNA
<213>  Artificial Sequence

<220>
<223>  siRNA - modified_base, modified as per summary sequence table at
      the end of the description

<400>  355
uggucaaggu cuucucucu                                                    19


<210>  356
<211>  19
<212>  RNA

<213>  Artificial Sequence

<220>
<223>  siRNA - modified_base, modified as per summary sequence table at
       the end of the description

<400>  356
ugagagaaga ccuugacca                                                    19


<210>  357
<211>  19
<212>  RNA
<213>  Artificial Sequence

<220>
<223>  siRNA - modified_base, modified as per summary sequence table at
       the end of the description

<400>  357
cagauccacu ucaccaaga                                                    19


<210>  358
<211>  19
<212>  RNA
<213>  Artificial Sequence

<220>
<223>  siRNA - modified_base, modified as per summary sequence table at
       the end of the description

<400>  358
cagauccacu ucaccaaga                                                    19


<210>  359
<211>  19
<212>  RNA
<213>  Artificial Sequence

<220>
<223>  siRNA - modified_base, modified as per summary sequence table at
       the end of the description

<400>  359
ucuuggugaa guggaucug                                                    19


<210>  360
<211>  19
<212>  RNA
<213>  Artificial Sequence

<220>
<223>  siRNA - modified_base, modified as per summary sequence table at
       the end of the description

<400>  360
ucuuggugaa guggaucug                                                    19


<210>  361

<211>  19
<212>  RNA
<213>  Artificial Sequence

<220>
<223>  siRNA

<400>  361
ugagagaaga ccuugacca                                                    19

## Description

### Field of the invention

The invention relates to nucleic acid products that interfere with or inhibit C3 complement component gene expression. It further relates to therapeutic uses of such inhibition such as for the treatment of diseases and disorders associated with complement pathway deregulation and/or over-activation or with ectopic expression or localisation or accumulation of the complement component C3 in the body.

### Background

Double stranded RNAs (dsRNA) able to complementarily bind expressed mRNA have been shown to be able to block gene expression (Fire et al., 1998, Nature. 1998 Feb 19;391(6669):806-11 and Elbashir et al., 2001, Nature. 2001 May 24;411(6836):494-8) by a mechanism that has been termed RNA interference (RNAi). Short dsRNAs direct gene specific, post transcriptional silencing in many organisms, including vertebrates, and have become a useful tool for studying gene function. RNAi is mediated by the RNA induced silencing complex (RISC), a sequence specific, multi component nuclease that degrades messenger RNAs homologous to the silencing trigger loaded into the RISC complex. Interfering RNA (termed herein iRNA) such as siRNAs, antisense RNAs, and micro RNAs are oligonucleotides that prevent the formation of proteins by gene silencing i.e. inhibiting gene translation of the protein through degradation of mRNA molecules. Gene silencing agents are becoming increasingly important for therapeutic applications in medicine.

According to Watts and Corey in the Journal of Pathology (2012; Vol 226, p 365 379) there are algorithms that can be used to design nucleic acid silencing triggers, but all of these have severe limitations. It may take various experimental methods to identify potent iRNAs, as algorithms do not take into account factors such as tertiary structure of the target mRNA or the involvement of RNA binding proteins. Therefore, the discovery of a potent nucleic acid silencing trigger with minimal off target effects is a complex process. For the pharmaceutical development of these highly charged molecules it is necessary that they can be synthesised economically, distributed to target tissues, enter cells and function within acceptable limits of toxicity.

The complement system or pathway is part of the innate immune system of host defence against invading pathogens. It mainly consists of a number of proteins that circulate in the bloodstream in the form of precursors. Most of the proteins that form the complement system, including the complement component protein C3 (also referred to herein simply as C3), are largely synthesised and secreted into the bloodstream by hepatocytes in the liver. Activation of the system leads to inflammatory responses resulting in phagocyte attraction and opsonization and consequently clearance of pathogens, immune complexes and cellular debris (Janeway's Immunobiology 9th Edition). The complement system consists of 3 pathways (Classical, Leptin and Alternative pathway), which all converge at the formation of so-called complement component 3 convertase enzyme complexes. These enzyme complexes cleave the complement component C3 protein into C3a and C3b. Once cleaved, C3b forms part of a complex that in turn cleaves C5 into C5a and C5b. After cleavage, C5b is one of the key components of the main complement pathway effectors, the membrane attack complex. C3 is therefore a key component of the complement system activation pathway.

Several diseases are associated with aberrant acquired or genetic activation of the complement pathway as well as with aberrant or over-expression of C3. Among others these are C3 Glomerulopathy (C3G), atypical Hemolytic Uremic Syndrome (aHUS), Immune Complex-mediated Glomerulonephritis (IC-mediated GN), post-Infectious Glomerulonephritis (PIGN), Systemic Lupus Erythematosus, Lupus nephritis, Ischemia/reperfusion injury and IgA nephropathy (IgA N; reviewed in Ricklin et al., Nephrology, 2016 and others). Most of these diseases are associated with the kidney, as this organ is uniquely sensitive to complement-induced damage. However, diseases of other organs are also known to be related to complement dysfunction, such as age-related macular degeneration (AMD), Rheumatoid arthritis (RA), antineutrophil Cytoplasmic Autoantibodies-associated Vasculitis (ANCA-AV), dysbiotic periodontal Disease, Malarial Anaemia, Paroxysmal Nocturnal Hemoglobinuria (PNH) and sepsis.

In C3G, C3 accumulates in the glomeruli in the kidney and clogs them. The accumulation of C3 also leads to kidney damage. In atypical Hemolytic Uremic Syndrome (aHUS) the complement system targets red blood cells, which leads to lysis of the red blood cells.

There are currently only few treatments for complement system mediated diseases, disorders and syndromes. The monoclonal humanized antibody Eculizumab is one of them. It is known to bind complement protein C5, thereby blocking the membrane attack complex at the end of the complement cascade (Hillmen et al., 2006 NEJM). However, only a subset of patients suffering from the above listed diseases respond to Eculizumab therapy. There is therefore a high unmet need for medical treatments of complement mediated or associated diseases. C3 is a pivotal factor in the complement pathway activation. Inhibiting C3 therefore presents a promising therapeutic strategy for many complement-mediated diseases.

### Summary of the invention

One aspect of the invention is a double-stranded nucleic acid for inhibiting expression of complement component C3, wherein the nucleic acid comprises a first strand and a second strand, wherein the first strand sequence comprises a sequence of at least 15 nucleotides differing by no more than 3 nucleotides from any one of the sequences SEQ ID NO: 361, 95, 111, 125, 131, 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 97, 99, 101, 103, 105, 107, 109, 113, 115, 117, 119, 121, 123, 127, 129 or 133.

One aspect relates to a double-stranded nucleic acid that is capable of inhibiting expression of complement component C3 for use as a medicament

One aspect relates to a composition comprising a nucleic acid disclosed herein and a delivery vehicle and/or a physiologically acceptable excipient and/or a carrier and/or a diluent and/or a buffer and/or a preservative.

One aspect relates to a composition comprising a nucleic acid disclosed herein and a further therapeutic agent selected from the group comprising an oligonucleotide, a small molecule, a monoclonal antibody, a polyclonal antibody and a peptide.

One aspect relates to a nucleic acid or composition disclosed herein for use as a medicament.

One aspect relates to a nucleic acid or composition disclosed herein for use in the prevention, decrease of the risk of suffering from, or treatment of a disease, disorder or syndrome.

One aspect relates to the use of a nucleic acid or composition disclosed herein in the prevention, decrease of the risk of suffering from, or treatment of a disease, disorder or syndrome, wherein the disease, disorder or syndrome is preferably C3 Glomerulopathy (C3G).

One aspect relates to a method of preventing, decreasing the risk of suffering from, or treating a disease, disorder or syndrome comprising administering a pharmaceutically effective dose of a nucleic acid or composition disclosed herein to an individual in need of treatment, preferably wherein the nucleic acid or composition is administered to the subject subcutaneously, intravenously or by oral, rectal or intraperitoneal administration.

### Detailed description of the invention

The present invention relates to a nucleic acid which is double stranded and directed to an expressed RNA transcript of the complement component C3 and compositions thereof. These nucleic acids or conjugated nucleic acids or compositions can be used in the treatment and prevention of a variety of diseases, disorders and syndromes in which reduced expression of the C3 gene product is desirable.

A first aspect of the invention is a double-stranded nucleic acid for inhibiting expression of C3, preferably in a cell, wherein the nucleic acid comprises a first strand and a second strand, wherein the first strand sequence comprises a sequence of at least 15 nucleotides differing by no more than 3 nucleotides from any one of the sequences SEQ ID NO: 361, 95, 111, 125, 131, 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 97, 99, 101, 103, 105, 107, 109, 113, 115, 117, 119, 121, 123, 127, 129 or 133. These nucleic acids among others have the advantage of being active in various species that are relevant for pre-clinical and clinical development and/or of having few relevant off-target effects.

Preferably, the first strand sequence comprises a sequence of at least 16, more preferably at least 17, yet more preferably at least 18 and most preferably all 19 nucleotides differing preferably by no more than 2 nucleotides, more preferably by no more than 1 nucleotide, and most preferably not differing by any nucleotide from any one of the sequences SEQ ID NO: 361, 95, 111, 125, 131, 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 97, 99, 101, 103, 105, 107, 109, 113, 115, 117, 119, 121, 123, 127, 129 or 133.

Preferably the first strand sequence of the nucleic acid consists of one of the sequences SEQ ID NO: 361, 95, 111, 125, 131, 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 97, 99, 101, 103, 105, 107, 109, 113, 115, 117, 119, 121, 123, 127, 129 or 133. The sequence may however be modified by a number of modifications that do not change the identity of the nucleotide. For examples, modifications of the backbone of the nucleic acid do not change the identity of the nucleotide because the base itself remains the same as in the reference sequence.

A nucleic acid that comprises a sequence according to a reference sequence herein means that the nucleic acid comprises a sequence of contiguous nucleotides in the order as defined in the reference sequence.

When reference is made herein to a reference sequence comprising or consisting of unmodified nucleotides, this reference is not limited to the sequence with unmodified nucleotides. The same reference also encompasses the same nucleotide sequence in which one, several, such as two, three, four, five, six, seven or more, including all, nucleotides are modified by modifications such as 2'-OMe, 2'-F, a ligand, a linker, a 3' end or 5' end modification or any other modification. It also refers to sequences in which two or more nucleotides are linked to each other by the natural phosphodiester linkage or by any other linkage such as a phosphorothioate or a phosphorodithioate linkage.

A double-stranded nucleic acid is a nucleic acid in which the first strand and the second strand hybridise to each other over at least part of their lengths and are therefore capable of forming a duplex region under physiological conditions, such as in PBS at 37°C at a concentration of 1 µM of each strand. The first and second strand are preferably able to hybridise to each other and therefore to form a duplex region over a region of at least 15 nucleotides, preferably 16, 17, 18 or 19 nucleotides. This duplex region comprises nucleotide base parings between the two strands, preferably based on Watson-Crick base pairing and/or wobble base pairing (such as GU base pairing). All the nucleotides of the two strands within a duplex region do not have to base pair to each other to form a duplex region. A certain number of mismatches, deletions or insertions between the nucleotide sequences of the two strands are acceptable. Overhangs on either end of the first or second strand or unpaired nucleotides at either end of the double-stranded nucleic acid are also possible. The double stranded nucleic acid is preferably a stable double stranded nucleic acid under physiological conditions and preferably has a melting temperature (Tm) of 45°C or more, preferably 50°C or more, and more preferably 55°C or more for example in PBS at a concentration 1 µM of each strand.

The first strand and the second strand are preferably capable of forming a duplex region (ie are complementary to each other) over i) at least a portion of their lengths, preferably over at least 15 nucleotides of both of their lengths, ii) over the entire length of the first strand, iii) over the entire length of the second strand and/or iv) over the entire length of both the first and the second strand. Strands being complementary to each other over a certain length means that the strands are able to base pair to each other, either via Watson-Crick or wobble base pairing, over that length. Each nucleotide of the length does not necessarily have to be able to base pair with its counterpart in the other strand over the entire given length as long as a stable double-stranded nucleotide under physiological conditions can be formed. This is however preferred.

A certain number of mismatches, deletions or insertions between the first strand and the target sequence, or between the first strand and the second strand can be tolerated in the context of siRNA and even have the potential in certain cases to increase activity

The inhibition activity of the nucleic acids according to the present invention relies on the formation of a duplex region between all or a portion of the first strand and a portion of a target nucleic acid. The portion of the target nucleic acid that forms a duplex region with the first strand, defined as beginning with the first base pair formed between the first strand and the target sequence and ending with the last base pair formed between the first strand and the target sequence, inclusive, is the target nucleic acid sequence or simply, target sequence. The duplex region formed between the first strand and the second strand need not be the same as the duplex region formed between the first strand and the target sequence. That is, the second strand may have a sequence different from the target sequence; however, the first strand must be able to form a duplex structure with both the second strand and the target sequence, at least under physiological conditions.

The complementarity between the first strand and the target sequence may be perfect (no nucleotide mismatches or insertions or deletions in the first strand as compared to the target sequence).

The complementarity between the first strand and the target sequence may not be perfect. The complementarity may be from about 70% to about 100%. More specifically, the complementarity may be at least 70%, 80%, 85%, 90% or 95% and intermediate values.

The identity between the first strand and the complementary sequence of the target sequence may range from about 75% to about 100%. More specifically, the complementarity may be at least 75%, 80%, 85%, 90% or 95% and intermediate values, provided a nucleic acid is capable of reducing or inhibiting the expression of the complement component C3.

A nucleic acid having less than 100% complementarity between the first strand and the target sequence may be able to reduce the expression of the complement component C3 to the same level as a nucleic acid having perfect complementarity between the first strand and target sequence. Alternatively, it may be able to reduce expression of the complement component C3 to a level that is 15% - 100% of the level of reduction achieved by the nucleic acid with perfect complementarity.

In one aspect, the nucleic acid is a nucleic acid wherein
(a) the first strand sequence comprises a sequence differing by no more than 3 nucleotides from any one of the first strand sequences of Table 1 and optionally wherein the second strand sequence comprises a sequence differing by no more than 3 nucleotides from the second strand sequence in the same line of the table;
(b) the first strand sequence comprises a sequence differing by no more than 1 nucleotide from any one of the first strand sequences of Table 1 and optionally wherein the second strand sequence comprises a sequence differing by no more than 1 nucleotide from the second strand sequence in the same line of the table;
(c) the first strand sequence comprises a sequence of any one of the first strand sequences of Table 1 and optionally wherein the second strand sequence comprises a sequence of the second strand sequence in the same line of the table; or
(d) the first strand sequence consists of any one of the first strand sequences of Table 1 and optionally wherein the second strand sequence consists of the sequence of the second strand sequence in the same line of the table;
wherein Table 1 is:

**Table 1**

| **First strand sequence (SEQ ID NO:)** | **Second strand sequence (SEQ ID NO:)** |
|---|---|
| 361 | 112 |
| 95 | 96 |
| | 112 |
| 125 | 126 |
| 131 | 132 |
| 1 | 2 |
| 3 | 4 |
| 5 | 6 |
| 7 | 8 |
| 9 | 10 |
| 11 | 12 |
| 13 | 14 |
| 15 | 16 |
| 17 | 18 |
| 19 | 20 |
| 21 | 22 |
| 23 | 24 |
| 25 | 26 |
| 27 | 28 |
| 29 | 30 |
| 31 | 32 |
| 33 | 34 |
| 35 | 36 |
| 37 | 38 |
| 39 | 40 |
| 41 | 42 |
| 43 | 44 |
| 45 | 46 |
| 47 | 48 |
| 49 | 50 |
| 51 | 52 |
| 53 | 54 |
| 55 | 56 |
| 57 | 58 |
| 59 | 60 |
| 61 | 62 |
| 63 | 64 |
| 65 | 66 |
| 67 | 68 |
| 69 | 70 |
| 71 | 72 |
| 73 | 74 |
| 75 | 76 |
| 77 | 78 |
| 79 | 80 |
| 81 | 82 |
| 83 | 84 |
| 85 | 86 |
| 87 | 88 |
| 89 | 90 |
| 91 | 92 |
| 93 | 94 |
| 97 | 98 |
| 99 | 100 |
| 101 | 102 |
| 103 | 104 |
| 105 | 106 |
| 107 | 108 |
| 109 | 110 |
| 113 | 114 |
| 115 | 116 |
| 117 | 118 |
| 119 | 120 |
| 121 | 122 |
| 123 | 124 |
| 127 | 128 |
| 129 | 130 |
| 133 | 134 |

In one aspect, the nucleic acid is a nucleic acid wherein:
(a) the first strand sequence comprises the sequence of SEQ ID NO 361 and optionally wherein the second strand sequence comprises the sequence of SEQ ID NO: 112; or
(b) the first strand sequence comprises the sequence of SEQ ID NO 95 and optionally wherein the second strand sequence comprises the sequence of SEQ ID NO: 96; or
(c) the first strand sequence comprises the sequence of SEQ ID NO 111 and optionally wherein the second strand sequence comprises the sequence of SEQ ID NO: 112; or
(d) the first strand sequence comprises the sequence of SEQ ID NO 125 and optionally wherein the second strand sequence comprises the sequence of SEQ ID NO: 126; or
(e) the first strand sequence comprises the sequence of SEQ ID NO 131 and optionally wherein the second strand sequence comprises the sequence of SEQ ID NO: 132; or
(f) the first strand sequence consists of SEQ ID NO: 361 and optionally wherein the second strand sequence consists of SEQ ID NO: 112; or
(g) the first strand sequence consists of SEQ ID NO: 95 and optionally wherein the second strand sequence consists of SEQ ID NO: 96; or
(h) the first strand sequence consists of SEQ ID NO: 111 and optionally wherein the second strand sequence consists of SEQ ID NO: 112; or
(i) the first strand sequence consists of SEQ ID NO: 125 and optionally wherein the second strand sequence consists of SEQ ID NO: 126; or
(j) the first strand sequence consists of SEQ ID NO: 131 and optionally wherein the second strand sequence consists of SEQ ID NO: 132.

In one aspect, if the 5'-most nucleotide of the first strand is a nucleotide other than A or U, this nucleotide is replaced by A or U in the sequence. Preferably, if the 5'-most nucleotide of the first strand is a nucleotide other than U, this nucleotide is replaced by U, and more preferably by U with a 5' vinylphosphonate, in the sequence.

When a nucleic acid of the invention does not comprise the entire sequence of a reference first strand and/or second strand sequence as for example given in Table 1, or one or both strands differ from the corresponding reference sequence by one, two or three nucleotides, this nucleic acid preferably retains at least 30%, more preferably at least 50%, more preferably at least 70%, more preferably at least 80%, even more preferably at least 90%, yet more preferably at least 95% and most preferably 100% of the C3 inhibition activity compared to the inhibition activity of the corresponding nucleic acid that comprises the entire first strand and second strand reference sequences in a comparable experiment.

In one aspect, the nucleic acid is a nucleic acid wherein the first strand sequence comprises, or preferably consists of, the sequence of SEQ ID NO: 361 and optionally wherein the second strand sequence comprises, or preferably consists of, a sequence of at least 15, preferably at least 16, more preferably at least 17, yet more preferably at least 18 and most preferably all nucleotides of the sequence of SEQ ID NO: 112; or wherein the first strand sequence comprises, or preferably consists of, the sequence of SEQ ID NO: 95 and optionally wherein the second strand sequence comprises, or preferably consists of, a sequence of at least 15, preferably at least 16, more preferably at least 17, yet more preferably at least 18 and most preferably all nucleotides of the sequence of SEQ ID NO: 96; or wherein the first strand sequence comprises, or preferably consists of, the sequence of SEQ ID NO: 111 and optionally wherein the second strand sequence comprises, or preferably consists of, a sequence of at least 15, preferably at least 16, more preferably at least 17, yet more preferably at least 18 and most preferably all nucleotides of the sequence of SEQ ID NO: 112; or wherein the first strand sequence comprises, or preferably consists of, the sequence of SEQ ID NO: 125 and optionally wherein the second strand sequence comprises, or preferably consists of, a sequence of at least 15, preferably at least 16, more preferably at least 17, yet more preferably at least 18 and most preferably all nucleotides of the sequence of SEQ ID NO: 126; or wherein the first strand sequence comprises, or preferably consists of, the sequence of SEQ ID NO: 131 and optionally wherein the second strand sequence comprises, or preferably consists of, a sequence of at least 15, preferably at least 16, more preferably at least 17, yet more preferably at least 18 and most preferably all nucleotides of the sequence of SEQ ID NO: 132.

In one aspect, the nucleic acid is a double stranded nucleic acid for inhibiting expression of C3, preferably in a cell, wherein the nucleic acid comprises a first nucleic acid strand and a second nucleic acid strand, wherein the first strand is capable of hybridising under physiological conditions to a nucleic acid of sequence SEQ ID NO: 112, 96, 126, 132, 2, 4, 6, 8, 10, 12, 14, 16, 18, 20, 22, 24, 26, 28, 30, 32, 34, 36, 38, 40, 42, 44, 46, 48, 50, 52, 54, 56 ,58, 60, 62, 64 ,66, 68, 70, 72, 74, 76, 78, 80, 82, 84, 86, 88, 90, 92, 94, 98, 100, 102, 104, 106, 108, 110, 114, 116, 118, 120, 122, 124, 128, 130 or 134; and
wherein the second strand is capable of hybridising under physiological conditions to the first strand to form a duplex region.

Nucleic acids that are capable of hybridising under physiological conditions are nucleic acids that are capable of forming base pairs, preferably Watson-Crick or wobble base-pairs, between at least a portion of the opposed nucleotides in the strands so as to form at least a duplex region. Such a double-stranded nucleic acid is preferably a stable double-stranded nucleic acid under physiological conditions (for example in PBS at 37°C at a concentration of 1 µM of each strand), meaning that under such conditions, the two strands stay hybridised to each other. The Tm of the double-stranded nucleotide is preferably 45°C or more, preferably 50°C or more and more preferably 55°C or more.

One aspect relates to a nucleic acid for inhibiting expression of the complement component C3, wherein the nucleic acid comprises a first sequence of at least 15, preferably at least 16, more preferably at least 17, yet more preferably at least 18 and most preferably all nucleotides differing by no more than 3 nucleotides, preferably no more than 2 nucleotides, more preferably no more than 1 nucleotide and most preferably not differing by any nucleotide from any of the sequences of Table 5, the first sequence being able to hybridise to a target gene transcript (such as an mRNA) under physiological conditions. Preferably the nucleic acid further comprises a second sequence of at least 15, preferably at least 16, more preferably at least 17, yet more preferably at least 18 and most preferably all nucleotides differing by no more than 3 nucleotides, preferably no more than 2 nucleotides, more preferably no more than 1 nucleotide and most preferably not differing by any nucleotide from any of the sequences of Table 5, the second sequence being able to hybridise to the first sequence under physiological conditions and preferably the nucleic acid being an siRNA that is capable of inhibiting C3 expression via the RNAi pathway.

One aspect relates to any double-stranded nucleic acid as disclosed in Table 3 for inhibiting expression of the complement component C3. These nucleic acids are all siRNAs with various nucleotide modifications. Some of them are conjugates comprising GalNAc moieties that can be specifically targeted to cells with GalNAc receptors such as hepatocytes.

One aspect relates to a double-stranded nucleic acid that is capable of inhibiting expression of the complement component C3, preferably in a cell, for use as a medicament.

The nucleic acids described herein may be capable of inhibiting the expression of the complement component C3. Inhibition may be complete, i.e. 0% remaining expression compared of the expression level of C3 in the absence of the nucleic acid of the invention. Inhibition of C3 expression may be partial, i.e. it may be 15%, 20%, 30%, 40%, 50%, 60%, 70%, 75%, 80%, 85%, 90%, 95% or intermediate values of C3 expression in the absence of a nucleic acid of the invention. The level of inhibition may be measured by comparing a treated sample with an untreated sample or with a sample treated with a control such as for example a siRNA that does not target C3. Inhibition may be measured by measuring C3 mRNA and/or protein levels or levels of a biomarker or indicator that correlates with C3 presence or activity. It may be measured in cells that may have been treated *in vitro* with a nucleic acid described herein. Alternatively, or in addition, inhibition may be measured in cells, such as hepatocytes, or tissue, such as liver tissue, or an organ, such as the liver, or in a body fluid such as blood, serum, lymph or any other body part that has been taken from a subject previously treated with a nucleic acid disclosed herein. Preferably inhibition of C3 expression is determined by comparing the C3 mRNA level measured in C3-expressiong cells after 24 or 48 hours *in vitro* treatment under ideal conditions (see the examples for appropriate concentrations and conditions) with a double-stranded RNA disclosed herein to the C3 mRNA level measured in the same cells that were untreated or mock treated or treated with a control double stranded RNA.

One aspect relates to a nucleic acid, wherein the first strand and the second strand are present on a single strand of a nucleic acid that loops around so that the first strand and the second strand are able to hybridise to each other and to thereby form a double stranded nucleic acid with a duplex region.

Preferably, the first strand and the second strand of the nucleic acid are separate strands. The two separate strands are preferably each 17-25 nucleotides in length, more preferably 18-25 nucleotides in length. The two strands may be of the same or different lengths. The first strand may be 17-25 nucleotides in length, preferably it may be 18-24 nucleotides in length, it may be 18, 19, 20, 21, 22, 23 or 24 nucleotides in length. Most preferably, the first strand is 19 nucleotides in length. The second strand may independently be 17-25 nucleotides in length, preferably it may be 18-24 nucleotides in length, it may be 18, 19, 20, 21, 22, 23 or 24 nucleotides in length. More preferably, the second strand is 18 or 19 nucleotides in length, and most preferably it is 18 nucleotides in length.

Preferably, the first strand and the second strand of the nucleic acid form a duplex region of 17-25 nucleotides in length. More preferably, the duplex region is 18-24 nucleotides in length. The duplex region may be 17, 18, 19, 20, 21, 22, 23, 24 or 25 nucleotides in length. In the most preferred embodiment, the duplex region is 18 nucleotides in length. The duplex region is defined here as the region between and including the 5'-most nucleotide of the first strand that is base paired to a nucleotide of the second strand to the 3'-most nucleotide of the first strand that is base paired to a nucleotide of the second strand. The duplex region may comprise nucleotides in either or both strands that are not base-paired to a nucleotide in the other strand. It may comprise one, two three or four such nucleotides on the first strand and/or on the second strand. However, preferably, the duplex region consists of 17-25 consecutive nucleotide base pairs. That is to say that it preferably comprises 17-25 consecutive nucleotides on both of the strands that all base pair to a nucleotide in the other strand. More preferably, the duplex region consists of 18 or 19 consecutive nucleotide base pairs, most preferably 18.

The nucleic acid may be blunt ended at both ends; have an overhang at one end and a blunt end at the other end; or have an overhang at both ends.

The nucleic acid may have an overhang at one end and a blunt end at the other end. The nucleic acid may have an overhang at both ends. The nucleic acid may be blunt ended at both ends. The nucleic acid may be blunt ended at the end with the 5' end of the first strand and the 3' end of the second strand or at the 3' end of the first strand and the 5' end of the second strand.

The nucleic acid may comprise an overhang at a 3' or 5' end. The nucleic acid may have a 3' overhang on the first strand. The nucleic acid may have a 3' overhang on the second strand. The nucleic acid may have a 5' overhang on the first strand. The nucleic acid may have a 5' overhang on the second strand. The nucleic acid may have an overhang at both the 5' end and 3' end of the first strand. The nucleic acid may have an overhang at both the 5' end and 3' end of the second strand. The nucleic acid may have a 5' overhang on the first strand and a 3' overhang on the second strand. The nucleic acid may have a 3' overhang on the first strand and a 5' overhang on the second strand. The nucleic acid may have a 3' overhang on the first strand and a 3' overhang on the second strand. The nucleic acid may have a 5' overhang on the first strand and a 5' overhang on the second strand.

An overhang at the 3' end or 5' end of the second strand or the first strand may be selected from consisting of 1, 2, 3, 4 and 5 nucleotides in length. Optionally, an overhang may consist of 1 or 2 nucleotides, which may or may not be modified.

In one embodiment, the 5' end of the first strand is a single-stranded overhang of one, two or three nucleotides, preferably of one nucleotide.

Preferably, the nucleic acid is an siRNA. siRNAs are short interfering or short silencing RNAs that are able to inhibit the expression of a target gene through the RNA interference (RNAi) pathway. Inhibition occurs through targeted degradation of mRNA transcripts of the target gene after transcription. The siRNA forms part of the RISC complex. The RISC complex specifically targets the target RNA by sequence complementarity of the first (antisense) strand with the target sequence.

Preferably, the nucleic acid mediates RNA interference (RNAi). The nucleic acid, or at least the first strand of the nucleic acid, is therefore preferably able to be incorporated into the RISC complex. As a result, the nucleic acid, or at least the first strand of the nucleic acid, is therefore able to guide the RISC complex to a specific target RNA with which the nucleic acid, or at least the first strand of the nucleic acid, is at least partially complementary. The RISC complex then specifically cleaves this target RNA and as a result leads to inhibition of the expression of the gene from which the RNA stems.

### Nucleic acid modifications

Modifications of the nucleic acid of the present invention generally provide a powerful tool in overcoming potential limitations including, but not limited to, *in vitro* and *in vivo* stability and bioavailability inherent to native RNA molecules. The nucleic acids according to the invention may be modified by chemical modifications. Modified nucleic acids can also minimise the possibility of inducing interferon activity in humans. Modifications can further enhance the functional delivery of a nucleic acid to a target cell. The modified nucleic acids of the present invention may comprise one or more chemically modified ribonucleotides of either or both of the first strand or the second strand. A ribonucleotide may comprise a chemical modification of the base, sugar or phosphate moieties. The ribonucleic acid may be modified by substitution with or insertion of analogues of nucleic acids or bases.

Preferably, at least one nucleotide of the first and/or second strand of the nucleic acid is a modified nucleotide, preferably a non-naturally occurring nucleotide such as preferably a 2'-F modified nucleotide.

A modified nucleotide can be a nucleotide with a modification of the sugar group. The 2' hydroxyl group (OH) can be modified or replaced with a number of different "oxy" or "deoxy" substituents.

Examples of "oxy"-2' hydroxyl group modifications include alkoxy or aryloxy (OR, e.g., R=H, alkyl (such as methyl), cycloalkyl, aryl, aralkyl, heteroaryl or sugar); polyethyleneglycols (PEG), O(CH2CH2O)nCH2CH2OR; "locked" nucleic acids (LNA) in which the 2' hydroxyl is connected, e.g., by a methylene bridge, to the 4' carbon of the same ribose sugar; O-AMINE (AMINE=NH2; alkylamino, dialkylamino, heterocyclyl, arylamino, diaryl amino, heteroaryl amino, or diheteroaryl amino, ethylene diamine, polyamino) and aminoalkoxy, O(CH2)nAMINE, (e.g., AMINE=NH2; alkylamino, dialkylamino, heterocyclyl, arylamino, diaryl amino, heteroaryl amino, or diheteroaryl amino, ethylene diamine, polyamino).

"Deoxy" modifications include hydrogen, halogen, amino (e.g., NH2; alkylamino, dialkylamino, heterocyclyl, arylamino, diaryl amino, heteroaryl amino, diheteroaryl amino, or amino acid); NH(CH2CH2NH)nCH2CH2-AMINE (AMINE=NH2; alkylamino, dialkylamino, heterocyclyl, arylamino, diaryl amino, heteroaryl amino, or diheteroaryl amino), -NHC(O)R (R=alkyl, cycloalkyl, aryl, aralkyl, heteroaryl or sugar), cyano; mercapto; alkyl-thio-alkyl; thioalkoxy; and alkyl, cycloalkyl, aryl, alkenyl and alkynyl, which may be optionally substituted with e.g., an amino functionality. Other substituents of certain embodiments include 2'-methoxyethyl, 2'-OCH3, 2'-O-allyl, 2'-C-allyl, and 2'-fluoro.

The sugar group can also contain one or more carbons that possess the opposite stereochemical configuration than that of the corresponding carbon in ribose. Thus, a modified nucleotide may contain a sugar such as arabinose.

Modified nucleotides can also include "abasic" sugars, which lack a nucleobase at C - 1'. These abasic sugars can further contain modifications at one or more of the constituent sugar atoms.

The 2' modifications may be used in combination with one or more phosphate linker modifications (e.g., phosphorothioate).

One or more nucleotides of a nucleic acid of the present invention may be modified. The nucleic acid may comprise at least one modified nucleotide. The modified nucleotide may be in the first strand. The modified nucleotide may be in the second strand. The modified nucleotide may be in the duplex region. The modified nucleotide may be outside the duplex region, i.e., in a single stranded region. The modified nucleotide may be on the first strand and may be outside the duplex region. The modified nucleotide may be on the second strand and may be outside the duplex region. The 3'-terminal nucleotide of the first strand may be a modified nucleotide. The 3'-terminal nucleotide of the second strand may be a modified nucleotide. The 5'-terminal nucleotide of the first strand may be a modified nucleotide. The 5'-terminal nucleotide of the second strand may be a modified nucleotide.

A nucleic acid of the invention may have 1 modified nucleotide or a nucleic acid of the invention may have about 2-4 modified nucleotides, or a nucleic acid may have about 4-6 modified nucleotides, about 6-8 modified nucleotides, about 8-10 modified nucleotides, about 10-12 modified nucleotides, about 12-14 modified nucleotides, about 14-16 modified nucleotides about 16-18 modified nucleotides, about 18-20 modified nucleotides, about 20-22 modified nucleotides, about 22-24 modified nucleotides, 24-26 modified nucleotides or about 26-28 modified nucleotides. In each case the nucleic acid comprising said modified nucleotides retains at least 50% of its activity as compared to the same nucleic acid but without said modified nucleotides or vice versa. The nucleic acid may retain 55%, 60%, 65%, 70%, 75%, 80%, 85%, 90%, 95% or 100% and intermediate values of its activity as compared to the same nucleic acid but without said modified nucleotides, or may have more than 100% of the activity of the same nucleic acid without said modified nucleotides.

The modified nucleotide may be a purine or a pyrimidine. At least half of the purines may be modified. At least half of the pyrimidines may be modified. All of the purines may be modified. All of the pyrimidines may be modified. The modified nucleotides may be selected from the group consisting of a 3' terminal deoxy thymine (dT) nucleotide, a 2'-O-methyl (2'-OMe) modified nucleotide, a 2' modified nucleotide, a 2' deoxy modified nucleotide, a locked nucleotide, an abasic nucleotide, a 2' amino modified nucleotide, a 2' alkyl modified nucleotide, a 2'-deoxy-2'-fluoro (2'-F) modified nucleotide, a morpholino nucleotide, a phosphoramidate, a non-natural base comprising nucleotide, a nucleotide comprising a 5'-phosphorothioate group, a nucleotide comprising a 5' phosphate or 5' phosphate mimic and a terminal nucleotide linked to a cholesteryl derivative or a dodecanoic acid bisdecylamide group.

The nucleic acid may comprise a nucleotide comprising a modified base, wherein the base is selected from 2-aminoadenosine, 2,6-diaminopurine,inosine, pyridin-4-one, pyridin-2-one, phenyl, pseudouracil, 2, 4, 6-trimethoxy benzene, 3-methyl uracil, dihydrouridine, naphthyl, aminophenyl, 5-alkylcytidine (e.g., 5-methylcytidine), 5-alkyluridine (e.g., ribothymidine), 5-halouridine (e.g., 5-bromouridine), 6-azapyrimidine, 6-alkylpyrimidine (e.g. 6-methyluridine), propyne, quesosine, 2-thiouridine, 4-thiouridine, wybutosine, wybutoxosine, 4-acetylcytidine, 5-(carboxyhydroxymethyl)uridine, 5'-carboxymethylaminomethyl-2-thiouridine, 5-carboxymethylaminomethyluridine, beta-D-galactosylqueosine, 1-methyladenosine, 1-methylinosine, 2,2-dimethylguanosine, 3-methylcytidine, 2-methyladenosine, 2-methylguanosine, N6-methyladenosine, 7-methylguanosine, 5-methoxyaminomethyl-2-thiouridine, 5-methylaminomethyluridine, 5-methylcarbonylmethyluridine, 5-methyloxyuridine, 5-methyl-2-thiouridine, 2-methylthio-N6-isopentenyladenosine, beta-D-mannosylqueosine, uridine-5-oxyacetic acid and 2-thiocytidine.

Nucleic acids discussed herein include unmodified RNA as well as RNA which has been modified, e.g., to improve efficacy or stability. Unmodified RNA refers to a molecule in which the components of the nucleic acid, namely sugars, bases, and phosphate moieties, are the same or essentially the same as those which occur in nature, for example as occur naturally in the human body. The term "modified nucleotide" as used herein refers to a nucleotide in which one or more of the components of the nucleotide, namely the sugar, base, and phosphate moiety, is/are different from those which occur in nature. The term "modified nucleotide" also refers in certain cases to molecules that are not nucleotides in the strict sense of the term because they lack, or have a substitute of, an essential component of a nucleotide, such as the sugar, base or phosphate moiety. A nucleic acid comprising such modified nucleotides is still to be understood as being a nucleic acid, even if one or more of the nucleotides of the nucleic acid has been replaced by a modified nucleotide that lacks, or has a substitution of, an essential component of a nucleotide.

Many of the modifications described herein and that occur within a nucleic acid will be repeated within a polynucleotide molecule, such as a modification of a base, or a phosphate moiety, or a non-linking O of a phosphate moiety. In some cases, the modification will occur at all of the possible positions/nucleotides in the polynucleotide but in many cases it will not. A modification may only occur at a 3' or 5' terminal position, may only occur in a terminal region, such as at a position on a terminal nucleotide or in the last 2, 3, 4, 5, or 10 nucleotides of a strand. A modification may occur in a double strand region, a single strand region, or in both. A modification may occur only in the double strand region of a nucleic acid of the invention or may only occur in a single strand region of a nucleic acid of the invention. A phosphorothioate modification at a non-linking O position may only occur at one or both termini, may only occur in a terminal region, e.g., at a position on a terminal nucleotide or in the last 2, 3, 4 or 5 nucleotides of a strand, or may occur in duplex and/or in single strand regions, particularly at termini. The 5' end and/or 3' end may be phosphorylated.

Stability of a nucleic acid of the invention may be increased by including particular bases in overhangs, or by including modified nucleotides, in single strand overhangs, e.g., in a 5' or 3' overhang, or in both. Purine nucleotides may be included in overhangs. All or some of the bases in a 3' or 5' overhang may be modified. Modifications can include the use of modifications at the 2' OH group of the ribose sugar, the use of deoxyribonucleotides, instead of ribonucleotides, and modifications in the phosphate group, such as phosphorothioate modifications. Overhangs need not be homologous with the target sequence.

Nucleases can hydrolyse nucleic acid phosphodiester bonds. However, chemical modifications to nucleic acids can confer improved properties, and, can render oligoribonucleotides more stable to nucleases.

Modified nucleic acids, as used herein, can include one or more of:
(i) alteration, e.g., replacement, of one or both of the non-linking phosphate oxygens and/or of one or more of the linking phosphate oxygens (referred to as linking even if at the 5' and 3' terminus of the nucleic acid of the invention);
(ii) alteration, e.g., replacement, of a constituent of the ribose sugar, e.g., of the 2' hydroxyl on the ribose sugar;
(iii) replacement of the phosphate moiety with "dephospho" linkers;
(iv) modification or replacement of a naturally occurring base;
(v) replacement or modification of the ribose-phosphate backbone; and
(vi) modification of the 3' end or 5' end of the first strand and/or the second strand, e.g., removal, modification or replacement of a terminal phosphate group or conjugation of a moiety, e.g., a fluorescently labelled moiety, to either the 3' or 5' end one or both strands.

The terms replacement, modification, alteration, indicate a difference from a naturally occurring molecule.

Specific modifications are discussed in more detail below.

The nucleic acid may comprise one or more nucleotides on the second and/or first strands that are modified. Alternating nucleotides may be modified, to form modified nucleotides.

Alternating as described herein means to occur one after another in a regular way. In other words, alternating means to occur in turn repeatedly. For example, if one nucleotide is modified, the next contiguous nucleotide is not modified and the following contiguous nucleotide is modified and so on. One nucleotide may be modified with a first modification, the next contiguous nucleotide may be modified with a second modification and the following contiguous nucleotide is modified with the first modification and so on, where the first and second modifications are different.

Some representative modified nucleic acid sequences of the present invention are shown in the examples. These examples are meant to be representative and not limiting.

In one aspect of the nucleic acid, at least nucleotides 2 and 14 of the first strand are modified, preferably by a first common modification, the nucleotides being numbered consecutively starting with nucleotide number 1 at the 5' end of the first strand. The first modification is preferably 2'-F.

In one aspect, at least one, several or preferably all the even-numbered nucleotides of the first strand are modified, preferably by a first common modification, the nucleotides being numbered consecutively starting with nucleotide number 1 at the 5' end of the first strand. The first modification is preferably 2'-F.

In one aspect, at least one, several or preferably all the odd-numbered nucleotides of the first strand are modified, the nucleotides being numbered consecutively starting with nucleotide number 1 at the 5' end of the first strand. Preferably, they are modified by a second modification. This second modification is preferably different from the first modification if the nucleic acid also comprises a first modification, for example of nucleotides 2 and 14 or of all the even-numbered nucleotides of the first strand. The first modification is preferably 2'-F and the second modification is preferably 2'-OMe.

In one aspect, at least one, several or preferably all the nucleotides of the second strand in a position corresponding to an even-numbered nucleotide of the first strand are modified, preferably by a third modification. Preferably in the same nucleic acid nucleotides 2 and 14 or all the even numbered nucleotides of the first strand are modified with a first modification. In addition, or alternatively, the odd-numbered nucleotides of the first strand are modified with a second modification. Preferably, the third modification is different from the first modification and/or the third modification is the same as the second modification. The first modification is preferably 2'-F and the second and third modifications are preferably 2'-OMe. The nucleotides on the first strand are numbered consecutively starting with nucleotide number 1 at the 5' end of the first strand.

A nucleotide of the second strand that is in a position corresponding for example to an even-numbered nucleotide of the first strand is a nucleotide of the second strand that is base-paired to an even-numbered nucleotide of the first strand.

In one aspect, at least one, several or preferably all the nucleotides of the second strand in a position corresponding to an odd-numbered nucleotide of the first strand are modified, preferably by a fourth modification. Preferably in the same nucleic acid nucleotides 2 and 14 or all the even numbered nucleotides of the first strand are modified with a first modification. In addition, or alternatively, the odd-numbered nucleotides of the first strand are modified with a second modification. In addition, or alternatively, all the nucleotides of the second strand in a position corresponding to an even-numbered nucleotide of the first strand are modified with a third modification. The fourth modification is preferably different from the second modification and preferably different from the third modification and the fourth modification is preferably the same as the first modification. The first and the fourth modification are preferably a 2'-OMe modification and the second and third modification are preferably a 2'-F modification. The nucleotides on the first strand are numbered consecutively starting with nucleotide number 1 at the 5' end of the first strand.

In one aspect of the nucleic acid, the nucleotide/nucleotides of the second strand in a position corresponding to nucleotide 11 or nucleotide 13 or nucleotides 11 and 13 or nucleotides 11-13 of the first strand is/are modified by a fourth modification. Preferably, all the nucleotides of the second strand other than the nucleotide/nucleotides in a position corresponding to nucleotide 11 or nucleotide 13 or nucleotides 11 and 13 or nucleotides 11-13 of the first strand is/are modified by a third modification. Preferably in the same nucleic acid nucleotides 2 and 14 or all the even numbered nucleotides of the first strand are modified with a first modification. In addition, or alternatively, the odd-numbered nucleotides of the first strand are modified with a second modification. The fourth modification is preferably different from the second modification and preferably different from the third modification and the fourth modification is preferably the same as the first modification. The first and the fourth modification are preferably a 2'-OMe modification and the second and third modification are preferably a 2'-F modification. The nucleotides on the first strand are numbered consecutively starting with nucleotide number 1 at the 5' end of the first strand.

In one aspect of the nucleic acid, all the even-numbered nucleotides of the first strand are modified by a first modification, all the odd-numbered nucleotides of the first strand are modified by a second modification, all the nucleotides of the second strand in a position corresponding to an even-numbered nucleotide of the first strand are modified by a third modification, all the nucleotides of the second strand in a position corresponding to an odd-numbered nucleotide of the first strand are modified by a fourth modification, wherein the first and fourth modification are 2'-F and the second and third modification are 2'-OMe.

In one aspect of the nucleic acid, all the even-numbered nucleotides of the first strand are modified by a first modification, all the odd-numbered nucleotides of the first strand are modified by a second modification, all the nucleotides of the second strand in positions corresponding to nucleotides 11-13 of the first strand are modified by a fourth modification, all the nucleotides of the second strand other than the nucleotides corresponding to nucleotides 11-13 of the first strand are modified by a third modification, wherein the first and fourth modification are 2'-F and the second and third modification are 2'-OMe. Preferably in this aspect, the 3' terminal nucleotide of the second strand is an inverted RNA nucleotide (ie the nucleotide is linked to the 3' end of the strand through its 3' carbon, rather than through its 5' carbon as would normally be the case). When the 3' terminal nucleotide of the second strand is an inverted RNA nucleotide, the inverted RNA nucleotide is preferably an unmodified nucleotide in the sense that it does not comprise any modifications compared to the natural nucleotide counterpart. Specifically, the inverted RNA nucleotide is preferably a 2'-OH nucleotide. Preferably, in this aspect when the 3' terminal nucleotide of the second strand is an inverted RNA nucleotide, the nucleic acid is blunt-ended at least at the end that comprises the 5' end of the first strand.

One aspect of the invention is a nucleic acid as disclosed herein for inhibiting expression of the C3 gene, preferably in a cell, wherein said first strand includes modified nucleotides or unmodified nucleotides at a plurality of positions in order to facilitate processing of the nucleic acid by RISC.

In one aspect "facilitate processing by RISC" means that the nucleic acid can be processed by RISC, for example any modification present will permit the nucleic acid to be processed by RISC, suitably such that siRNA activity can take place.

A nucleic acid as disclosed herein, wherein the nucleotides at positions 2 and 14 from the 5' end of the first strand are not modified with a 2' O-methyl modification, and the nucleotide/nucleotides on the second strand which corresponds to position 11 or position 13 or positions 11 and 13 or positions 11, 12 and 13 of the first strand is/are not modified with a 2'-OMe modification (in other words, they are not modified or are modified with a modification other than 2'-OMe).

In one aspect the nucleotide on the second strand which corresponds to position 13 of the first strand is the nucleotide that forms a base pair with position 13 of the first strand.

In one aspect the nucleotide on the second strand which corresponds to position 11 of the first strand is the nucleotide that forms a base pair with position 11 of the first strand.

In one aspect the nucleotide on the second strand which corresponds to position 12 of the first strand is the nucleotide that forms a base pair with position 12 of the first strand.

For example, in a 19-mer nucleic acid which is double stranded and blunt ended, position 13 of the first strand would pair with position 7 of the second strand. Position 11 of the first strand would pair with position 9 of the second strand. This nomenclature may be applied to other positions of the second strand.

In one aspect, in the case of a partially complementary first and second strand, the nucleotide on the second strand that "corresponds to" a position on the first strand may not necessarily form a base pair if that position is the position in which there is a mismatch, but the principle of the nomenclature still applies.

One aspect is a nucleic acid as disclosed herein, wherein the nucleotides at positions 2 and 14 from the 5' end of the first strand are not modified with a 2'-OMe modification, and the nucleotides on the second strand which correspond to position 11, or 13, or 11 and 13, or 11-13 of the first strand are modified with a 2'-F modification.

One aspect is a nucleic acid as disclosed herein, wherein the nucleotides at positions 2 and 14 from the 5' end of the first strand are modified with a 2'-F modification, and the nucleotides on the second strand which correspond to position 11, or 13, or 11 and 13, or 11-13 of the first strand are not modified with a 2'-OMe modification.

One aspect is a nucleic acid as disclosed herein, wherein the nucleotides at positions 2 and 14 from the 5' end of the first strand are modified with a 2'-F modification, and the nucleotides on the second strand which correspond to position 11, or 13, or 11 and 13, or 11-13 of the first strand are modified with a 2'-F modification.

One aspect is a nucleic acid as disclosed herein wherein greater than 50% of the nucleotides of the first and/or second strand comprise a 2'-OMe modification, such as greater than 55%, 60%, 65%, 70%, 75%, 80%, or 85%, or more, of the first and/or second strand comprise a 2'-OMe modification, preferably measured as a percentage of the total nucleotides of both the first and second strands.

One aspect is a nucleic acid as disclosed herein wherein greater than 50% of the nucleotides of the first and/or second strand comprise a naturally occurring RNA modification, such as wherein greater than 55%, 60%, 65%, 70%, 75%, 80%, or 85% or more of the first and/or second strands comprise such a modification, preferably measured as a percentage of the total nucleotides of both the first and second strands. Suitable naturally occurring modifications include, as well as 2'-OMe, other 2' sugar modifications, in particular a 2'-H modification resulting in a DNA nucleotide.

One aspect is a nucleic acid as disclosed herein comprising no more than 20%, such as no more than 15% such as no more than 10%, of nucleotides which have 2' modifications that are not 2'-OMe modifications on the first and/or second strand, preferably as a percentage of the total nucleotides of both the first and second strands.

One aspect is a nucleic acid as disclosed herein comprising no more than 20%, (such as no more than 15% or no more than 10%) of 2'-F modifications on the first and/or second strand, preferably as a percentage of the total nucleotides of both strands.

One aspect is a nucleic acid as disclosed herein, wherein all nucleotides are modified with a 2'-OMe modification except positions 2 and 14 from the 5' end of the first strand and the nucleotides on the second strand which correspond to position 11, or 13, or 11 and 13, or 11-13 of the first strand. Preferably the nucleotides that are not modified with 2'-OMe are modified with fluoro at the 2' position (2'-F modification).

Preferred is a nucleic acid as disclosed herein wherein all nucleotides of the nucleic acid are modified at the 2' position of the sugar. Preferably these nucleotides are modified with a 2'-F modification where the modification is not a 2'-OMe modification.

In one aspect the nucleic acid is modified on the first strand with alternating 2'-OMe modifications and 2-F modifications, and positions 2 and 14 (starting from the 5' end) are modified with 2'-F. Preferably the second strand is modified with 2'-F modifications at nucleotides on the second strand which correspond to position 11, or 13, or 11 and 13, or 11-13 of the first strand. Preferably the second strand is modified with 2'-F modifications at positions 11-13 counting from the 3' end starting at the first position of the complementary (double stranded) region, and the remaining modifications are naturally occurring modifications, preferably 2'-OMe.

In one aspect of the nucleic acid, each of the nucleotides of the first strand and of the second strand is a modified nucleotide.

The term "odd numbered" as described herein means a number not divisible by two. Examples of odd numbers are 1, 3, 5, 7, 9, 11 and so on. One or more of the even numbered nucleotides of the first strand of the nucleic acid of the invention may be modified, wherein the first strand is numbered 5' to 3'. The term "even numbered" as described herein means a number which is evenly divisible by two. Examples of even numbers are 2, 4, 6, 8, 10, 12, 14 and so on.

Herein the nucleotides of the first strand are numbered contiguously starting with nucleotide number 1 at the 5' end of the first strand. Nucleotides of the second strand are numbered contiguously starting with nucleotide number 1 at the 3' end of the second strand.

One or more nucleotides on the first and/or second strand may be modified, to form modified nucleotides. One or more of the odd-numbered nucleotides of the first strand may be modified. One or more of the even-numbered nucleotides of the first strand may be modified by at least a second modification, wherein the at least second modification is different from the modification on the one or more odd nucleotides. At least one of the one or more modified even numbered-nucleotides may be adjacent to at least one of the one or more modified odd-numbered nucleotides.

A plurality of odd-numbered nucleotides in the first strand may be modified in the nucleic acid of the invention. A plurality of even-numbered nucleotides in the first strand may be modified by a second modification. The first strand may comprise adjacent nucleotides that are modified by a common modification. The first strand may also comprise adjacent nucleotides that are modified by a second different modification.

One or more of the odd-numbered nucleotides of the second strand (wherein the nucleotides are numbered contiguously starting with nucleotide number 1 at the 3' end of the second strand) may be modified by a modification that is different to the modification of the odd-numbered nucleotides on the first strand (wherein the nucleotides are numbered contiguously starting with nucleotide number 1 at the 5' end of the first strand) and/or one or more of the even-numbered nucleotides of the second strand may be modified by the same modification of the odd-numbered nucleotides of the first strand. At least one of the one or more modified even-numbered nucleotides of the second strand may be adjacent to the one or more modified odd-numbered nucleotides. A plurality of odd-numbered nucleotides of the second strand may be modified by a common modification and/or a plurality of even-numbered nucleotides may be modified by the same modification that is present on the first stand odd-numbered nucleotides. A plurality of odd-numbered nucleotides on the second strand may be modified by a modification that is different from the modification of the first strand odd-numbered nucleotides.

The second strand may comprise adjacent nucleotides that are modified by a common modification, which may be a modification that is different from the modification of the odd-numbered nucleotides of the first strand.

In the nucleic acid of the invention, each of the odd-numbered nucleotides in the first strand and each of the even-numbered nucleotides in the second strand may be modified with a common modification and, each of the even-numbered nucleotides may be modified in the first strand with a different modification and each of the odd-numbered nucleotides may be modified in the second strand with the different modification.

The nucleic acid of the invention may have the modified nucleotides of the first strand shifted by at least one nucleotide relative to the unmodified or differently modified nucleotides of the second strand.

One or more or each of the odd numbered-nucleotides may be modified in the first strand and one or more or each of the even-numbered nucleotides may be modified in the second strand. One or more or each of the alternating nucleotides on either or both strands may be modified by a second modification. One or more or each of the even-numbered nucleotides may be modified in the first strand and one or more or each of the even-numbered nucleotides may be modified in the second strand. One or more or each of the alternating nucleotides on either or both strands may be modified by a second modification. One or more or each of the odd-numbered nucleotides may be modified in the first strand and one or more of the odd-numbered nucleotides may be modified in the second strand by a common modification. One or more or each of the alternating nucleotides on either or both strands may be modified by a second modification. One or more or each of the even-numbered nucleotides may be modified in the first strand and one or more or each of the odd-numbered nucleotides may be modified in the second strand by a common modification. One or more or each of the alternating nucleotides on either or both strands may be modified by a second modification.

The nucleic acid of the invention may comprise single or double-stranded constructs that comprise at least two regions of alternating modifications in one or both of the strands. These alternating regions can comprise up to about 12 nucleotides but preferably comprise from about 3 to about 10 nucleotides. The regions of alternating nucleotides may be located at the termini of one or both strands of the nucleic acid of the invention. The nucleic acid may comprise from 4 to about 10 nucleotides of alternating nucleotides at each termini (3' and 5') and these regions may be separated by from about 5 to about 12 contiguous unmodified or differently or commonly modified nucleotides.

The odd numbered nucleotides of the first strand may be modified and the even numbered nucleotides may be modified with a second modification. The second strand may comprise adjacent nucleotides that are modified with a common modification, which may be the same as the modification of the odd-numbered nucleotides of the first strand. One or more nucleotides of the second strand may also be modified with the second modification. One or more nucleotides with the second modification may be adjacent to each other and to nucleotides having a modification that is the same as the modification of the odd-numbered nucleotides of the first strand. The first strand may also comprise phosphorothioate linkages between the two nucleotides at the 3' end and at the 5' end. The second strand may comprise a phosphorothioate linkage between the two nucleotides at the 5' end. The second strand may also be conjugated to a ligand at the 5' end.

The nucleic acid of the invention may comprise a first strand comprising adjacent nucleotides that are modified with a common modification. One or more such nucleotides may be adjacent to one or more nucleotides which may be modified with a second modification. One or more nucleotides with the second modification may be adjacent. The second strand may comprise adjacent nucleotides that are modified with a common modification, which may be the same as one of the modifications of one or more nucleotides of the first strand. One or more nucleotides of the second strand may also be modified with the second modification. One or more nucleotides with the second modification may be adjacent. The first strand may also comprise phosphorothioate linkages between the two nucleotides at the 5' end and at the 3' end. The second strand may comprise a phosphorothioate linkage between the two nucleotides at the 3' end. The second strand may also be conjugated to a ligand at the 5' end.

The nucleotides numbered from 5' to 3' on the first strand and 3' to 5' on the second strand, 1, 3, 5, 7, 9, 11, 13, 15, 17, 19, 21, 23 and 25 may be modified by a modification on the first strand. The nucleotides numbered 2, 4, 6, 8, 10, 12, 14, 16, 18, 20, 22 and 24 may be modified by a second modification on the first strand. The nucleotides numbered 1, 3, 5, 7, 9, 11, 13, 15, 17, 19, 21, 23 may be modified by a modification on the second strand. The nucleotides numbered 2, 4, 6, 8, 10, 12, 14, 16, 18, 20, 22 and 24 may be modified by a second modification on the second strand. Nucleotides are numbered for the sake of the nucleic acid of the present invention from 5' to 3' on the first strand and 3' to 5' on the second strand.

The nucleotides numbered 2, 4, 6, 8, 10, 12, 14, 16, 18, 20, 22 and 24 may be modified by a modification on the first strand. The nucleotides numbered 1, 3, 5, 7, 9, 11, 13, 15, 17, 19, 21, 23 may be modified by a second modification on the first strand. The nucleotides numbered 1, 3, 5, 7, 9, 11, 13, 15, 17, 19, 21, 23 may be modified by a modification on the second strand. The nucleotides numbered 2, 4, 6, 8, 10, 12, 14, 16, 18, 20, 22 and 24 may be modified by a second modification on the second strand.

Clearly, if the first and/or the second strand are shorter than 25 nucleotides in length, such as 19 nucleotides in length, there are no nucleotides numbered 20, 21, 22, 23, 24 and 25 to be modified. The skilled person understands the description above to apply to shorter strands, accordingly.

One or more modified nucleotides on the first strand may be paired with modified nucleotides on the second strand having a common modification. One or more modified nucleotides on the first strand may be paired with modified nucleotides on the second strand having a different modification. One or more modified nucleotides on the first strand may be paired with unmodified nucleotides on the second strand. One or more modified nucleotides on the second strand may be paired with unmodified nucleotides on the first strand. In other words, the alternating nucleotides can be aligned on the two strands such as, for example, all the modifications in the alternating regions of the second strand are paired with identical modifications in the first strand or alternatively the modifications can be offset by one nucleotide with the common modifications in the alternating regions of one strand pairing with dissimilar modifications (i.e. a second or further modification) in the other strand. Another option is to have dissimilar modifications in each of the strands.

The modifications on the first strand may be shifted by one nucleotide relative to the modified nucleotides on the second strand, such that common modified nucleotides are not paired with each other.

The modification and/or modifications may each and individually be selected from the group consisting of 3' terminal deoxy thymine, 2'-OMe, a 2' deoxy modification, a 2' amino modification, a 2' alkyl modification, a morpholino modification, a phosphoramidate modification, 5'-phosphorothioate group modification, a 5' phosphate or 5' phosphate mimic modification and a cholesteryl derivative or a dodecanoic acid bisdecylamide group modification and/or the modified nucleotide may be any one of a locked nucleotide, an abasic nucleotide or a non natural base comprising nucleotide.

At least one modification may be 2'-OMe and/or at least one modification may be 2'-F. Further modifications as described herein may be present on the first and/or second strand.

The nucleic acid of the invention may comprise an inverted RNA nucleotide at one or several of the strand ends. Such inverted nucleotides provide stability to the nucleic acid. Preferably, the nucleic acid comprises at least an inverted nucleotide at the 3' end of the first and/or the second strand and/or at the 5' end of the second strand. More preferably, the nucleic acid comprises an inverted nucleotide at the 3' end of the second strand. Most preferably, the nucleic acid comprises an inverted RNA nucleotide at the 3' end of the second strand and this nucleotide is preferably an inverted A. An inverted nucleotide is a nucleotide that is linked to the 3' end of a nucleic acid through its 3' carbon, rather than its 5' carbon as would normally be the case or is linked to the 5' end of a nucleic acid through its 5' carbon, rather than its 3' carbon as would normally be the case. The inverted nucleotide is preferably present at an end of a strand not as an overhang but opposite a corresponding nucleotide in the other strand. Accordingly, the nucleic acid is preferably blunt-ended at the end that comprises the inverted RNA nucleotide. An inverted RNA nucleotide being present at the end of a strand preferably means that the last nucleotide at this end of the strand is the inverted RNA nucleotide. A nucleic acid with such a nucleotide is stable and easy to synthesise. The inverted RNA nucleotide is preferably an unmodified nucleotide in the sense that it does not comprise any modifications compared to the natural nucleotide counterpart. Specifically, the inverted RNA nucleotide is preferably a 2'-OH nucleotide.

Nucleic acids of the invention may comprise one or more nucleotides modified at the 2' position with a 2'-H, and therefore having a DNA nucleotide within the nucleic acid. Nucleic acids of the invention may comprise DNA nucleotides at positions 2 and/or 14 of the first strand counting from the 5' end of the first strand. Nucleic acids may comprise DNA nucleotides on the second strand which correspond to position 11, or 13, or 11 and 13, or 11-13 of the first strand.

In one aspect there is no more than one DNA nucleotide per nucleic acid of the invention.

Nucleic acids of the invention may comprise one or more LNA nucleotides. Nucleic acids of the invention may comprise LNA nucleotides at positions 2 and/or 14 of the first strand counting from the 5' end of the first strand. Nucleic acids may comprise LNA on the second strand which correspond to position 11, or 13, or 11 and 13, or 11-13 of the first strand.

Throughout the description of the invention, "same or common modification" means the same modification to any nucleotide, be that A, G, C or U modified with a group such as a methyl group (2'-OMe) or a fluoro group (2'-F). For example, 2'-F-dU, 2'-F-dA, 2'-F-dC, 2'-F-dG are all considered to be the same or common modification, as are 2'-OMe-rU, 2'-OMe-rA; 2'-OMe-rC; 2'-OMe-rG. A 2'-F modification is a different modification to a 2'-OMe modification.

Some representative modified nucleic acid sequences of the present invention are shown in the examples. These examples are meant to be representative and not limiting.

Preferably, the nucleic acid may comprise a modification and a second or further modification which are each and individually selected from the group comprising 2'-OMe modification and 2'-F modification. The nucleic acid may comprise a modification that is 2'-OMe that may be a first modification, and a second modification that is 2'-F. The nucleic acid of the invention may also include a phosphorothioate modification and/or a deoxy modification which may be present in or between the terminal 2 or 3 nucleotides of each or any end of each or both strands.

In one aspect of the nucleic acid, at least one nucleotide of the first and/or second strand is a modified nucleotide, wherein if the first strand comprises at least one modified nucleotide:
(i) at least one or both of the nucleotides 2 and 14 of the first strand is/are modified by a first modification; and/or
(ii) at least one, several, or all the even-numbered nucleotides of the first strand is/are modified by a first modification; and/or
(iii) at least one, several, or all the odd-numbered nucleotides of the first strand is/are modified by a second modification; and/or
wherein if the second strand comprises at least one modified nucleotide:
(iv) at least one, several, or all the nucleotides of the second strand in a position corresponding to an even-numbered nucleotide of the first strand is/are modified by a third modification; and/or
(v) at least one, several, or all the nucleotides of the second strand in a position corresponding to an odd-numbered nucleotide of the first strand is/are modified by a fourth modification; and/or
(vi) at least one, several, or all the nucleotides of the second strand in a position corresponding to nucleotide 11 or nucleotide 13 or nucleotides 11 and 13 or nucleotides 11-13 of the first strand is/are modified by a fourth modification; and/or
(vii) at least one, several, or all the nucleotides of the second strand in a position other than the position corresponding to nucleotide 11 or nucleotide 13 or nucleotides 11 and 13 or nucleotides 11-13 of the first strand is/are modified by a third modification;
wherein the nucleotides on the first strand are numbered consecutively starting with nucleotide number 1 at the 5' end of the first strand;
wherein the modifications are preferably at least one of the following:
(a) the first modification is preferably different from the second and from the third modification;
(b) the first modification is preferably the same as the fourth modification;
(c) the second and the third modification are preferably the same modification;
(d) the first modification is preferably a 2'-F modification;
(e) the second modification is preferably a 2'-OMe modification;
(f) the third modification is preferably a 2'-OMe modification; and/or
(g) the fourth modification is preferably a 2'-F modification; and
wherein optionally the nucleic acid is conjugated to a ligand.

One aspect is a double-stranded nucleic acid for inhibiting expression of C3, preferably in a cell, wherein the nucleic acid comprises a first strand and a second strand, wherein the first strand sequence comprises a sequence of at least 15 nucleotides differing by no more than 3 nucleotides from any one of the sequences SEQ ID NO: 361, 95, 111, 125, 131, 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 97, 99, 101, 103, 105, 107, 109, 113, 115, 117, 119, 121, 123, 127, 129 or 133, preferably SEQ ID NO: 361, 95, 111, 125 or 131, wherein all the even-numbered nucleotides of the first strand are modified by a first modification, all the odd-numbered nucleotides of the first strand are modified by a second modification, all the nucleotides of the second strand in a position corresponding to an even-numbered nucleotide of the first strand are modified by a third modification, all the nucleotides of the second strand in a position corresponding to an odd-numbered nucleotide of the first strand are modified by a fourth modification, wherein the first and fourth modification are 2'-F and the second and third modification are 2'-OMe.

One aspect is a double-stranded nucleic acid for inhibiting expression of C3, preferably in a cell, wherein the nucleic acid comprises a first strand and a second strand, wherein the first strand sequence comprises a sequence of at least 15 nucleotides differing by no more than 3 nucleotides from any one of the sequences SEQ ID NO: 361, 95, 111, 125, 131, 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 97, 99, 101, 103, 105, 107, 109, 113, 115, 117, 119, 121, 123, 127, 129 or 133, preferably SEQ ID NO: 361, 95, 111, 125 or 131, wherein all the even-numbered nucleotides of the first strand are modified by a first modification, all the odd-numbered nucleotides of the first strand are modified by a second modification, all the nucleotides of the second strand in positions corresponding to nucleotides 11-13 of the first strand are modified by a fourth modification, all the nucleotides of the second strand other than the nucleotides corresponding to nucleotides 11-13 of the first strand modified by a third modification, wherein the first and fourth modification are 2'-F and the second and third modification are 2'-OMe.

The 3' and 5' ends of an oligonucleotide can be modified. Such modifications can be at the 3' end or the 5' end or both ends of the molecule. They can include modification or replacement of an entire terminal phosphate or of one or more of the atoms of the phosphate group. For example, the 3' and 5' ends of an oligonucleotide can be conjugated to other functional molecular entities such as labelling moieties, e.g., fluorophores (e.g., pyrene, TAMRA, fluorescein, Cy3 or Cy5 dyes) or protecting groups (based e.g., on sulfur, silicon, boron or ester). The functional molecular entities can be attached to the sugar through a phosphate group and/or a linker. The terminal atom of the linker can connect to or replace the linking atom of the phosphate group or the C-3' or C-5' O, N, S or C group of the sugar. Alternatively, the linker can connect to or replace the terminal atom of a nucleotide surrogate (e.g., PNAs). These spacers or linkers can include e.g., -(CH2)n-, -(CH2)nN-, -(CH2)nO-, -(CH2)nS-, -(CH2CH2O)nCH2CH2O- (e.g., n=3 or 6), abasic sugars, amide, carboxy, amine, oxyamine, oxyimine, thioether, disulfide, thiourea, sulfonamide, or morpholino, or biotin and fluorescein reagents. The 3' end can be an -OH group.

Other examples of terminal modifications include dyes, intercalating agents (e.g., acridines), cross-linkers (e.g., psoralene, mitomycin C), porphyrins (TPPC4, texaphyrin, Sapphyrin), polycyclic aromatic hydrocarbons (e.g., phenazine, dihydrophenazine), artificial endonucleases, EDTA, lipophilic carriers (e.g., cholesterol, cholic acid, adamantane acetic acid, 1-pyrene butyric acid, dihydrotestosterone, 1,3-Bis-O(hexadecyl)glycerol, geranyloxyhexyl group, hexadecylglycerol, borneol, menthol, 1,3-propanediol, heptadecyl group, palmitic acid, myristic acid, O3-(oleoyl)lithocholic acid, O3-(oleoyl)cholenic acid, dimethoxytrityl, or phenoxazine) and peptide conjugates (e.g., antennapedia peptide, Tat peptide), alkylating agents, phosphate, amino, mercapto, PEG (e.g., PEG-40K), MPEG, [MPEG]2, polyamino, alkyl, substituted alkyl, radiolabeled markers, enzymes, haptens (e.g., biotin), transport/absorption facilitators (e.g., aspirin, vitamin E, folic acid), synthetic ribonucleases (e.g., imidazole, bisimidazole, histamine, imidazole clusters, acridine-imidazole conjugates, Eu3+ complexes of tetraazamacrocycles).

Terminal modifications can also be useful for monitoring distribution, and in such cases the groups to be added may include fluorophores, e.g., fluorescein or an Alexa dye. Terminal modifications can also be useful for enhancing uptake, useful modifications for this include cholesterol. Terminal modifications can also be useful for cross-linking an RNA agent to another moiety.

Terminal modifications can be added for a number of reasons, including to modulate activity or to modulate resistance to degradation. Terminal modifications useful for modulating activity include modification of the 5' end with phosphate or phosphate analogues. Nucleic acids of the invention, on the first or second strand, may be 5' phosphorylated or include a phosphoryl analogue at the 5' prime terminus. 5'-phosphate modifications include those which are compatible with RISC mediated gene silencing. Suitable modifications include: 5'-monophosphate ((HO)2(O)P-O-5'); 5'-diphosphate ((HO)2(O)P-O-P(HO)(O)-O-5'); 5'-triphosphate ((HO)2(O)P-O-(HO)(O)P-O-P(HO)(O)-O-5'); 5'-guanosine cap (7-methylated or non-methylated) (7m-G-O-5'-(HO)(O)P-O-(HO)(O)P-O-P(HO)(O)-O-5'); 5'-adenosine cap (Appp), and any modified or unmodified nucleotide cap structure (N-O-5'-(HO)(O)P-O-(HO)(O)P-O-P(HO)(O)-O-5'); 5'-monothiophosphate (phosphorothioate; (HO)2(S)P-O-5'); 5'-monodithiophosphate (phosphorodithioate; (HO)(HS)(S)P-O-5'), 5'-phosphorothiolate ((HO)2(O)P-S-5'); any additional combination of oxygen/sulfur replaced monophosphate, diphosphate and triphosphates (e.g., 5'-alpha-thiotriphosphate, 5'-gamma-thiotriphosphate, etc.), 5'-phosphoramidates ((HO)2(O)P-NH-5', (HO)(NH2)(O)P-O-5'), 5'-alkylphosphonates (R=alkyl=methyl, ethyl, isopropyl, propyl, etc., e.g., RP(OH)(O)-O-5'-, (OH)2(O)P-5'-CH2-), 5'vinylphosphonate, 5'-alkyletherphosphonates (R=alkylether=methoxymethyl (MeOCH2-), ethoxymethyl, etc., e.g., RP(OH)(O)-O-5'-).

Certain moieties may be linked to the 5' terminus of the first strand or the second strand. These include abasic ribose moiety, abasic deoxyribose moiety, modifications abasic ribose and abasic deoxyribose moieties including 2'-O alkyl modifications; inverted abasic ribose and abasic deoxyribose moieties and modifications thereof, C6-imino-Pi; a mirror nucleotide including L-DNA and L-RNA; 5'OMe nucleotide; and nucleotide analogues including 4',5'-methylene nucleotide; 1-(β-D-erythrofuranosyl)nucleotide; 4'-thio nucleotide, carbocyclic nucleotide; 5'-amino-alkyl phosphate; 1,3-diamino-2-propyl phosphate, 3-aminopropyl phosphate; 6-aminohexyl phosphate; 12-aminododecyl phosphate; hydroxypropyl phosphate; 1,5-anhydrohexitol nucleotide; alpha-nucleotide; threo-pentofuranosyl nucleotide; acyclic 3',4'-seco nucleotide; 3,4-dihydroxybutyl nucleotide; 3,5-dihydroxypentyl nucleotide, 5'-5'-inverted abasic moiety; 1,4-butanediol phosphate; 5'-amino; and bridging or non-bridging methylphosphonate and 5'-mercapto moieties.

The invention also provides a nucleic acid according to any aspect of the invention described herein, wherein the first strand has a terminal 5' (E)-vinylphosphonate nucleotide at its 5' end. This terminal 5' (E)-vinylphosphonate nucleotide is preferably linked to the second nucleotide in the first strand by a phosphodiester linkage.

The first strand of the nucleic acid may comprise formula (I):

(vp)-N₍ₚₒ₎[N₍ₚₒ₎]ₙ- (I)

where '(vp)-' is the 5' (E)-vinylphosphonate, 'N' is a nucleotide, 'po' is a phosphodiester linkage, and n is from 1 to (the total number of nucleotides in the first strand - 2), preferably wherein n is from 1 to (the total number of nucleotides in the first strand -3), more preferably wherein n is from 1 to (the total number of nucleotides in the first strand -4).

Preferably, the terminal 5' (E)-vinylphosphonate nucleotide is an RNA nucleotide, preferably a (vp)-U.

A terminal 5' (E)-vinylphosphonate nucleotide is a nucleotide wherein the natural phosphate group at the 5'-end has been replaced with a E-vinylphosphonate, in which the bridging 5'-oxygen atom of the terminal nucleotide of the 5' phosphorylated strand is replaced with a methynyl (-CH=) group:

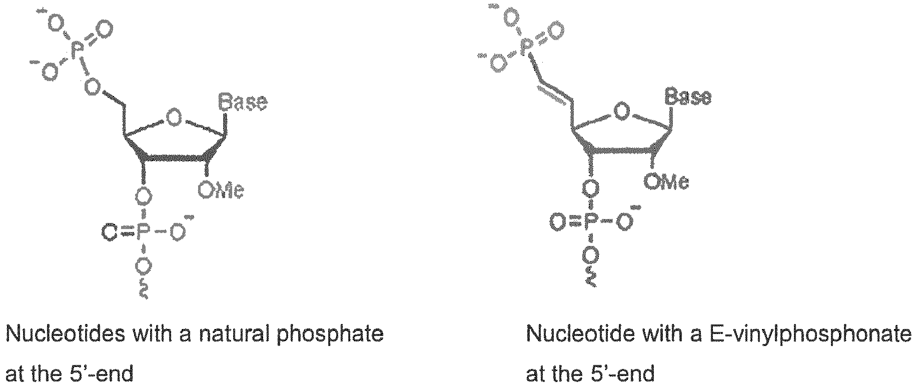

A 5' (E) vinylphosphonate is a 5' phosphate mimic. A biological mimic is a molecule that is capable of carrying out the same function as and is structurally very similar to the original molecule that is being mimicked. In the context of the present invention, 5' (E) vinylphosphonate mimics the function of a normal 5' phosphate, e.g. enabling efficient RISC loading. In addition, because of its slightly altered structure, 5' (E) vinylphosphonate is capable of stabilizing the 5'-end nucleotide by protecting it from dephosphorylation by enzymes such as phosphatases.

In one aspect, the first strand has a terminal 5' (E)-vinylphosphonate nucleotide at its 5' end, the terminal 5' (E)-vinylphosphonate nucleotide is linked to the second nucleotide in the first strand by a phosphodiester linkage and the first strand comprises a) more than 1 phosphodiester linkage; b) phosphodiester linkages between at least the terminal three 5' nucleotides and/or c) phosphodiester linkages between at least the terminal four 5' nucleotides.

In one aspect, the first strand and/or the second strand of the nucleic acid comprises at least one phosphorothioate (ps) linkage between two nucleotides.

In one aspect, the first strand and/or the second strand of the nucleic acid comprises more than 1 phosphorothioate linkage.

In one aspect, the first strand and/or the second strand of the nucleic acid comprises a phosphorothioate linkage between the terminal two 3' nucleotides or phosphorothioate linkages between the terminal three 3' nucleotides. Preferably, the linkages between the other nucleotides in the first strand and/or the second strand are phosphodiester linkages.

In one aspect, the first strand and/or the second strand of the nucleic acid comprises a phosphorothioate linkage between the terminal two 5' nucleotides or phosphorothioate linkages between the terminal three 5' nucleotides.

In one aspect, the nucleic acid of the present invention comprises one or more phosphorothioate modifications on one or more of the terminal ends of the first and/or the second strand. Optionally, each or either end of the first strand may comprise one or two or three phosphorothioate modified nucleotides. Optionally, each or either end of the second strand may comprise one or two or three phosphorothioate modified nucleotides.

In one aspect, the nucleic acid comprises a phosphorothioate linkage between the terminal two or three 3' nucleotides and/or 5' nucleotides of the first and/or the second strand. Preferably, the nucleic acid comprises a phosphorothioate linkage between each of the terminal three 3' nucleotides and the terminal three 5' nucleotides of the first strand and of the second strand. Preferably, all remaining linkages between nucleotides of the first and/or of the second strand are phosphodiester linkages.

In one aspect, the nucleic acid comprises a phosphorodithioate linkage between each of the two, three or four terminal nucleotides at the 3' end of the first strand and/or comprises a phosphorodithioate linkage between each of the two, three or four terminal nucleotides at the 3' end of the second strand and/or a phosphorodithioate linkage between each of the two, three or four terminal nucleotides at the 5' end of the second strand and comprises a linkage other than a phosphorodithioate linkage between the two, three or four terminal nucleotides at the 5' end of the first strand.

In one aspect, the nucleic acid comprises a phosphorothioate linkage between the terminal three 3' nucleotides and the terminal three 5' nucleotides of the first strand and of the second strand. Preferably, all remaining linkages between nucleotides of the first and/or of the second strand are phosphodiester linkages.

In one aspect, the nucleic acid:
(i) has a phosphorothioate linkage between the terminal three 3' nucleotides and the terminal three 5' nucleotides of the first strand;
(ii) is conjugated to a triantennary ligand either on the 3' end nucleotide or on the 5' end nucleotide of the second strand;
(iii) has a phosphorothioate linkage between the terminal three nucleotides of the second strand at the end opposite to the one conjugated to the triantennary ligand; and
(iv) all remaining linkages between nucleotides of the first and/or of the second strand are phosphodiester linkages.

In one aspect, the nucleic acid:
(i) has a terminal 5' (E)-vinylphosphonate nucleotide at the 5' end of the first strand;
(ii) has a phosphorothioate linkage between the terminal three 3' nucleotides on the first and second strand and between the terminal three 5' nucleotides on the second strand; and
(iii) all remaining linkages between nucleotides of the first and/or of the second strand are phosphodiester linkages.

The use of a phosphorodithioate linkage in the nucleic acid of the invention reduces the variation in the stereochemistry of a population of nucleic acid molecules compared to molecules comprising a phosphorothioate in that same position. Phosphorothioate linkage indeed introduce a chiral centre and it is difficult to control which non-linking oxygen is substituted for sulphur. The use of a phosphorodithioate ensures that no chiral centre exists in that linkage and thus reduces or eliminates any variation in the population of nucleic acid molecules, depending on the number of phosphorodithioate and phosphorothioate linkages used in the nucleic acid molecule.

In one aspect, the nucleic acid comprises a phosphorodithioate linkage between the two terminal nucleotides at the 3' end of the first strand and a phosphorodithioate linkage between the two terminal nucleotides at the 3' end of the second strand and a phosphorodithioate linkage between the two terminal nucleotides at the 5' end of the second strand and comprises a linkage other than a phosphorodithioate linkage between the two, three or four terminal nucleotides at the 5' end of the first strand. Preferably, the first strand has a terminal 5' (E)-vinylphosphonate nucleotide at its 5' end. This terminal 5' (E)-vinylphosphonate nucleotide is preferably linked to the second nucleotide in the first strand by a phosphodiester linkage. Preferably, all the linkages between the nucleotides of both strands other than the linkage between the two terminal nucleotides at the 3' end of the first strand and the linkages between the two terminal nucleotides at the 3' end and at the 5' end of the second strand are phosphodiester linkages.

In one aspect, the nucleic acid comprises a phosphorothioate linkage between each of the three terminal 3' nucleotides and/or between each of the three terminal 5' nucleotides on the first strand, and/or between each of the three terminal 3' nucleotides and/or between each of the three terminal 5' nucleotides of the second strand when there is no phosphorodithioate linkage present at that end. No phosphorodithioate linkage being present at an end means that the linkage between the two terminal nucleotides, or preferably between the three terminal nucleotides of the nucleic acid end in question are linkages other than phosphorodithioate linkages.

In one aspect, all the linkages of the nucleic acid between the nucleotides of both strands other than the linkage between the two terminal nucleotides at the 3' end of the first strand and the linkages between the two terminal nucleotides at the 3' end and at the 5' end of the second strand are phosphodiester linkages.

Other phosphate linkage modifications are possible.

The phosphate linker can also be modified by replacement of a linking oxygen with nitrogen (bridged phosphoroamidates), sulfur (bridged phosphorothioates) and carbon (bridged methylenephosphonates). The replacement can occur at a terminal oxygen. Replacement of the non-linking oxygens with nitrogen is possible.

The phosphate groups can also individually be replaced by non-phosphorus containing connectors.

Examples of moieties which can replace the phosphate group include siloxane, carbonate, carboxymethyl, carbamate, amide, thioether, ethylene oxide linker, sulfonate, sulfonamide, thioformacetal, formacetal, oxime, methyleneimino, methylenemethylimino, methylenehydrazo, methylenedimethylhydrazo and methyleneoxymethylimino. In certain embodiments, replacements may include the methylenecarbonylamino and methylenemethylimino groups.

The phosphate linker and ribose sugar may be replaced by nuclease resistant nucleotides. Examples include the morpholino, cyclobutyl, pyrrolidine and peptide nucleic acid (PNA) nucleoside surrogates. In certain embodiments, PNA surrogates may be used.

In one aspect, the nucleic acid, which is preferably an siRNA that inhibits expression of the complement component C3, preferably via RNAi, comprises one or more or all of:
(i) a modified nucleotide;
(ii) a modified nucleotide other than a 2'-OMe modified nucleotide at positions 2 and 14 from the 5' end of the first strand, preferably a 2'-F modified nucleotide;
(iii) each of the odd-numbered nucleotides of the first strand as numbered starting from one at the 5' end of the first strand are 2'-OMe modified nucleotides;
(iv) each of the even-numbered nucleotides of the first strand as numbered starting from one at the 5' end of the first strand are 2'-F modified nucleotides;
(v) the second strand nucleotide corresponding to position 11 or 13 of the first strand is modified by a modification other than a 2'-OMe modification, preferably wherein one or both of these positions comprise a 2'-F modification;
(vi) an inverted nucleotide, preferably a 3'-3' linkage at the 3' end of the second strand;
(vii) one or more phosphorothioate linkages;
(viii) one or more phosphorodithioate linkages; and/or
(ix) the first strand has a terminal 5' (E)-vinylphosphonate nucleotide at its 5' end, in which case the terminal 5' (E)-vinylphosphonate nucleotide is preferably a uridine and is preferably linked to the second nucleotide in the first strand by a phosphodiester linkage.

All the features of the nucleic acids can be combined with all other aspects of the invention disclosed herein.

### Ligands

The nucleic acid of the invention may be conjugated to a ligand. Efficient delivery of oligonucleotides, in particular double stranded nucleic acids of the invention, to cells *in vivo* is important and requires specific targeting and substantial protection from the extracellular environment, particularly serum proteins. One method of achieving specific targeting is to conjugate a ligand to the nucleic acid. The ligand helps in targeting the nucleic acid to the required target site. There is a need to conjugate appropriate ligands for the desired receptor molecules in order for the conjugated molecules to be taken up by the target cells by mechanisms such as different receptor-mediated endocytosis pathways or functionally analogous processes.

One example is the asialoglycoprotein receptor complex (ASGP-R) composed by varying ratios of multimers of membrane ASGR1 and ASGR2 receptors, which is highly abundant on hepatocytes and has high affinity to the here described GalNAc moiety. One of the first disclosures of the use of triantennary cluster glycosides as conjugated ligands was in US patent number US 5,885,968. Conjugates having three GalNAc ligands and comprising phosphate groups are known and are described in Dubber et al. (Bioconjug. Chem. 2003 Jan-Feb;14(1):239-46.). The ASGP-R complex shows a 50-fold higher affinity for N-Acetyl-D-Galactosamine (GalNAc) than D-Gal.

The asialoglycoprotein receptor complex (ASGP-R), which recognizes specifically terminal β-galactosyl subunits of glycosylated proteins or other oligosaccharides (Weigel, P.H. et. al., Biochim. Biophys. Acta. 2002 Sep 19; 1572(2-3):341-63) can be used for delivering a drug to the liver's hepatocytes expressing the receptor complex by covalent coupling of galactose or galactosamine to the drug substance (Ishibashi,S.; et. al., J Biol. Chem. 1994 Nov 11;269(45):27803-6). Furthermore the binding affinity can be significantly increased by the multi-valency effect, which is achieved by the repetition of the targeting moiety (Biessen EA, et al., J Med Chem. 1995 Apr 28;38(9):1538-46.).

The ASGP-R complex is a mediator for an active uptake of terminal β-galactosyl containing glycoproteins to the cell's endosomes. Thus, the ASGPR is highly suitable for targeted delivery of drug candidates conjugated to such ligands like, e.g., nucleic acids into receptor-expressing cells (Akinc et al., Mol Ther. 2010 Jul;18(7):1357-64).

More generally the ligand can comprise a saccharide that is selected to have an affinity for at least one type of receptor on a target cell. In particular, the receptor is on the surface of a mammalian liver cell, for example, the hepatic asialoglycoprotein receptor complex described before (ASGP-R).

The saccharide may be selected from N-acetyl galactosamine, mannose, galactose, glucose, glucosamine and fucose. The saccharide may be N-acetyl galactosamine (GalNAc).

A ligand for use in the present invention may therefore comprise (i) one or more N-acetyl galactosamine (GalNAc) moieties and derivatives thereof, and (ii) a linker, wherein the linker conjugates the GalNAc moieties to a nucleic acid as defined in any preceding aspects. The linker may be a monovalent structure or bivalent or trivalent or tetravalent branched structure. The nucleotides may be modified as defined herein.

The ligand may therefore comprise GalNAc.

In one aspect, the nucleic acid is conjugated to a ligand comprising a compound of formula (II):

[S-X¹-P-X²]₃-A-X³- (II)

wherein:
S represents a saccharide, preferably wherein the saccharide is N-acetyl galactosamine;
X¹ represents C₃-C₆ alkylene or (-CH₂-CH₂-O)ₘ(-CH₂)₂- wherein m is 1, 2, or 3;
P is a phosphate or modified phosphate, preferably a thiophosphate;
X² is alkylene or an alkylene ether of the formula (-CH₂)ₙ-O-CH₂- where n = 1- 6;
A is a branching unit;
X³ represents a bridging unit;
wherein a nucleic acid according to the present invention is conjugated to X³ via a phosphate or modified phosphate, preferably a thiophosphate.

In formula (II), the branching unit "A" preferably branches into three in order to accommodate three saccharide ligands. The branching unit is preferably covalently attached to the remaining tethered portions of the ligand and the nucleic acid. The branching unit may comprise a branched aliphatic group comprising groups selected from alkyl, amide, disulphide, polyethylene glycol, ether, thioether and hydroxyamino groups. The branching unit may comprise groups selected from alkyl and ether groups.

The branching unit A may have a structure selected from: 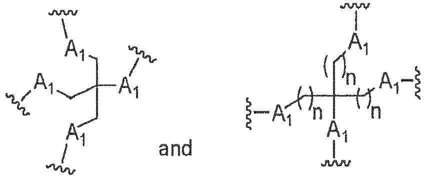 wherein each A₁ independently represents O, S, C=O or NH; and each n independently represents an integer from 1 to 20.

The branching unit may have a structure selected from: 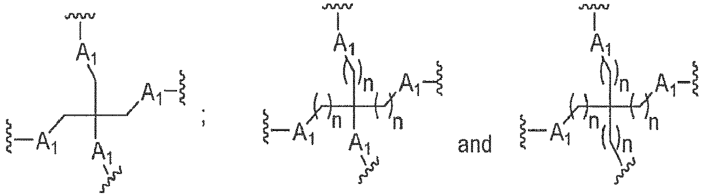 wherein each A₁ independently represents O, S, C=O or NH; and each n independently represents an integer from 1 to 20.

The branching unit may have a structure selected from: 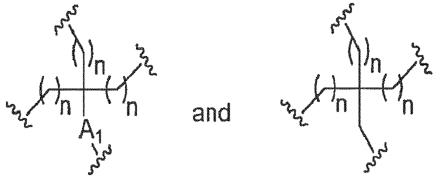 wherein A₁ is O, S, C=O or NH; and each n independently represents an integer from 1 to 20. The branching unit may have the structure:

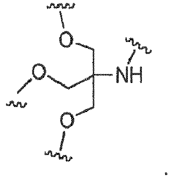

The branching unit may have the structure:

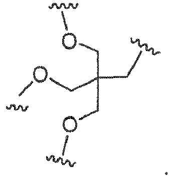

The branching unit may have the structure:

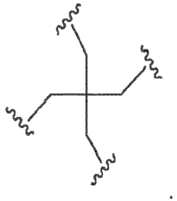

Optionally, the branching unit consists of only a carbon atom.

The "X³" portion is a bridging unit. The bridging unit is linear and is covalently bound to the branching unit and the nucleic acid.

X³ may be selected from -C₁-C₂₀ alkylene-, -C₂-C₂₀ alkenylene-, an alkylene ether of formula-(C₁-C₂₀ alkylene)-O-(C₁-C₂₀ alkylene)-, -C(O)-C₁-C₂₀ alkylene-, -C₀-C₄ alkylene(Cy)C₀-C₄ alkylene- wherein Cy represents a substituted or unsubstituted 5 or 6 membered cycloalkylene, arylene, heterocyclylene or heteroarylene ring, -C₁-C₄ alkylene-NHC(O)-C₁-C₄ alkylene-, -C₁-C₄ alkylene-C(O)NH-C₁-C₄ alkylene-, -C₁-C₄ alkylene-SC(O)-C₁-C₄ alkylene-, -C₁-C₄ alkylene-C(O)S-C₁-C₄ alkylene-, -C₁-C₄ alkylene-OC(O)-C₁-C₄ alkylene-, -C₁-C₄ alkylene-C(O)O-C₁-C₄ alkylene-, and -C₁-C₆ alkylene-S-S-C₁-C₆ alkylene-.

X³ may be an alkylene ether of formula -(C₁-C₂₀ alkylene)-O-(C₁-C₂₀ alkylene)-. X³ may be an alkylene ether of formula -(C₁-C₂₀ alkylene)-O-(C₄-C₂₀ alkylene)-, wherein said (C₄-C₂₀ alkylene) is linked to Z. X³ may be selected from the group consisting of -CH₂-O-C₃H₆-, -CH₂-O-C₄H₈-, -CH₂-O-C₆H₁₂- and -CH₂-O-C₈H₁₆-, especially -CH₂-O-C₄H₈-, -CH₂-O-C₆H₁₂- and-CH₂-O-C₈H₁₆-, wherein in each case the -CH₂- group is linked to A.

In one aspect, the nucleic acid is conjugated to a ligand comprising a compound of formula (III):

[S-X¹-P-X²]₃-A-X³- (III)

wherein:
S represents a saccharide, preferably GalNAc;
X¹ represents C₃-C₆ alkylene or (-CH₂-CH₂-O)ₘ(-CH₂)₂- wherein m is 1, 2, or 3;
P is a phosphate or modified phosphate, preferably a thiophosphate;
X² is C₁-C₈ alkylene;
A is a branching unit selected from: 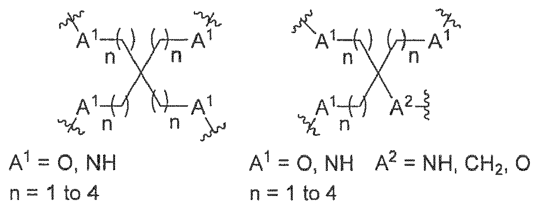
   X³ is a bridging unit;
   wherein a nucleic acid according to the present invention is conjugated to X³ via a phosphate or a modified phosphate, preferably a thiophosphate.

The branching unit A may have the structure:

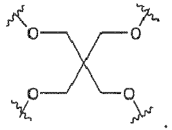

The Branching unit A may have the structure: 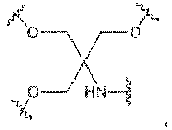 wherein X³ is attached to the nitrogen atom.

X³ may be C₁-C₂₀ alkylene. Preferably, X³ is selected from the group consisting of -C₃H₆-,-C₄H₈-, -C₆H₁₂- and -C₈H₁₆-, especially -C₄H₈-, -C₆H₁₂- and -C₈H₁₆-.

In one aspect, the nucleic acid is conjugated to a ligand comprising a compound of formula (IV):

[S-X¹-P-X²]₃-A-X³- (IV)

wherein:
S represents a saccharide, preferably GalNAc;
X¹ represents C₃-C₆ alkylene or (-CH₂-CH₂-O)ₘ(-CH₂)₂- wherein m is 1, 2, or 3;
P is a phosphate or modified phosphate, preferably a thiophosphate;
X² is an alkylene ether of formula -C₃H₆-O-CH₂-;
A is a branching unit;
X³ is an alkylene ether of formula selected from the group consisting of -CH₂-O-CH₂-,-CH₂-O-C₂H₄-, -CH₂-O-C₃H₆-, -CH₂-O-C₄H₈-, -CH₂-O-C₅H₁₀-, -CH₂-O-C₆H₁₂-, -CH₂-O-C₇H₁₄-, and -CH₂-O-C₈H₁₆-, wherein in each case the -CH₂- group is linked to A,
and wherein X³ is conjugated to a nucleic acid according to the present invention by a phosphate or modified phosphate, preferably a thiophosphate.

The branching unit may comprise carbon. Preferably, the branching unit is a carbon.

X³ may be selected from the group consisting of -CH₂-O-C₄H₈-, -CH₂-O-C₅H₁₀-, -CH₂-O-C₆H₁₂-, -CH₂-O-C₇H₁₄-, and -CH₂-O-C₈H₁₆-. Preferably, X³ is selected from the group consisting of-CH₂-O-C₄H₈-, -CH₂-O-C₆H₁₂- and -CH₂-O-C₈H₁₆.

X¹ may be (-CH₂-CH₂-O)(-CH₂)₂-. X¹ may be (-CH₂-CH₂-O)₂(-CH₂)₂-. X¹ may be (-CH₂-CH₂-O)₃(-CH₂)₂-. Preferably, X¹ is (-CH₂-CH₂-O)₂(-CH₂)₂-. Alternatively, X¹ represents C₃-C₆ alkylene. X¹ may be propylene. X¹ may be butylene. X¹ may be pentylene. X¹ may be hexylene. Preferably the alkyl is a linear alkylene. In particular, X¹ may be butylene.

X² represents an alkylene ether of formula -C₃H₆-O-CH₂- i.e. C₃ alkoxy methylene, or-CH₂CH₂CH₂OCH₂-.

For any of the above aspects, when P represents a modified phosphate group, P can be represented by: 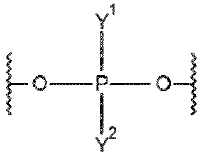 wherein Y¹ and Y² each independently represent =O, =S, -O-, -OH, -SH, -BH₃, -OCH₂CO₂,-OCH₂CO₂R^{x}, -OCH₂C(S)OR^{x}, and -OR^{x}, wherein R^{x} represents C₁-C₆ alkyl and wherein 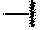 indicates attachment to the remainder of the compound.

By modified phosphate it is meant a phosphate group wherein one or more of the non-linking oxygens is replaced. Examples of modified phosphate groups include phosphorothioate, phosphoroselenates, borano phosphates, borano phosphate esters, hydrogen phosphonates, phosphoroamidates, alkyl or aryl phosphonates and phosphotriesters. Phosphorodithioates have both non-linking oxygens replaced by sulphur. One, each or both non-linking oxygens in the phosphate group can be independently any one of S, Se, B, C, H, N, or OR (R is alkyl or aryl).

The phosphate can also be modified by replacement of a linking oxygen with nitrogen (bridged phosphoroamidates), sulfur (bridged phosphorothioates) and carbon (bridged methylenephosphonates). The replacement can occur at a terminal oxygen. Replacement of the non-linking oxygens with nitrogen is possible.

For example, Y¹ may represent -OH and Y² may represent =O or =S; or
Y¹ may represent -O⁻ and Y² may represent =O or =S;
Y¹ may represent =O and Y² may represent -CH₃, -SH, -OR^{x}, or -BH₃
Y¹ may represent =S and Y² may represent -CH₃, OR^{x} or -SH.

It will be understood by the skilled person that in certain instances there will be delocalisation between Y¹ and Y².

Preferably, the modified phosphate group is a thiophosphate group. Thiophosphate groups include bithiophosphate (i.e. where Y¹ represents =S and Y² represents -S⁻) and monothiophosphate (i.e. where Y¹ represents -O⁻ and Y² represents =S, or where Y¹ represents =O and Y² represents -S⁻). Preferably, P is a monothiophosphate. The inventors have found that conjugates having thiophosphate groups in replacement of phosphate groups have improved potency and duration of action *in vivo.*

P may also be an ethylphosphate (i.e. where Y¹ represents =O and Y² represents OCH₂CH₃).

The saccharide may be selected to have an affinity for at least one type of receptor on a target cell. In particular, the receptor is on the surface of a mammalian liver cell, for example, the hepatic asialoglycoprotein receptor complex (ASGP-R).

For any of the above or below aspects, the saccharide may be selected from N-acetyl with one or more of galactosamine, mannose, galactose, glucose, glucosamine and fructose. Typically a ligand to be used in the present invention may include N-acetyl galactosamine (GalNAc). Preferably the compounds of the invention may have 3 ligands, which will each preferably include N-acetyl galactosamine.

"GalNAc" refers to 2-(Acetylamino)-2-deoxy-D-galactopyranose, commonly referred to in the literature as N-acetyl galactosamine. Reference to "GalNAc" or "N-acetyl galactosamine" includes both the β- form: 2-(Acetylamino)-2-deoxy-β-D-galactopyranose and the α-form: 2-(Acetylamino)-2-deoxy-α-D-galactopyranose. In certain embodiments, both the β-form: 2-(Acetylamino)-2-deoxy-β-D-galactopyranose and α-form: 2-(Acetylamino)-2-deoxy-α-D-galactopyranose may be used interchangeably. Preferably, the compounds of the invention comprise the β-form, 2-(Acetylamino)-2-deoxy-β-D-galactopyranose. 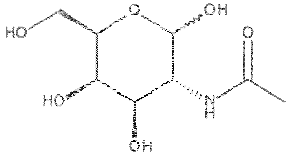 2-(Acetylamino)-2-deoxy-D-galactopyranose 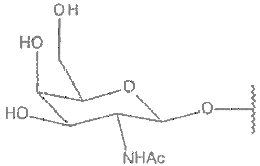 2-(Acetylamino)-2-deoxy-β-D-galactopyranose 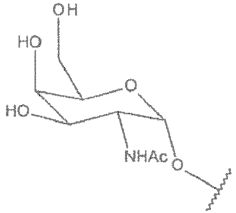 2-(Acetylamino)-2-deoxy-α-D-galactopyranose

In one aspect, the nucleic acid is a conjugated nucleic acid, wherein the nucleic acid is conjugated to a triantennary ligand with one of the following structures: 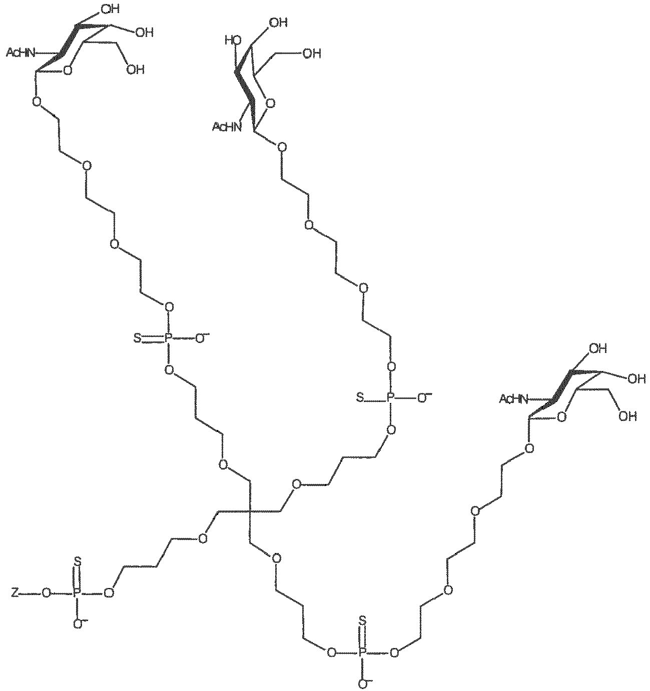 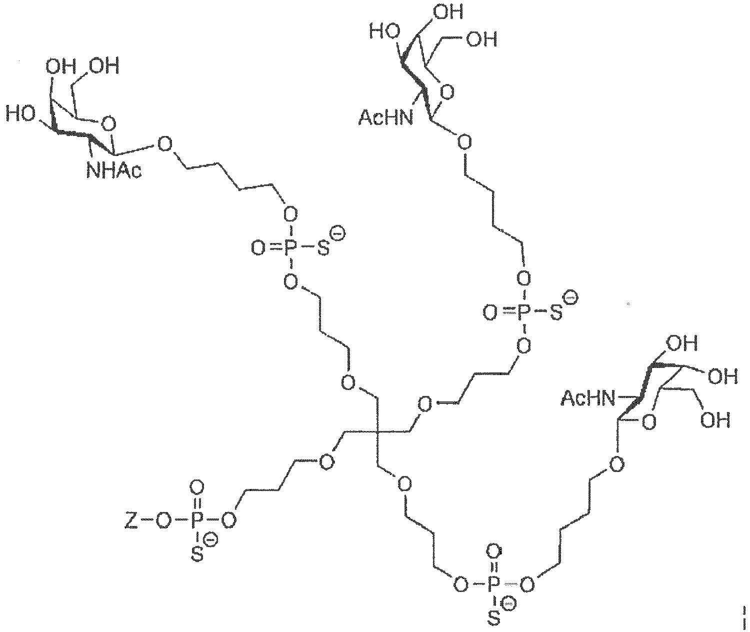 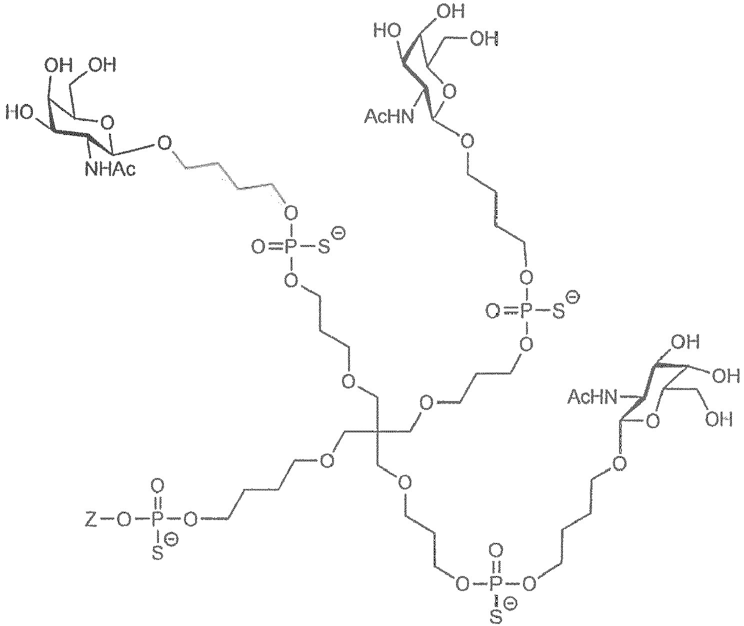 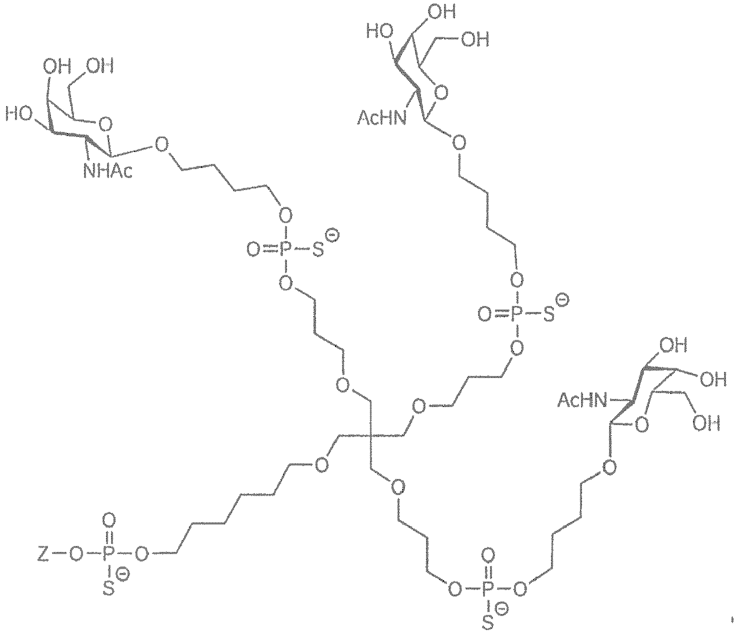 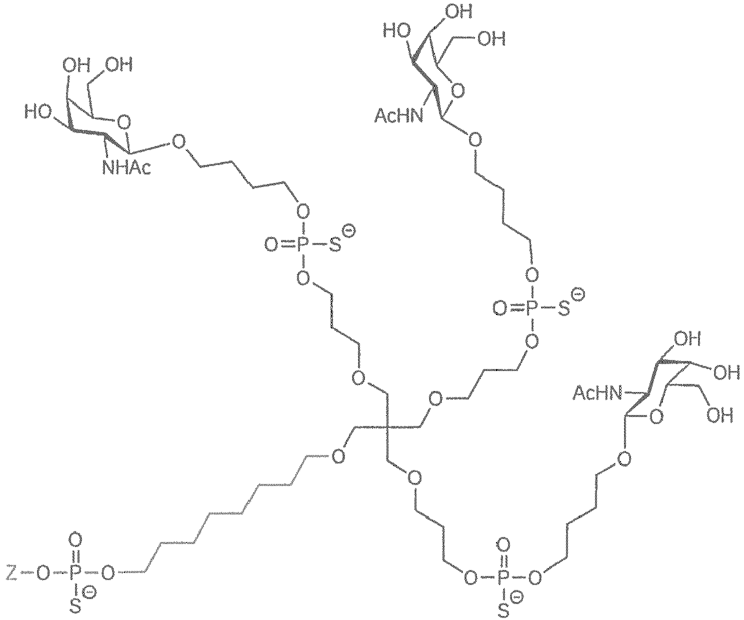 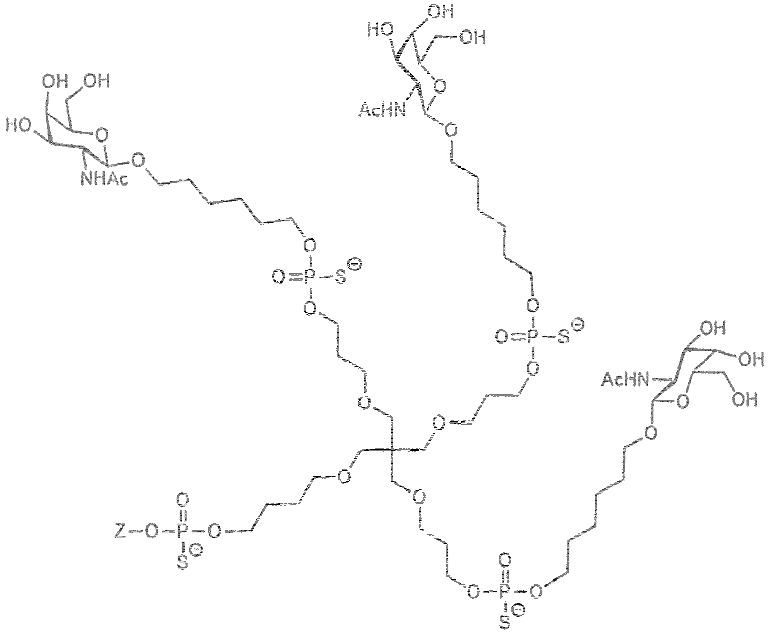 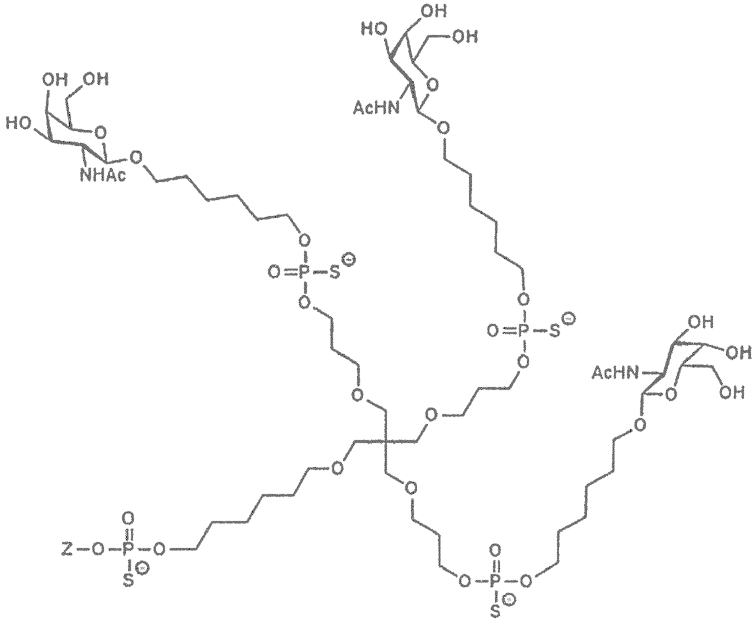 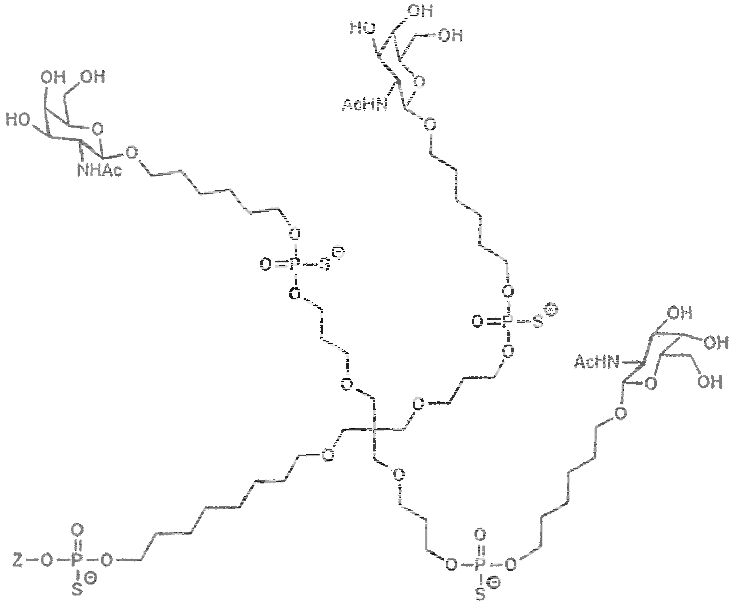 wherein Z is any nucleic acid as defined herein.

Preferably, the nucleic acid is a conjugated nucleic acid, wherein the nucleic acid is conjugated to a triantennary ligand with the following structures: 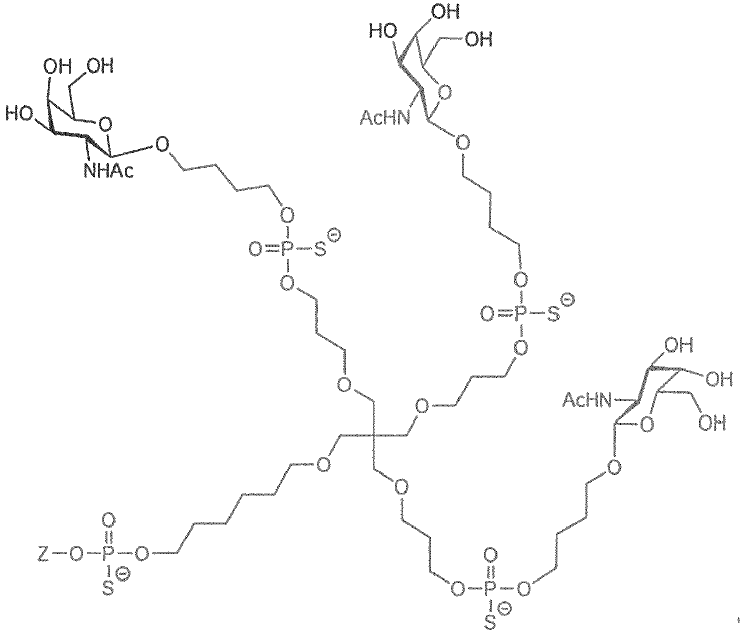 wherein Z is any nucleic acid as defined herein.

A ligand of formula (II), (III) or (IV) or any one of the triantennary ligands disclosed herein can be attached at the 3'-end of the first (antisense) strand and/or at any of the 3' and/or 5' end of the second (sense) strand. The nucleic acid can comprise more than one ligand of formula (II), (III) or (IV) or any one of the triantennary ligands disclosed herein. However, a single ligand of formula (II), (III) or (IV) or any one of the triantennary ligands disclosed herein is preferred because a single such ligand is sufficient for efficient targeting of the nucleic acid to the target cells. Preferably in that case, at least the last two, preferably at least the last three and more preferably at least the last four nucleotides at the end of the nucleic acid to which the ligand is attached are linked by a phosphodiester linkage.

Preferably, the 5'-end of the first (antisense) strand is not attached to a ligand of formula (II), (III) or (IV) or any one of the triantennary ligands disclosed herein, since a ligand in this position can potentially interfere with the biological activity of the nucleic acid.

A nucleic acid with a single ligand of formula (II), (III) or (IV) or any one of the triantennary ligands disclosed herein at the 5' end of a strand is easier and therefore cheaper to synthesis than the same nucleic acid with the same ligand at the 3' end. Preferably therefore, a single ligand of any of formulae (II), (III) or (IV) or any one of the triantennary ligands disclosed herein is covalently attached to (conjugated with) the 5' end of the second strand of the nucleic acid.

In one aspect, the first strand of the nucleic acid is a compound of formula (V): 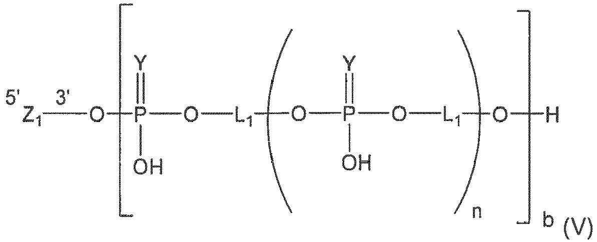 wherein b is preferably 0 or 1; and
the second strand is a compound of formula (VI): 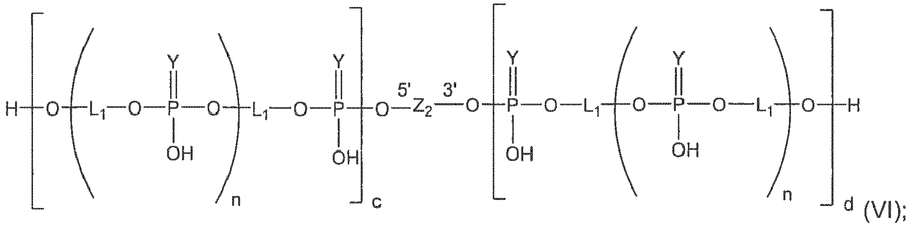 wherein:
c and d are independently preferably 0 or 1;
Z₁ and Z₂ are respectively the first and second strand of the nucleic acid;
Y is independently O or S;
n is independently 0, 1, 2 or 3; and
L₁ is a linker to which a ligand is attached, wherein L₁ is the same or different in formulae (V) and (VI), and is the same or different within formulae (V) and (VI) when L₁ is present more than once within the same formula, wherein L₁ is preferably of formula (VII);
and wherein b + c + d is preferably 2 or 3.

Preferably, L₁ in formulae (V) and (VI) is of formula (VII): 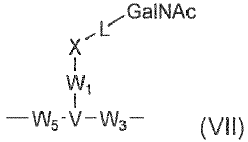 wherein:
L is selected from the group comprising, or preferably consisting of:
   -(CH₂)ᵣ-C(O)-, wherein r = 2-12;
   -(CH₂-CH₂-O)_{S}-CH₂-C(O)-, wherein s = 1-5;
   -(CH₂)ₜCO-NH-(CH₂)ₜNH-C(O)-, wherein t is independently 1-5;
   -(CH₂)ᵤ-CO-NH-(CH₂)ᵤ-C(O)-, wherein u is independently 1-5; and
   -(CH₂)ᵥ-NH-C(O)-, wherein v is 2-12; and
wherein the terminal C(O), if present, is attached to X of formula (VII), or if X is absent, to W₁ of formula (VII), or if W₁ is absent, to V of formula (VII);
W₁, W₃ and W₅ are individually absent or selected from the group comprising, or preferably consisting of:
   -(CH₂)ᵣ-, wherein r = 1-7;
   -(CH₂)ₛ-O-(CH₂)ₛ-, wherein s is independently 0-5;
   -(CH₂)ₜ-S-(CH₂)ₜ-, wherein t is independently 0-5;
X is absent or is selected from the group comprising, or preferably consisting of: NH, NCH₃ or NC₂H₅;
V is selected from the group comprising, or preferably consisting of:
   CH, N, 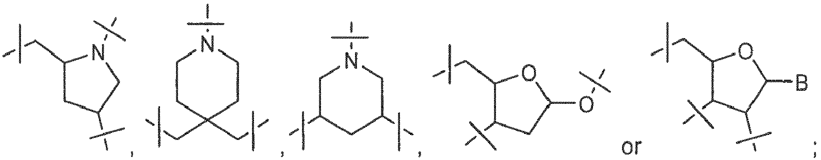
wherein B, if present, is a modified or natural nucleobase.

In one aspect, the first strand is a compound of formula (VIII) 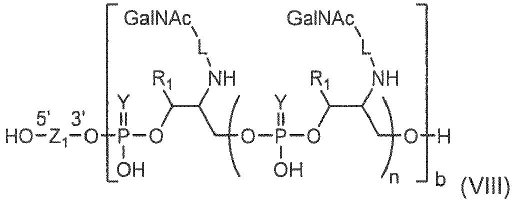 wherein b is preferably 0 or 1; and
the second strand is a compound of formula (IX): 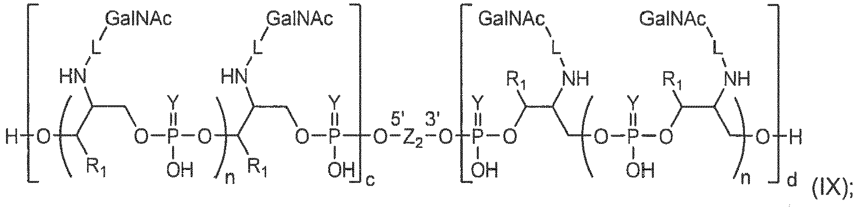 wherein c and d are independently preferably 0 or 1;
wherein:
Z₁ and Z₂ are respectively the first and second strand of the nucleic acid;
Y is independently O or S;
R₁ is H or methyl;
n is independently preferably 0, 1, 2 or 3; and
L is the same or different in formulae (VIII) and (IX), and is the same or different within formulae (VIII) and (IX) when L is present more than once within the same formula, and is selected from the group comprising, or preferably consisting of:
   -(CH₂)ᵣ-C(O)-, wherein r = 2-12;
   -(CH₂-CH₂-O)ₛ-CH₂-C(O)-, wherein s = 1-5;
   -(CH₂)ₜ-CO-NH-(CH₂)ₜ-NH-C(O)-, wherein t is independently 1-5;
   -(CH₂)ᵤ-CO-NH-(CH₂)ᵤ-C(O)-, wherein u is independently 1-5; and
   -(CH₂)ᵥ-NH-C(O)-, wherein v is 2-12; and
wherein the terminal C(O), if present, is attached to the NH group (of the linker, not of the targeting ligand);
and wherein b + c + d is preferably 2 or 3.

In one aspect, the first strand of the nucleic acid is a compound of formula (X): 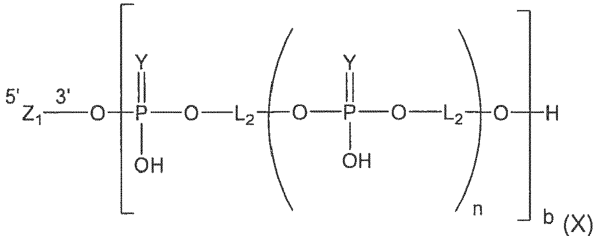 wherein b is preferably 0 or 1; and
the second strand is a compound of formula (XI): 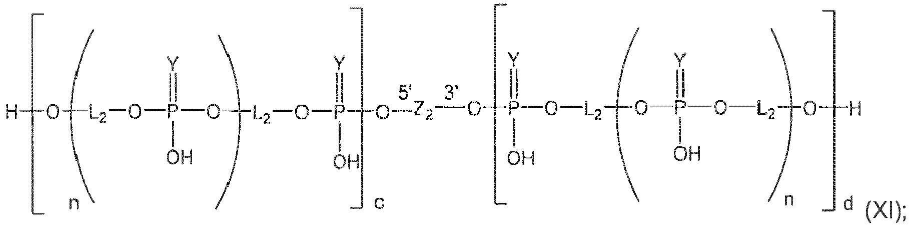 wherein:
c and d are independently preferably 0 or 1;
Z₁ and Z₂ are respectively the first and second RNA strand of the nucleic;
Y is independently O or S;
n is independently preferably 0, 1, 2 or 3; and
L₂ is the same or different in formulae (X) and (XI) and is the same or different in moieties bracketed by b, c and d, and is selected from the group comprising, or preferably consisting of: 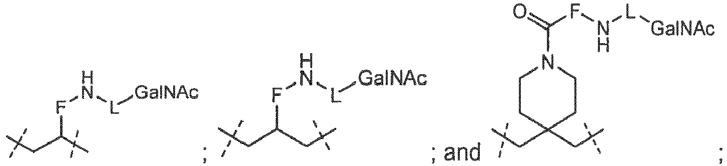 or
   n is 0 and L₂ is: 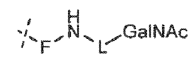 and the terminal OH group is absent such that the following moiety is formed: 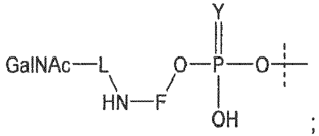 wherein:
   F is a saturated branched or unbranched (such as unbranched) C₁₋₈alkyl (e.g. C₁₋₆alkyl) chain wherein one of the carbon atoms is optionally replaced with an oxygen atom provided that said oxygen atom is separated from another heteroatom (e.g. an O or N atom) by at least 2 carbon atoms;
   L is the same or different in formulae (X) and (XI) and is selected from the group comprising, or preferably consisting of:
      -(CH₂)ᵣ-C(O)-, wherein r = 2-12;
      -(CH₂-CH₂-O)ₛ-CH₂-C(O)-, wherein s = 1-5;
      -(CH₂)ₜ-CO-NH-(CH₂)ₜNH-C(O)-, wherein t is independently 1-5;
      -(CH₂)ᵤ-CO-NH-(CH₂)ᵤ-C(O)-, wherein u is independently 1-5; and
      -(CH₂)ᵥ-NH-C(O)-, wherein v is 2-12; and
   wherein the terminal C(O), if present, is attached to the NH group (of the linker, not of the targeting ligand);
and wherein b + c + d is preferably 2 or 3.

In one aspect, b is 0, c is 1 and d is 1; b is 1, c is 0 and d is 1; b is 1, c is 1 and d is 0; or b is 1, c is 1 and d is 1 in any of the nucleic acids of formulae (V) and (VI) or (VIII) and (IX) or (X) and (XI). Preferably, b is 0, c is 1 and d is 1; b is 1, c is 0 and d is 1; or b is 1, c is 1 and d is 1. Most preferably, b is 0, c is 1 and d is 1.

In one aspect, Y is O in any of the nucleic acids of formulae (V) and (VI) or (VIII) and (IX) or (X) and (XI). In another aspect, Y is S. In a preferred aspect, Y is independently selected from O or S in the different positions in the formulae.

In one aspect, R₁ is H or methyl in any of the nucleic acids of formulae (VIII) and (IX). In one aspect, R₁ is H. In another aspect, R₁ is methyl.

In one aspect, n is 0, 1, 2 or 3 in any of the nucleic acids of formulae (V) and (VI) or (VIII) and (IX) or (X) and (XI). Preferably, n is 0.

Examples of F moieties in any of the nucleic acids of formulae (X) and (XI) include (CH₂)₁₋₆ e.g. (CH₂)₁₋₄ e.g. CH₂, (CH₂)₄, (CH₂)₅ or (CH₂)₆, or CH₂O(CH₂)₂₋₃, e.g. CH₂O(CH₂)CH₃.

In one aspect, L₂ in formulae (X) and (XI) is:

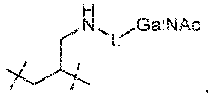

In one aspect, L₂ is:

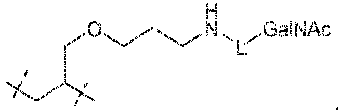

In one aspect, L₂ is:

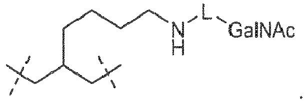

In one aspect, L₂ is:

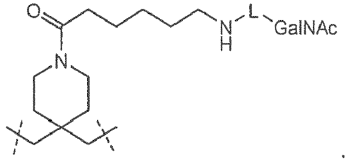

In one aspect, n is 0 and L₂ is: 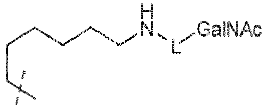 and the terminal OH group is absent such that the following moiety is formed: 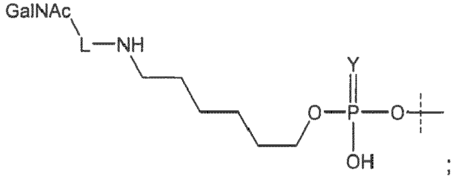 wherein Y is O or S.

In one aspect, L in the nucleic acids of formulae (V) and (VI) or (VIII) and (IX) or (X) and (XI), is selected from the group comprising, or preferably consisting of:
-(CH₂)ᵣ-C(O)-, wherein r = 2-12;
-(CH₂-CH₂-O)ₛ-CH₂-C(O)-, wherein s = 1-5;
-(CH₂)ₜ-CO-NH-(CH₂)ₜ-NH-C(O)-, wherein t is independently 1-5;
-(CH₂)ᵤ-CO-NH-(CH₂)ᵤ-C(O)-, wherein u is independently 1-5; and
-(CH₂)ᵥ-NH-C(O)-, wherein v is 2-12;
wherein the terminal C(O) is attached to the NH group.

Preferably, L is -(CH₂)ᵣ-C(O)-, wherein r = 2-12, more preferably r = 2-6 even more preferably, r = 4 or 6 e.g. 4.

Preferably, L is:

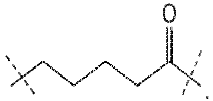

Within the moiety bracketed by b, c and d, L₂ in the nucleic acids of formulae (X) and (XI) is typically the same. Between moieties bracketed by b, c and d, L₂ may be the same or different. In an embodiment, L₂ in the moiety bracketed by c is the same as the L₂ in the moiety bracketed by d. In an embodiment, L₂ in the moiety bracketed by c is not the same as L₂ in the moiety bracketed by d. In an embodiment, the L₂ in the moieties bracketed by b, c and d is the same, for example when the linker moiety is a serinol-derived linker moiety.

Serinol derived linker moieties may be based on serinol in any stereochemistry i.e. derived from L-serine isomer, D-serine isomer, a racemic serine or other combination of isomers. In a preferred aspect of the invention, the serinol-GalNAc moiety (SerGN) has the following stereochemistry: 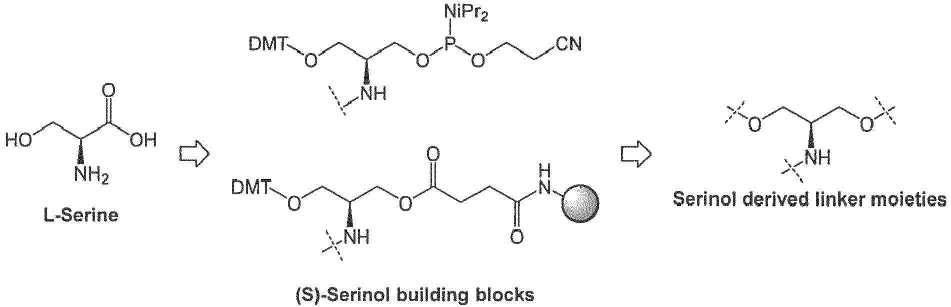 i.e. is based on an (S)-serinol-amidite or (S)-serinol succinate solid supported building block derived from L-serine isomer.

In a preferred aspect, the first strand of the nucleic acid is a compound of formula (VIII) and the second strand of the nucleic acid is a compound of formula (IX), wherein:
b is 0;
c and d are 1,
n is 0,
Z₁ and Z₂ are respectively the first and second strand of the nucleic acid,
Y is S,
R₁ is H, and
L is -(CH₂)₄-C(O)-, wherein the terminal C(O) of L is attached to the N atom of the linker (ie not a possible N atom of a targeting ligand).

In another preferred aspect, the first strand of the nucleic acid is a compound of formula (V) and the second strand of the nucleic acid is a compound of formula (VI), wherein:
b is 0,
c and d are 1,
n is 0,
Z₁ and Z₂ are respectively the first and second strand of the nucleic acid,
Y is S,
L₁ is of formula (VII), wherein:
   W₁ is -CH₂-O-(CH₂)₃-,
   W₃ is -CH₂-,
   W₅ is absent,
   V is CH,
   X is NH, and
   L is -(CH₂)₄-C(O)- wherein the terminal C(O) of L is attached to the N atom of X in formula (VII).

In another preferred aspect, the first strand of the nucleic acid is a compound of formula (V) and the second strand of the nucleic acid is a compound of formula (VI), wherein:
b is 0,
c and d are 1,
n is 0,
Z₁ and Z₂ are respectively the first and second strand of the nucleic acid,
Y is S,
L₁ is of formula (VII), wherein:
   W₁, W₃ and W₅ are absent,
   V is 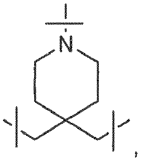
   X is absent, and
   L is -(CH₂)₄-C(O)-NH-(CH₂)₅-C(O)-, wherein the terminal C(O) of L is attached to the N atom of V in formula (VII).

In one aspect, the nucleic acid is conjugated to a triantennary ligand with the following structure: 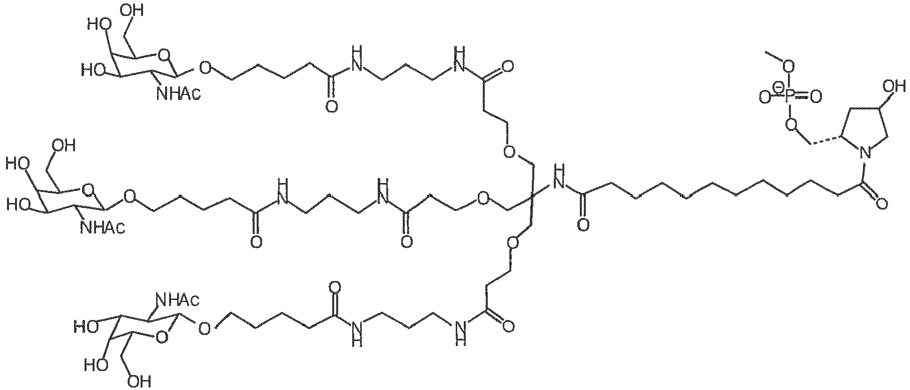 wherein the nucleic acid is conjugated to the ligand via the phosphate group of the ligand a) to the last nucleotide at the 5' end of the second strand; b) to the last nucleotide at the 3' end of the second strand; or c) to the last nucleotide at the 3' end of the first strand.

In one aspect of the nucleic acid, the cells that are targeted by the nucleic acid with ligand are hepatocytes.

In any one of the above ligands where GalNAc is present, the GalNAc may be substituted for any other targeting ligand, such as those mentioned herein, in particular mannose, galactose, glucose, glucosamine and fucose.

In one aspect, the nucleic acid is conjugated to a ligand that comprises a lipid, and more preferably a ligand that comprises a cholesterol.

### Compositions, uses and methods

The present invention also provides compositions comprising a nucleic acid of the invention. The nucleic acids and compositions may be used as medicaments or as diagnostic agents, alone or in combination with other agents. For example, one or more nucleic acid of the invention can be combined with a delivery vehicle (e.g., liposomes) and/or excipients, such as carriers, diluents. Other agents such as preservatives and stabilizers can also be added. Methods for the delivery of nucleic acids are known in the art and within the knowledge of the person skilled in the art.

In one aspect the composition comprises a nucleic acid disclosed herein and a delivery vehicle and/or a physiologically acceptable excipient and/or a carrier and/or a diluent and/or a buffer and/or a preservative.

The nucleic acid or conjugated nucleic acid of the present invention can also be administered in combination with other therapeutic compounds, either administrated separately or simultaneously, e.g., as a combined unit dose. The invention also includes a composition comprising one or more nucleic acids according to the present invention in a physiologically/pharmaceutically acceptable excipient, such as a stabilizer, preservative, diluent, buffer, and the like.

In one aspect, the composition comprises a nucleic acid disclosed herein and a further therapeutic agent selected from the group comprising an oligonucleotide, a small molecule, a monoclonal antibody, a polyclonal antibody and a peptide. Preferably, the further therapeutic agent is an agent that targets, preferably inhibits the expression or the activity, of the complement component C3 or of another element, such as a protein, of the immune system or more specifically of the complement pathway. Preferably, the further therapeutic agent is one of the following: a) a peptide that inhibits the expression or activity of one of the components of the complement pathway, preferably either C3 or C5 or one of their subunits; b) an antibody that specifically binds under physiological conditions to one of the components of the complement pathway, preferably either C3 or C5 or one of their subunits; c) Eculizumab or and antigen-binding derivative thereof.

Eculizumab is a humanised monoclonal antibody that specifically binds to the complement component C5 and is commercialised under the trade name SOLIRIS®. It specifically binds the complement component C5 with high affinity and inhibits cleavage of C5 to C5a and C5b. The antibody is for example described in the patent EP 0 758 904 B1 and its family members.

Dosage levels for the medicament and compositions of the invention can be determined by those skilled in the art by routine experimentation. In one aspect, a unit dose may contain between about 0.01 mg/kg and about 100 mg/kg body weight of nucleic acid or conjugated nucleic acid. Alternatively, the dose can be from 10 mg/kg to 25 mg/kg body weight, or 1 mg/kg to 10 mg/kg body weight, or 0.05 mg/kg to 5 mg/kg body weight, or 0.1 mg/kg to 5 mg/kg body weight, or 0.1 mg/kg to1 mg/kg body weight, or 0.1 mg/kg to 0.5 mg/kg body weight, or 0.5 mg/kg to 1 mg/kg body weight. Dosage levels may also be calculated via other parameters such as, e.g., body surface area.

The pharmaceutical composition may be a sterile injectable aqueous suspension or solution, or in a lyophilized form.

The pharmaceutical compositions and medicaments of the present invention may be administered to a mammalian subject in a pharmaceutically effective dose. The mammal may be selected from a human, a non-human primate, a simian or prosimian, a dog, a cat, a horse, cattle, a pig, a goat, a sheep, a mouse, a rat, a hamster, a hedgehog and a guinea pig, or other species of relevance. On this basis, "C3" as used herein denotes nucleic acid or protein in any of the above-mentioned species, if expressed therein naturally or artificially, but preferably this wording denotes human nucleic acids or proteins.

One aspect of the invention is a nucleic acid or a composition disclosed herein for use as a medicament. The nucleic acid or composition is preferably for use in the prevention, decrease of the risk of suffering from, or treatment of a disease, disorder or syndrome.

One aspect of the invention is the use of a nucleic acid or a composition as disclosed herein in the prevention, decrease of the risk of suffering from, or treatment of a disease, disorder or syndrome.

One aspect of the invention is a method of preventing, decreasing the risk of suffering from, or treating a disease, disorder or syndrome comprising administering a pharmaceutically effective dose of a nucleic acid or a composition disclosed herein to an individual in need of treatment, preferably wherein the nucleic acid or composition is administered to the subject subcutaneously, intravenously or by oral, rectal or intraperitoneal administration. Preferably, it is administered subcutaneously.

The disease, disorder or syndrome to be prevented, or treated with a nucleic acid or composition disclosed herein is preferably a complement-mediated disease, disorder or syndrome or a disease disorder or syndrome associated with the complement pathway.

The disease, disorder or syndrome to be prevented or treated with a nucleic acid or composition disclosed herein is preferably associated with aberrant activation and/or over-activation (hyper-activation) of the complement pathway and/or with over-expression or ectopic expression or localisation or accumulation of the complement component C3. One example of a disease that involves accumulation of C3 is C3 Glomerulopathy (C3G). In this disease, C3 accumulates in the kidney glomeruli. The aberrant or over activation of the complement pathway to be prevented or treated can have genetic causes or can be acquired. Preferably, the disease, disorder or syndrome to be prevented or treated is C3 Glomerulopathy (C3G).

The disease, disorder or syndrome to be prevented or treated with a nucleic acid or composition disclosed herein is preferably a) selected from the group comprising, and preferably consisting of C3 Glomerulopathy (C3G), Paroxysmal Nocturnal Hemoglobinuria (PNH), atypical Hemolytic Uremic Syndrome (aHUS), Lupus nephritis, IgA nephropathy (IgA N), Cold Agglutinin Disease (CAD), Myasthenia gravis (MG), Primary Membranous Nephropathy, Immune Complex-mediated Glomerulonephritis (IC-mediated GN), post-Infectious Glomerulonephritis (PIGN), Systemic Lupus Erythematosus (SLE), Ischemia/reperfusion injury, age-related macular degeneration (AMD), Rheumatoid arthritis (RA), antineutrophil Cytoplasmic Autoantibodies-associated Vasculitis (ANCA-AV), dysbiotic periodontal Disease, Malarial Anaemia and sepsis; or b) selected from the group comprising, or preferably consisting of C3 Glomerulopathy (C3G), Paroxysmal Nocturnal Hemoglobinuria (PNH), atypical Hemolytic Uremic Syndrome (aHUS), Lupus nephritis, IgA nephropathy (IgA N) and Primary Membranous Nephropathy; or c) selected from the group comprising, or preferably consisting of C3 Glomerulopathy (C3G), antineutrophil Cytoplasmic Autoantibodies-associated Vasculitis (ANCA-AV), atypical Hemolytic Uremic Syndrome (aHUS), Cold Agglutinin Disease (CAD), Myasthenia gravis (MG), IgA nephropathy (IgA N) Paroxysmal Nocturnal Hemoglobinuria (PNH); or d) it is C3 Glomerulopathy (C3G). The subjects to be treated with a nucleic acid or composition according to the invention are preferably subjects that suffer from one of these diseases, disorders or syndromes.

A nucleic acid or compositions disclosed herein may be for use in a regimen comprising treatments once or twice weekly, every week, every two weeks, every three weeks, every four weeks, every five weeks, every six weeks, every seven weeks, or every eight weeks, or in regimens with varying dosing frequency such as combinations of the before-mentioned intervals. The nucleic acid or composition may be for use subcutaneously, intravenously or using any other application routes such as oral, rectal or intraperitoneal. Preferably, it is for use subcutaneously.

In cells and/or subjects treated with or receiving a nucleic acid or composition as disclosed herein, the C3 expression may be inhibited compared to untreated cells and/or subjects by a range from 15% up to 100% but at least about 30%, 35%, 40%, 45%, 50%, 55%, 60%, 65%, 70%, 75%, 80%, 85%, 90%, 95%, 98%, or 100% or intermediate values. The level of inhibition may allow treatment of a disease associated with C3 expression or overexpression or complement over-activation, or may serve to further investigate the functions and physiological roles of the C3 gene products.

One aspect is the use of a nucleic acid or composition as disclosed herein in the manufacture of a medicament for treating a disease, disorder or syndromes, such as those as listed above or additional pathologies associated with elevated levels of C3, preferably in the blood or in the kidneys, or over activation of the complement pathway, or additional therapeutic approaches where inhibition of C3 expression is desired.

Also included in the invention is a method of treating or preventing a disease, disorder or syndrome, such as those listed above, comprising administration of a composition comprising a nucleic acid or composition as described herein, to an individual in need of treatment (to improve such pathologies). The nucleic acid or composition may be administered in a regimen comprising treatments twice every week, once every week, every two weeks, every three weeks, every four weeks, every five weeks, every six weeks, every seven weeks, or every eight weeks or in regimens with varying dosing frequency such as combinations of the before-mentioned intervals. The nucleic acid or conjugated nucleic acid may be for use subcutaneously or intravenously or other application routes such as oral, rectal or intraperitoneal.

The nucleic acid or composition of the present invention can be produced using routine methods in the art including chemical synthesis or expressing the nucleic acid either in vitro (e.g., run off transcription) or *in vivo.* For example, using solid phase chemical synthesis or using a nucleic acid-based expression vector including viral derivates or partially or completely synthetic expression systems. In one aspect, the expression vector can be used to produce the nucleic acid of the invention *in vitro,* within an intermediate host organism or cell type, within an intermediate or the final organism or within the desired target cell. Methods for the production (synthesis or enzymatic transcription) of the nucleic acid described herein are known to persons skilled in the art.

The use of a chemical modification pattern of the nucleic acids confers nuclease stability in serum and makes for example subcutaneous application route feasible.

The invention is characterized by high specificity at the molecular and tissue-directed delivery level, potentially conferring a better safety profile than the currently available treatments.

The nucleic acid as described herein may be formulated with a lipid in the form of a liposome. Such a formulation may be described in the art as a lipoplex. The composition with a lipid/liposome may be used to assist with delivery of the nucleic acid of the invention to the target cells. The lipid delivery system herein described may be used as an alternative to a conjugated ligand. The modifications herein described may be present when using the nucleic acid of the invention with a lipid delivery system or with a ligand conjugate delivery system.

Such a lipoplex may comprise a lipid composition comprising:
i) a cationic lipid, or a pharmaceutically acceptable salt thereof;
ii) a steroid;
iii) a phosphatidylethanolamine phospholipid;
iv) a PEGylated lipid.

The cationic lipid may be an amino cationic lipid.

The cationic lipid may have the formula (XII): 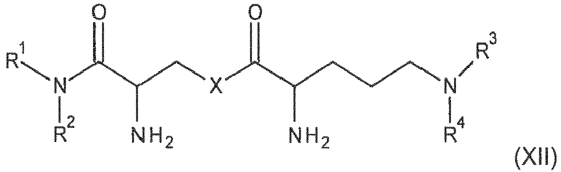 or a pharmaceutically acceptable salt thereof, wherein:
X represents O, S or NH;
R¹ and R² each independently represents a C₄-C₂₂ linear or branched alkyl chain or a C₄-C₂₂ linear or branched alkenyl chain with one or more double bonds, wherein the alkyl or alkenyl chain optionally contains an intervening ester, amide or disulfide;
when X represents S or NH, R³ and R⁴ each independently represent hydrogen, methyl, ethyl, a mono- or polyamine moiety, or R³ and R⁴ together form a heterocyclyl ring;
when X represents O, R3 and R4 each independently represent hydrogen, methyl, ethyl, a mono- or polyamine moiety, or R³ and R⁴ together form a heterocyclyl ring, or R³ represents hydrogen and R⁴ represents C(NH)(NH₂).

The cationic lipid may have the formula (XIII): 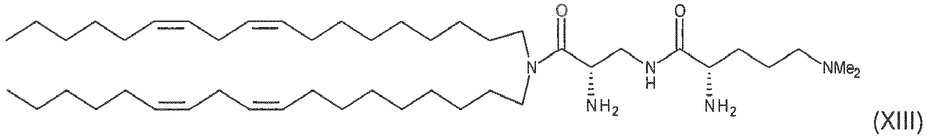 or a pharmaceutically acceptable salt thereof.

The cationic lipid may have the formula (XIV): 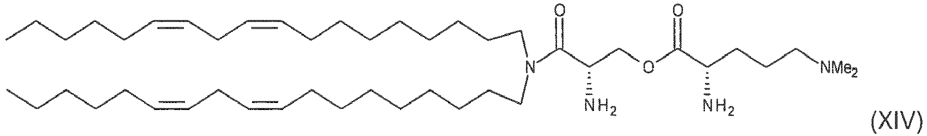 or a pharmaceutically acceptable salt thereof.

The content of the cationic lipid component may be from about 55 mol% to about 65 mol% of the overall lipid content of the composition. In particular, the cationic lipid component is about 59 mol% of the overall lipid content of the composition.

The compositions can further comprise a steroid. The steroid may be cholesterol. The content of the steroid may be from about 26 mol% to about 35 mol% of the overall lipid content of the lipid composition. More particularly, the content of steroid may be about 30 mol% of the overall lipid content of the lipid composition.

The phosphatidylethanolamine phospholipid may be selected from the group consisting of 1,2-diphytanoyl-sn-glycero-3-phosphoethanolamine (DPhyPE), 1,2-dioleoyl-sn-glycero-3-phosphoethanolamine (DOPE), 1,2-distearoyl-sn-glycero-3-phosphoethanolamine (DSPE), 1,2-Dilauroyl-sn-glycero-3-phosphoethanolamine (DLPE), 1,2-Dimyristoyl-sn-glycero-3-phosphoethanolamine (DMPE), 1,2-Dipalmitoyl-sn-glycero-3-phosphoethanolamine (DPPE), 1,2-Dilinoleoyl-sn-glycero-3-phosphoethanolamine (DLoPE), 1-Palmitoyl-2-oleoyl-sn-glycero-3-phosphoethanolamine (POPE), 1,2-Dierucoyl-sn-glycero-3-phosphoethanolamine (DEPE), 1,2-Disqualeoyl-sn-glycero-3-phosphoethanolamine (DSQPE) and 1-Stearoyl-2-linoleoyl-sn-glycero-3-phosphoethanolamine (SLPE). The content of the phospholipid may be about 10 mol% of the overall lipid content of the composition.

The PEGylated lipid may be selected from the group consisting of 1,2-dimyristoyl-sn-glycerol, methoxypolyethylene glycol (DMG-PEG) and C16-Ceramide-PEG. The content of the PEGylated lipid may be about 1 to 5 mol% of the overall lipid content of the composition.

The content of the cationic lipid component in the composition may be from about 55 mol% to about 65 mol% of the overall lipid content of the lipid composition, preferably about 59 mol% of the overall lipid content of the lipid composition.

The composition may have a molar ratio of the components of i):ii): iii): iv) selected from 55:34:10:1; 56:33:10:1; 57:32:10:1; 58:31:10:1; 59:30:10:1; 60:29:10:1; 61:28:10:1; 62:27:10:1; 63:26:10:1; 64:25:10:1; and 65:24:10:1.

The composition may comprise a cationic lipid having the structure 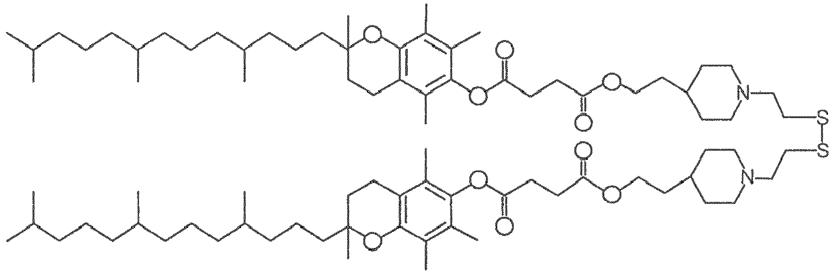 a steroid having the structure 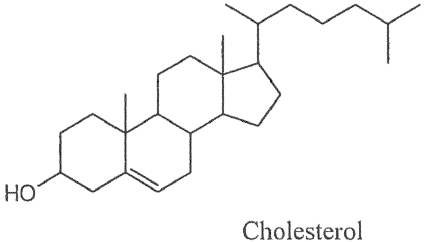 a phosphatidylethanolamine phospholipid having the structure 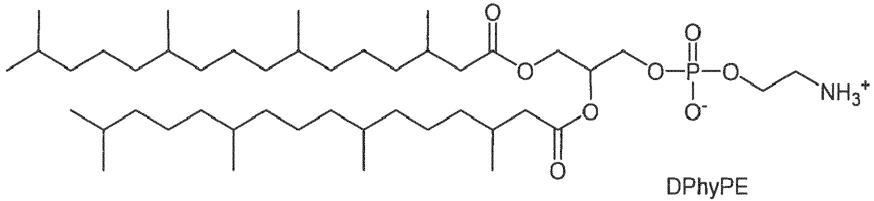 and a PEGylated lipid having the structure

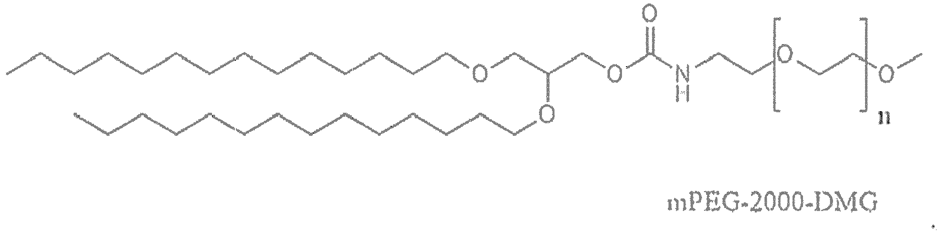

Neutral liposome compositions may be formed from, for example, dimyristoyl phosphatidylcholine (DMPC) or dipalmitoyl phosphatidylcholine (DPPC). Anionic liposome compositions may be formed from dimyristoyl phosphatidylglycerol, while anionic fusogenic liposomes may be formed primarily from dioleoyl phosphatidylethanolamine (DOPE). Another type of liposomal composition may be formed from phosphatidylcholine (PC) such as, for example, soybean PC, and egg PC. Another type is formed from mixtures of phospholipid and/or phosphatidylcholine and/or cholesterol.

A positively charged synthetic cationic lipid, N-[1-(2,3-dioleyloxy)propyl]-N,N,N-trimethylammonium chloride (DOTMA) can be used to form small liposomes that interact spontaneously with nucleic acid to form lipid-nucleic acid complexes which are capable of fusing with the negatively charged lipids of the cell membranes of tissue culture cells. DOTMA analogues can also be used to form liposomes.

Derivatives and analogues of lipids described herein may also be used to form liposomes.

A liposome containing a nucleic acid can be prepared by a variety of methods. In one example, the lipid component of a liposome is dissolved in a detergent so that micelles are formed with the lipid component. For example, the lipid component can be an amphipathic cationic lipid or lipid conjugate. The detergent can have a high critical micelle concentration and may be nonionic. Exemplary detergents include cholate, CHAPS, octylglucoside, deoxycholate, and lauroyl sarcosine. The nucleic acid preparation is then added to the micelles that include the lipid component. The cationic groups on the lipid interact with the nucleic acid and condense around the nucleic acid to form a liposome. After condensation, the detergent is removed, e.g., by dialysis, to yield a liposomal preparation of nucleic acid.

If necessary a carrier compound that assists in condensation can be added during the condensation reaction, e.g., by controlled addition. For example, the carrier compound can be a polymer other than a nucleic acid (e.g., spermine or spermidine). pH can also be adjusted to favour condensation.

Nucleic acid formulations may include a surfactant. In one embodiment, the nucleic acid is formulated as an emulsion that includes a surfactant.

A surfactant that is not ionized is a non-ionic surfactant. Examples include non-ionic esters, such as ethylene glycol esters, propylene glycol esters, glyceryl esters etc., nonionic alkanolamides, and ethers such as fatty alcohol ethoxylates, propoxylated alcohols, and ethoxylated/propoxylated block polymers.

A surfactant that carries a negative charge when dissolved or dispersed in water is an anionic surfactant. Examples include carboxylates, such as soaps, acyl lactylates, acyl amides of amino acids, esters of sulfuric acid such as alkyl sulfates and ethoxylated alkyl sulfates, sulfonates such as alkyl benzene sulfonates, acyl isethionates, acyl taurates and sulfosuccinates, and phosphates.

A surfactant that carries a positive charge when dissolved or dispersed in water is a cationic surfactant. Examples include quaternary ammonium salts and ethoxylated amines.

A surfactant that has the ability to carry either a positive or negative charge is an amphoteric surfactant. Examples include acrylic acid derivatives, substituted alkylamides, N-alkylbetaines and phosphatides.

"Micelles" are defined herein as a particular type of molecular assembly in which amphipathic molecules are arranged in a spherical structure such that all the hydrophobic portions of the molecules are directed inward, leaving the hydrophilic portions in contact with the surrounding aqueous phase. The converse arrangement exists if the environment is hydrophobic. A micelle may be formed by mixing an aqueous solution of the nucleic acid, an alkali metal alkyl sulphate, and at least one micelle forming compound.

Exemplary micelle forming compounds include lecithin, hyaluronic acid, pharmaceutically acceptable salts of hyaluronic acid, glycolic acid, lactic acid, chamomile extract, cucumber extract, oleic acid, linoleic acid, linolenic acid, monoolein, monooleates, monolaurates, borage oil, evening of primrose oil, menthol, trihydroxy oxo cholanyl glycine and pharmaceutically acceptable salts thereof, glycerol, polyglycerol, lysine, polylysine, triolein, polyoxyethylene ethers and analogues thereof, polidocanol alkyl ethers and analogues thereof, chenodeoxycholate, deoxycholate, and mixtures thereof.

Phenol and/or m-cresol may be added to the mixed micellar composition to act as a stabiliser and preservative. An isotonic agent such as glycerine may as be added.

A nucleic acid preparation may be incorporated into a particle such as a microparticle. Microparticles can be produced by spray-drying, lyophilisation, evaporation, fluid bed drying, vacuum drying, or a combination of these methods.

### Definitions

As used herein, the terms "inhibit", "down-regulate", or "reduce" with respect to gene expression mean that the expression of the gene, or the level of RNA molecules or equivalent RNA molecules encoding one or more proteins or protein subunits (e.g., mRNA), or the activity of one or more proteins or protein subunits, is reduced below that observed either in the absence of the nucleic acid or conjugated nucleic acid of the invention or as compared to that obtained with an siRNA molecule with no known homology to the human transcript (herein termed non-silencing control). Such control may be conjugated and modified in an analogous manner to the molecule of the invention and delivered into the target cell by the same route. The expression after treatment with the nucleic acid of the invention may be reduced to 90%, 80%, 70%, 60%, 50%, 40%, 30%, 20%, 15%, or to intermediate values, or less than that observed in the absence of the nucleic acid or conjugated nucleic acid. The expression may be measured in the cells to which the nucleic acid is applied. Alternatively, especially if the nucleic acid is administered to a subject, the level can be measured in a different group of cells or in a tissue or an organ or in a body fluid such as blood or plasma. The level of inhibition is preferably measured in conditions that have been selected because they show the greatest effect of the nucleic acid on the target mRNA level in cells treated with the nucleic acid *in vitro.* The level of inhibition may for example be measured after 24 hours or 48 hours of treatment with a nucleic acid at a concentration of between 0.038 nM - 10 µM, preferably 1 nM, 10 nM or 100 nM. These conditions may be different for different nucleic acid sequences or for different types of nucleic acids, such as for nucleic acids that are unmodified or modified or conjugated to a ligand or not. Examples of suitable conditions for determining levels of inhibition are described in the examples.

By nucleic acid it is meant a nucleic acid comprising two strands comprising nucleotides, that is able to interfere with gene expression. Inhibition may be complete or partial and results in down regulation of gene expression in a targeted manner. The nucleic acid comprises two separate polynucleotide strands; the first strand, which may also be a guide strand; and a second strand, which may also be a passenger strand. The first strand and the second strand may be part of the same polynucleotide molecule that is self-complementary which 'folds' back to form a double stranded molecule. The nucleic acid may be an siRNA molecule.

The nucleic acid may comprise ribonucleotides, modified ribonucleotides, deoxynucleotides, deoxyribonucleotides, or nucleotide analogues non-nucleotides that are able to mimic nucleotides such that they may 'pair' with the corresponding base on the target sequence or complementary strand. The nucleic acid may further comprise a double stranded nucleic acid portion or duplex region formed by all or a portion of the first strand (also known in the art as a guide strand) and all or a portion of the second strand (also known in the art as a passenger strand). The duplex region is defined as beginning with the first base pair formed between the first strand and the second strand and ending with the last base pair formed between the first strand and the second strand, inclusive.

By duplex region it is meant the region in two complementary or substantially complementary oligonucleotides that form base pairs with one another, either by Watson-Crick base pairing or any other manner that allows for a duplex between oligonucleotide strands that are complementary or substantially complementary. For example, an oligonucleotide strand having 21 nucleotide units can base pair with another oligonucleotide of 21 nucleotide units, yet only 19 nucleotides on each strand are complementary or substantially complementary, such that the "duplex region" consists of 19 base pairs. The remaining base pairs may exist as 5' and 3' overhangs, or as single stranded regions. Further, within the duplex region, 100% complementarity is not required; substantial complementarity is allowable within a duplex region. Substantial complementarity refers to complementarity between the strands such that they are capable of annealing under biological conditions. Techniques to empirically determine if two strands are capable of annealing under biological conditions are well known in the art. Alternatively, two strands can be synthesised and added together under biological conditions to determine if they anneal to one another. The portion of the first strand and second strand that form at least one duplex region may be fully complementary and is at least partially complementary to each other. Depending on the length of a nucleic acid, a perfect match in terms of base complementarity between the first strand and the second strand is not necessarily required. However, the first and second strands must be able to hybridise under physiological conditions.

As used herein, the terms "non-pairing nucleotide analogue" means a nucleotide analogue which includes a non-base pairing moiety including but not limited to: 6 des amino adenosine (Nebularine), 4-Me-indole, 3-nitropyrrole, 5-nitroindole, Ds, Pa, N3-Me ribo U, N3-Me riboT, N3-Me dC, N3-Me-dT, N1-Me-dG, N1-Me-dA, N3-ethyl-dC, N3-Me dC. In some embodiments the non-base pairing nucleotide analogue is a ribonucleotide. In other embodiments it is a deoxyribonucleotide.

As used herein, the term, "terminal functional group" includes without limitation a halogen, alcohol, amine, carboxylic, ester, amide, aldehyde, ketone, ether groups.

An "overhang" as used herein has its normal and customary meaning in the art, i.e. a single stranded portion of a nucleic acid that extends beyond the terminal nucleotide of a complementary strand in a double strand nucleic acid. The term "blunt end" includes double stranded nucleic acid whereby both strands terminate at the same position, regardless of whether the terminal nucleotide(s) are base-paired. The terminal nucleotide of a first strand and a second strand at a blunt end may be base paired. The terminal nucleotide of a first strand and a second strand at a blunt end may not be paired. The terminal two nucleotides of a first strand and a second strand at a blunt end may be base-paired. The terminal two nucleotides of a first strand and a second strand at a blunt end may not be paired.

The term "serinol-derived linker moiety" means the linker moiety comprises the following structure:

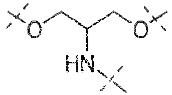

An O atom of said structure typically links to an RNA strand and the N atom typically links to the targeting ligand.

The invention will now be described with reference to the following non-limiting Figures and Examples.

### Brief description of the figures

Figures 1A, 1B and 1C show concentration-response-curves of selected siRNAs.
Figures 2A and 2B show concentration-response-curves of selected siRNA GalNAc conjugates in human primary hepatocytes.
Figures 3A and 3B show concentration-response-curves of selected siRNA GalNAc conjugates in mouse primary hepatocytes.
Figures 4A, 4B and 4C show concentration-dependent C3 mRNA inhibition of selected siRNA GalNAc conjugates respectively in primary mouse, human and cynomolgus hepatocytes.
Figure 5 shows a possible synthesis route to DMT-Serinol(GalNAc)-CEP and CPG.
Figures 6A and 6B show *in vivo* C3 mRNA levels in hepatocytes as well as C3 protein levels in serum in response to sc treatment of mice with selected siRNA GalNAc conjugates.
Figures 7A, 7B and 7C show relative C3 mRNA expression in primary mouse (A), cynomolgus (B) and human (C) hepatocytes after incubation with selected siRNA GalNac conjugates (1nM, 10nM and 100 nM) normalized to ACTIN mRNA.
Figures 8A and 8B show relative C3 mRNA expression in % in murine liver 14 days (A) or 42 days (B) after a single dosing of GalNAc conjugated siRNAs EV0201, EV0203, EV0204, EV0205 and EV0207. Data is shown in bar charts as mean ± SD (n=4 per group).
Figures 9A-E show relative C3 protein serum levels in % from mouse serum samples taken before (BL), at day 4, at day 10, day 14, day 21, day 28, day 35 and day 42 of the study after dosing of 5 or 10 mg/kg siRNA. Data points depict serum C3 level of individual animals determined using a standard C3 ELISA. Data was normalized to each group's baseline mean and then to the time matched PBS control, which was set as 100%. The plotted line connects the individual group means at the respective timepoints.
   A) Normalised C3 serum levels after dosing of 5 and 10 mg/kg EV0201
   B) Normalised C3 serum levels after dosing of 5 and 10 mg/kg EV0203
   C) Normalised C3 serum levels after dosing of 5 mg/kg EV0204
   D) Normalised C3 serum levels after dosing of 5 and 10 mg/kg EV0205
   E) Normalised C3 serum levels after dosing of 5 and 10 mg/kg EV0207.

### Examples

### Example 1

*In vitro* study in HepG2 cells showing C3 mRNA knockdown efficacy of tested siRNAs after transfection of 10 nM siRNA.

C3 knockdown efficacy of siRNAs EV0001-EV0100 was determined after transfection of 10 nM siRNA in HepG2 cells. The results are shown in Table 2 below. Remaining C3 mRNA levels after knockdown were in the range of 6 to 83 %. The most potent siRNAs were EV0001, EV0007, EV0008, EV0009, EV0012, EV0013, EV0018, EV0020, EV0030, EV0033, and EV0004.

For transfection of HepG2 cells with siRNAs, cells were seeded at a density of 15,000 cells / well into collagen-coated 96-well tissue culture plates (#655150, GBO, Germany). Transfection of siRNAs was carried out with Lipofectamine RNAiMax (Invitrogen/Life Technologies, Karlsruhe, Germany) according to the manufacturer's instructions directly after seeding. The screen was performed with C3 siRNAs in quadruplicates at 10 nM, with siRNAs targeting Aha1, Firefly-Luciferase and Factor VII as unspecific controls and a mock transfection. After 24h of incubation with siRNAs, medium was removed, and cells were lysed in 150 µl Medium-Lysis Mixture (1 volume lysis mixture, 2 volumes cell culture medium) and then incubated at 53°C for 30 minutes. bDNA assay was performed according to the manufacturer's instructions. Luminescence was read using 1420 Luminescence Counter (WALLAC VICTOR Light, Perkin Elmer, Rodgau-Jügesheim, Germany) following 30 minutes of incubation at RT in the dark. For each well, the C3 mRNA level was normalized to the respective GAPDH mRNA level. The activity of a given C3 siRNA was expressed as percent remaining C3 mRNA concentration (normalized to GAPDH mRNA) in treated cells, relative to the C3 mRNA concentration (normalized to GAPDH mRNA) averaged across control wells.

**Table 2**

| | **% remaining mRNA** | | | **% remaini ing mRNA** | |
|---|---|---|---|---|---|
| **Duplex ID** | **Mean** | **SD** | **Duplex ID** | **Mean** | **SD** |
| EV0001 | 8.87 | 0.61 | EV0051 | 11.84 | 0.60 |
| EV0002 | 10.61 | 0.72 | EV0052 | 18.23 | 1.26 |
| EV0003 | 10.68 | 0.82 | EV0053 | 15.40 | 0.60 |
| EV0004 | 9.59 | 0.43 | EV0054 | 12.80 | 0.69 |
| EV0005 | 22.27 | 0.97 | EV0055 | 44.97 | 4.50 |
| EV0006 | 13.10 | 1.16 | EV0056 | 83.23 | 5.03 |
| EV0007 | 8.89 | 0.90 | EV0057 | 55.45 | 3.76 |
| EV0008 | 6.02 | 0.47 | EV0058 | 22.12 | 0.84 |
| EV0009 | 6.80 | 0.45 | EV0059 | 12.74 | 0.48 |
| EV0010 | 20.18 | 1.32 | EV0060 | 14.38 | 0.79 |
| EV0011 | 10.09 | 0.34 | EV0061 | 12.92 | 0.83 |
| EV0012 | 9.48 | 1.06 | EV0062 | 13.26 | 0.83 |
| EV0013 | 7.40 | 1.08 | EV0063 | 21.32 | 1.61 |
| EV0014 | 9.75 | 1.25 | EV0064 | 15.14 | 0.86 |
| EV0015 | 13.08 | 1.52 | EV0065 | 12.01 | 0.42 |
| EV0016 | 16.68 | 0.66 | EV0066 | 13.14 | 1.32 |
| EV0017 | 47.72 | 1.80 | EV0067 | 13.57 | 0.61 |
| EV0018 | 7.86 | 0.77 | EV0068 | 24.89 | 0.87 |
| EV0019 | 18.83 | 1.08 | EV0069 | 15.36 | 2.68 |
| EV0020 | 8.80 | 0.87 | EV0070 | 16.50 | 1.29 |
| EV0021 | 13.88 | 0.98 | EV0071 | 9.69 | 0.71 |
| EV0022 | 79.91 | 4.20 | EV0072 | 25.55 | 1.40 |
| EV0023 | 13.32 | 1.29 | EV0073 | 12.79 | 1.34 |
| EV0024 | 11.11 | 0.76 | EV0074 | 14.63 | 0.51 |
| EV0025 | 16.35 | 0.50 | EV0075 | 13.05 | 0.83 |
| EV0026 | 10.03 | 0.88 | EV0076 | 16.29 | 0.87 |
| EV0027 | 10.11 | 1.03 | EV0077 | 18.63 | 0.98 |
| EV0028 | 11.83 | 0.53 | EV0078 | 21.78 | 1.18 |
| EV0029 | 9.71 | 1.02 | EV0079 | 20.78 | 1.04 |
| EV0030 | 9.05 | 0.49 | EV0080 | 17.59 | 1.31 |
| EV0031 | 10.70 | 0.64 | EV0081 | 15.27 | 0.70 |
| EV0032 | 12.93 | 0.55 | EV0082 | 20.75 | 1.03 |
| EV0033 | 9.29 | 0.59 | EV0083 | 11.84 | 0.60 |
| EV0034 | 15.20 | 0.50 | EV0084 | 18.23 | 1.26 |
| EV0035 | 16.87 | 0.62 | EV0085 | 15.40 | 0.60 |
| EV0036 | 14.37 | 1.05 | EV0086 | 12.80 | 0.69 |
| EV0037 | 15.36 | 2.68 | EV0087 | 44.97 | 4.50 |
| EV0038 | 16.50 | 1.29 | EV0088 | 83.23 | 5.03 |
| EV0039 | 9.69 | 0.71 | EV0089 | 55.45 | 3.76 |
| EV0040 | 25.55 | 1.40 | EV0090 | 22.12 | 0.84 |
| EV0041 | 12.79 | 1.34 | EV0091 | 12.74 | 0.48 |
| EV0042 | 14.63 | 0.51 | EV0092 | 14.38 | 0.79 |
| EV0043 | 13.05 | 0.83 | EV0093 | 12.92 | 0.83 |
| EV0044 | 16.29 | 0.87 | EV0094 | 13.26 | 0.83 |
| EV0045 | 18.63 | 0.98 | EV0095 | 21.32 | 1.61 |
| EV0046 | 21.78 | 1.18 | EV0096 | 15.14 | 0.86 |
| EV0047 | 20.78 | 1.04 | EV0097 | 12.01 | 0.42 |
| EV0048 | 17.59 | 1.31 | EV0098 | 13.14 | 1.32 |
| EV0049 | 15.27 | 0.70 | EV0099 | 13.57 | 0.61 |
| EV0050 | 20.75 | 1.03 | EV0100 | 24.89 | 0.87 |

Table 2: Results of screening of C3 siRNAs - the identity of the single strands forming each of the siRNA duplexes as well as their sequences and modifications are to be found in the tables at the end of the description.

### Example 2

*In vitro* study in HepG2 cells showing C3 mRNA knockdown efficacy of tested siRNAs after transfection of 0.01 pM - 100 nM siRNA (concentration-response-curve experiment).

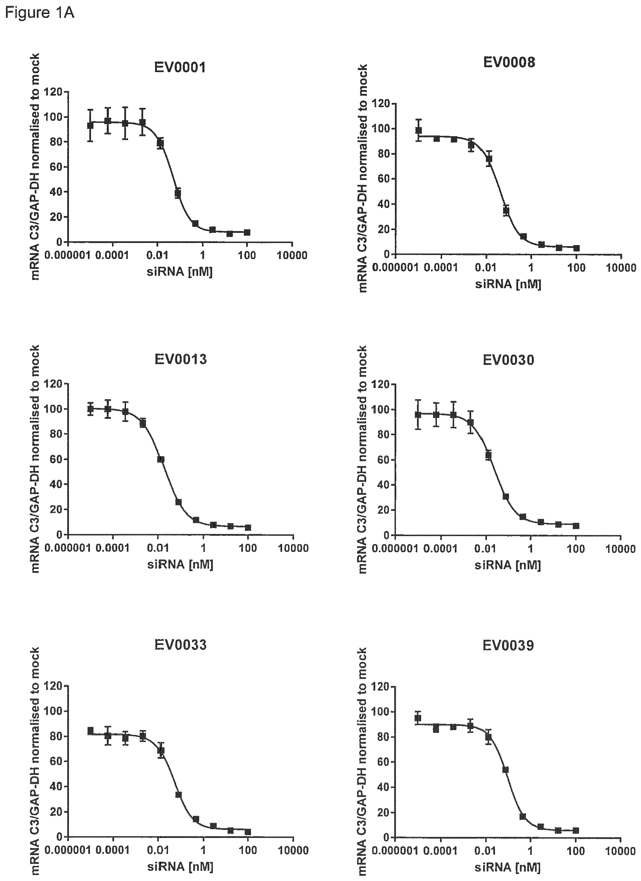

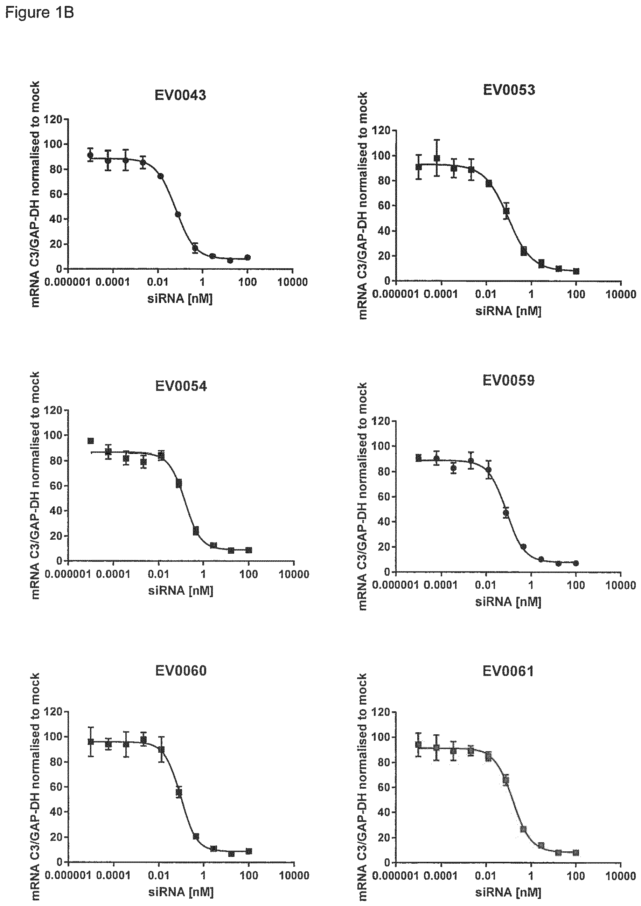

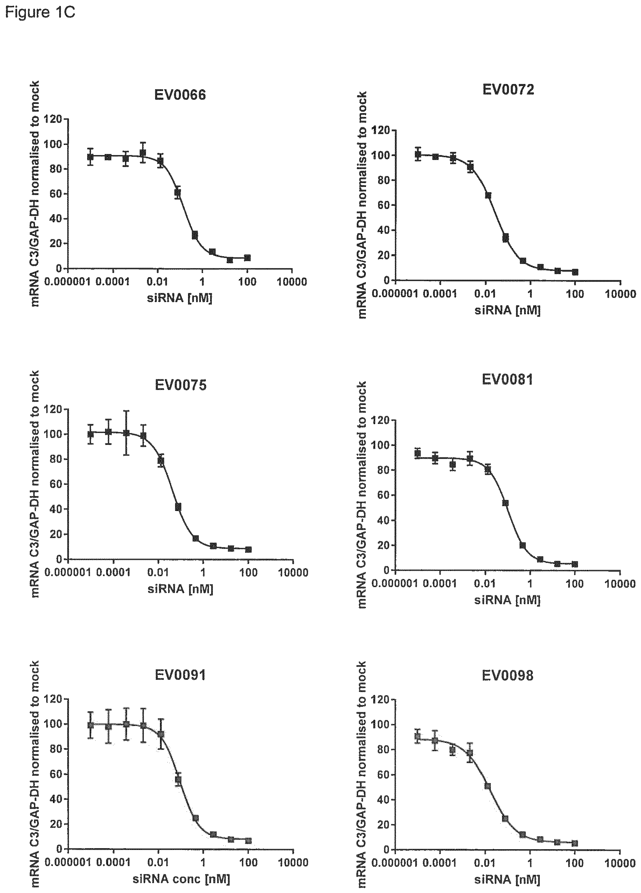

C3 knockdown efficacy of siRNAs EV0001, EV0008, EV0013, EV0030, EV0033, EV0039, EV0043, EV0053, EV0054, EV0059, EV0060, EV0061, EV0066, EV0072, EV0075, EV0081, EV0091 and EV0098 was determined after transfection of 0.01 pM -100 nM siRNA in HepG2 cells. The identity of the single strands forming each of the siRNA duplexes as well as their sequences are to be found in the tables at the end of the description. Results are presented in Figures 1A, 1B and 1C. All siRNAs showed a dose-dependent knockdown of C3 mRNA after transfection. The most potent siRNAs were EV0008, EV0033 and EV0081, with a residual C3 expression of 4.3, 5.3 and 5.3 % at 100 nM siRNA, respectively.

For transfection of HepG2 cells with siRNAs, cells were seeded at a density of 15,000 cells / well into collagen-coated 96-well tissue culture plates (#655150, GBO, Germany). Transfection of siRNA was carried out with Lipofectamine RNAiMax (Invitrogen/Life Technologies, Karlsruhe, Germany) according to the manufacturer's instructions directly after seeding. Concentration-response experiments were done with C3 siRNA in 10 concentrations transfected in quadruplicates, starting at 100 nM in 6-fold dilution steps down to 0.01 pM. Mock transfected cells served as control in CRC experiments. After 24h of incubation with siRNAs, medium was removed and cells were lysed in 150µl Medium-Lysis Mixture (1 volume lysis mixture, 2 volumes cell culture medium) and then incubated at 53°C for 30 minutes. bDNA assay was performed according to the manufacturer's instructions. Luminescence was read using 1420 Luminescence Counter (WALLAC VICTOR Light, Perkin Elmer, Rodgau-Jügesheim, Germany) following 30 minutes of incubation at RT in the dark. For each well, the C3 mRNA level was normalized to the respective GAPDH mRNA level. The activity of a given C3 siRNA was expressed as percent remaining C3 mRNA (normalized to GAPDH mRNA) in treated cells, relative to the C3 mRNA concentration (normalized to GAPDH mRNA) averaged across control wells. Concentration-response-curves were fitted with GraphPad Prism version 7.05 using a four-parameter logistic (4PL) model without further constraints.

### Example 3

*In vitro* study in primary human hepatocytes showing C3 mRNA knockdown efficacy of tested siRNA-GalNAc conjugates in concentration-response-curve format (0.038 nM - 10 µM siRNA conjugate).

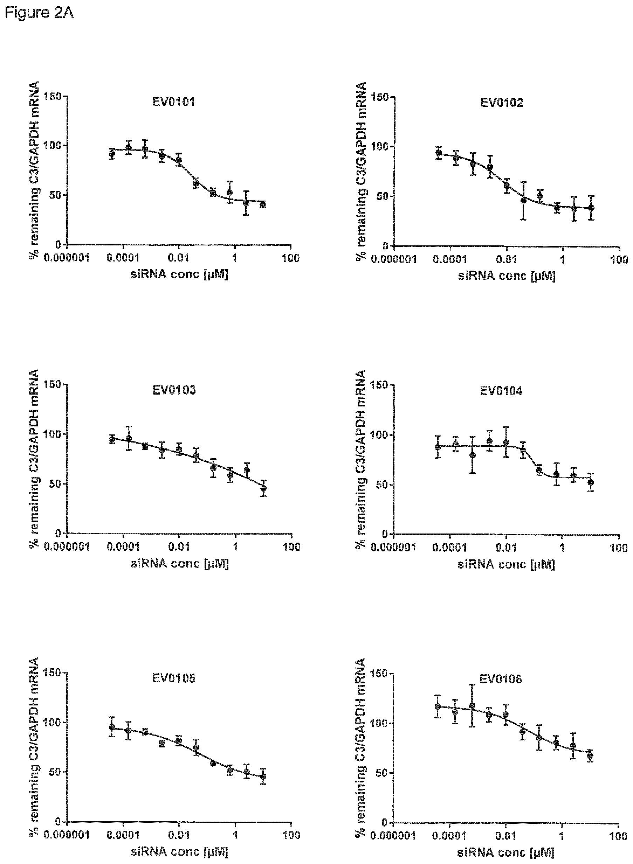

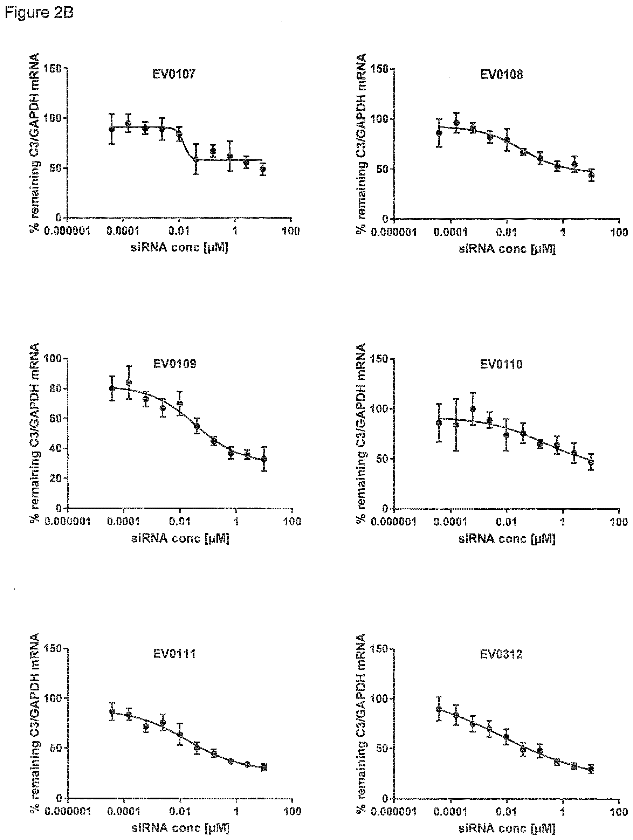

Expression of C3 mRNA after incubation with the GalNAc siRNA conjugates EV0101, EV0102, EV0103, EV0104, EV0105, EV0106, EV0107, EV0108, EV0109, EV0110, EV0111 and EV0312 was analysed in a concentration-response format. The identity of the single strands forming each of the siRNA duplexes as well as their sequences are to be found in the tables at the end of the description. Results are shown in Figures 2A and 2B. The mRNA level of the house keeping gene GAPDH served as control for all experiments. All siRNA GalNAc conjugates were able to decrease C3 mRNA level in a concentration-dependent fashion with maximal inhibition at 10 µM between 32 and 70 %, respectively. The most potent siRNAs were EV102 with 61 %, EV109 with 67 %, EV0111 with 69 % and EV0312 with 70 % reduction of the C3 mRNA level at 10 µM.

Human cryopreserved primary hepatocytes were purchased from Primacyt (Schwerin, Germany, cat#GuCPI, Lot# BHum16061-P). Directly before treatment, cells were thawed, transferred to a tube with thawing medium (Primacyt, cat#HTM), centrifuged and washed with washing Medium (Primacyt, cat#HWM). Cells were seeded at a density of 90,000 cells per well in plating medium (Primacyt, cat#MPM-cryo) on collagen coated 96-well plates (Greiner-Bio-One, #655150). Directly after seeding, cells were treated with the siRNAs as they adhered as a monolayer in plating medium. Each siRNA was applied to the cells for the concentration-response-curve at concentrations starting with 10 µM, sequentially diluted in 4-fold dilution steps down to 38 pM. Each concentration was applied as quadruplicate. After 5 hours, the medium was changed to maintenance medium (Primacyt cat#HHMM). The medium was changed every 24 hours and the cells were harvested for analysis by Quantigene bDNA assay 48 hours after seeding. The C3 mRNA concentrations were normalised to GAPDH mRNA. Concentration-response-curves were fitted with GraphPad Prism version 7.05 using a four-parameter logistic (4PL) model without further constraints.

### Example 4

*In vitro* study in primary mouse hepatocytes showing C3 mRNA knockdown efficacy of tested siRNA-GalNAc conjugates in concentration-response-curve format (0.038 nM - 10 µM siRNA conjugate).

Expression of C3 mRNA after incubation with the GalNAc siRNA conjugates EV0104, EV0105, EV0107, EV0108, EV0109, EV0110, EV0111 and EV0312 in a concentration-response format was analysed. The identity of the single strands forming each of the siRNA duplexes as well as their sequences are to be found in the tables at the end of the description. The mRNA level of the house keeping gene GAPDH served as control. The siRNA GalNAc conjugates were able to decrease C3 mRNA levels in a concentration dependent fashion with maximal inhibition at 10 µM between 56 and 72 %. The most potent siRNAs were EV0104 with 68 %, EV0109 with 67 % and EV0111 with 72 % reduction of C3 mRNA at 10 µM, respectively.

Cryopreserved murine hepatocytes were purchased from Thermo Fisher (#MSCP10, Lot#MC817) and plated in plating medium (Thermo Fisher Sci, Cat. No. CM3000 supplement pack added to William's E Medium, no phenol red - to 500 ml total, Thermo Fisher Sci, Cat. No. A12176-01). On the day of seeding, the cells were thawed and plated at a density of 60,000 cells per well into a collagen-coated 96-well plate (Greiner-Bio-One, #655150). Directly after seeding, cells were treated with the siRNAs as they adhered as a monolayer in plating medium. Each siRNA was applied to the cells as concentration-response-curve at concentrations starting with 10 µM, sequentially diluted in 4-fold dilution steps down to 0.038 nM. Each concentration was applied as quadruplicate. After 5 hours, the medium was changed to maintenance medium (Thermo Fisher Sci, Cat. No. CM4000 supplement pack added to William's E Medium, no phenol red - to 500 ml total, Thermo Fisher Sci, Cat. No. A12176-01). The medium was changed every 24 hours and the cells were harvested for analysis by Quantigene bDNA assay 48 hours after seeding.

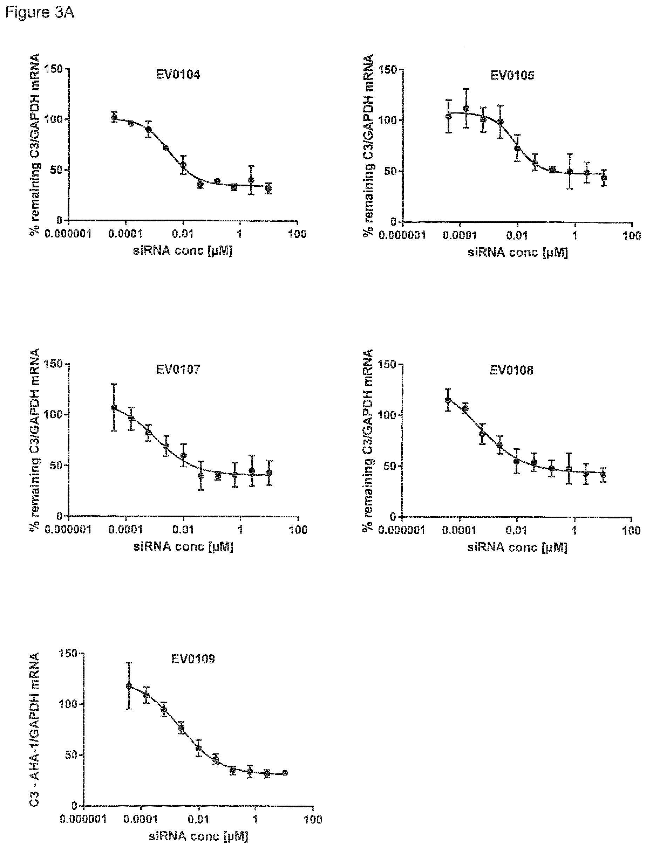

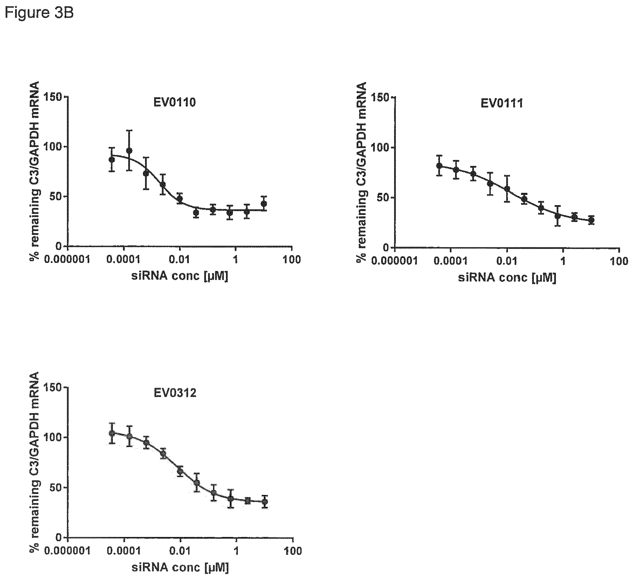

The results are shown in Figures 3A and 3B. They depict % remaining C3 mRNA expression in primary mouse hepatocytes after incubation with siRNA GalNAc conjugates (0.038 nM - 10 µM) normalized to GAPDH mRNA. Concentration-response curves were fitted with GraphPad Prism version 7.05 using a four-parameter logistic (4PL) model without further constraints.

### Example 5

*In vitro* study in primary mouse, human and cynomolgus hepatocytes showing C3 mRNA knockdown efficacy of tested siRNA-GalNAc conjugates at 1, 10 and 100nM.

The expression of C3 mRNA after incubation with the GalNAc siRNA conjugates EV0312 and EV0313 at 1, 10 and 100nM was analysed. The identity of the single strands forming each of the siRNA duplexes as well as their sequences are to be found in the tables at the end of the description. The mRNA level of the house keeping gene Actin served as control.

40,000 (human), 30,000 (mouse) or 45,000 (cynomolgus) cells were seeded on collagen-coated 96-well plates. siRNAs in indicated concentrations were added immediately after seeding. 24 hours post treatment, cells were lysed using InviTrap RNA Cell HTS96 Kit/C (Stratec). qPCR was performed using mRNA-specific primers and probes against C3 and Actin.

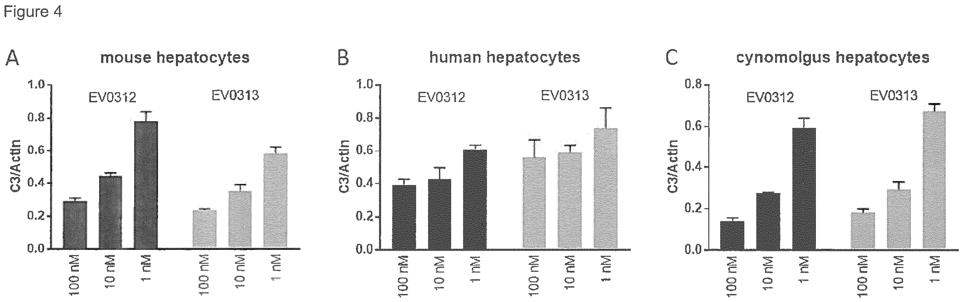

The results are shown in Figures 4A, 4B and 4C.

### Example 6 - Synthesis of building blocks

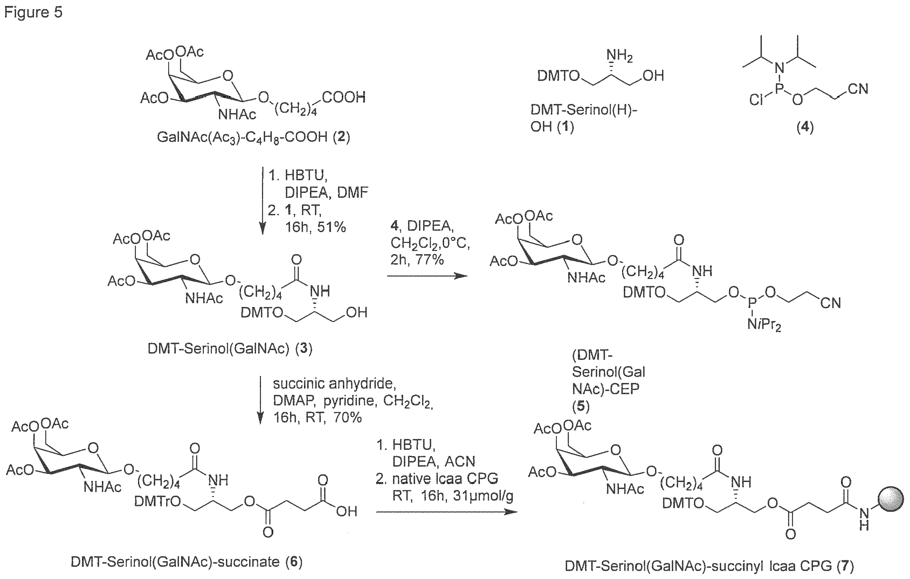

The synthesis route for DMT-Serinol(GalNAc)-CEP and CPG as described below is outlined in Figure 5. Starting material DMT-Serinol(H) (**1**) was made according to literature published methods (Hoevelmann et al. Chem. Sci., 2016,7, 128-135) from commercially available L-Serine. GalNAc(Ac₃)-C₄H₈-COOH (**2**) was prepared according to literature published methods (Nair et al. J. Am. Chem. Soc., 2014, 136 (49), pp 16958-1696), starting from commercially available per-acetylated galactose amine. Phosphitylation reagent 2-Cyanoethyl-N,N-diisopropylchlorophosphor-amidite (**4**) is commercially available. Synthesis of (vp)-mU-phos was performed as described in Prakash, Nucleic Acids Res. 2015, 43(6), 2993-3011 and Haraszti, Nucleic Acids Res. 2017, 45(13), 7581-7592. Synthesis of the phosphoramidite derivatives of ST43 (ST43-phos) as well as ST23 (ST23-phos) can be performed as described in WO2017/174657.

### DMT-Serinol(GalNAc) (3)

HBTU (9.16 g, 24.14 mmol) was added to a stirring solution of GalNAc(Ac₃)-C₄H₈-COOH (**2**) (11.4 g, 25.4 mmol) and DIPEA (8.85 ml, 50.8 mmol). After 2 minutes activation time a solution of DMT-Serinol(H) (**1**) (10 g, 25.4 mmol) in Acetonitrile (anhydrous) (200 ml) was added to the stirring mixture. After 1h LCMS showed good conversion. The reaction mixture was concentrated in vacuo. The residue was dissolved up in EtOAc, washed subsequently with water (2x) and brine. The organic layer was dried over Na₂SO₄, filtered and concentrated under reduced pressure. The residue was further purified by column chromatography (3% MeOH in CH₂Cl₂ + 1% Et₃N, 700g silica). Product containing fractions were pooled, concentrated and stripped with CH₂Cl₂ (2x) to yield to yield 10.6g (51%) of DMT-Serinol(GalNAc) (**3**) as an off-white foam.

### DMT-Serinol(GalNAc)-CEP (5)

2-Cyanoethyl-N,N-diisopropylchlorophosphoramidite (**4**) (5.71 ml, 25.6 mmol) was added slowly to a stirring mixture of DMT-Serinol(GalNAc) (**3**) (15.0 g, 17.0 mmol), DIPEA (14.9 ml, 85 mmol) and 4Å molecular sieves in Dichloromethane (dry) (150 ml) at 0°C under argon atmosphere. The reaction mixture was stirred at 0°C for 1h. TLC indicated complete conversion. The reaction mixture was filtered and concentrated in vacuo to give a thick oil. The residue was dissolved in Dichloromethane and was further purified by flash chromatography (0-50% acetone in toluene 1%Et3N, 220 g silica). Product containing fractions were pooled and concentrated in vacuo. The resulting oil was stripped with MeCN (2x) to yield 13.5g (77%) of the colorless DMT-Serinol(GalNAc)-CEP (5) foam.

### DMT-Serinol(GalNAc)-succinate (6)

DMAP (1.11 g, 9.11 mmol) was added to a stirring solution of DMT-Serinol(GalNAc) (**3**) (7.5 g, 9.11 mmol) and succinic anhydride (4.56 g, 45.6 mmol) in a mixture of Dichloromethane (50 ml) and Pyridine (50 ml) under argon atmosphere. After 16h of stirring the reaction mixture was concentrated in vacuo and the residue was taken up in EtOAc and washed with 5% citric acid (aq). The aqueous layer was extracted with EtOAc. The combined organic layers were washed subsequently with sat NaHCO₃ (aq.) and brine, dried over Na₂SO₄, filtered and concentrated in vacuo. Further purification was achieved by flash chromatography (0-5% MeOH in CH₂Cl₂ +1% Et₃N, 120g silica). Product containing fractions were pooled and concentrated in vacuo. The residue was stripped with MeCN (3x) to yield 5.9g (70%) DMT-Serinol(GalNAc)-succinate (**6**).

### DMT-Serinol(GalNAc)-succinyl-lcaa-CPG (7)

The DMT-Serinol(GalNAc)-succinate (**6**) (1 eq.) and HBTU (1.1 eq.) were dissolved in CH₃CN (10 ml). Diisopropylethylamine (2 eq.) was added to the solution, and the mixture was swirled for 2 min followed by addition native amino-Icaa-CPG (500 A, 88µmol/g, 1 eq.). The suspension was gently shaken at room temperature on a wrist-action shaker for 16h, then filtered and washed with acetonitrile. The solid support was dried under reduced pressure for 2 h. The unreacted amines on the support were capped by stirring with Ac₂O/2,6-lutidine/NMI at room temperature (2x15min). The washing of the support was repeated as above. The solid was dried under vacuum to yield DMT-Serinol(GalNAc)-succinyl-lcaa-CPG (**7**) (loading: 34 µmol/g, determined by detritylation assay).

### Example 7 - Oligonucleotide Synthesis

Example compounds were synthesised according to methods described below and known to the person skilled in the art. Assembly of the oligonucleotide chain and linker building blocks was performed by solid phase synthesis applying phosphoramidite methodology.

Downstream cleavage, deprotection and purification followed standard procedures that are known in the art.

Oligonucleotide syntheses was performed on an AKTA oligopilot 10 using commercially available 2'O-Methyl RNA and 2'Fluoro-2'Deoxy RNA base loaded CPG solid support and phosphoramidites (all standard protection, ChemGenes, LinkTech) were used. Synthesis of DMT-(S)-Serinol(GalNAc)-succinyl Icaa CPG (**7**) and DMT-(S)-Serinol(GalNAc)-CEP (**5**) are described in example 6.

Ancillary reagents were purchased from EMP Biotech. Synthesis was performed using a 0.1 M solution of the phosphoramidite in dry acetonitrile (<20 ppm H₂O) and benzylthiotetrazole (BTT) was used as activator (0.3M in acetonitrile). Coupling time was 10 min. A Cap/OX/Cap or Cap/Thio/Cap cycle was applied (Cap: Ac₂O/NMI/Lutidine/Acetonitrile, Oxidizer: 0.05M I₂ in pyridine/H₂O). Phosphorothioates were introduced using commercially available thiolation reagent 50mM EDITH in acetonitrile (Link technologies). DMT cleavage was achieved by treatment with 3% dichloroacetic acid in toluene. Upon completion of the programmed synthesis cycles a diethylamine (DEA) wash was performed. All oligonucleotides were synthesized in DMT-off mode.

Attachment of the Serinol(GalNAc) moiety was achieved by use of either base-loaded (S)-DMT-Serinol(GalNAc)-succinyl-lcaa-CPG (**7**) or a (S)-DMT-Serinol(GalNAc)-CEP (**5**). Triantennary GalNAc clusters (ST23/ST43) were introduced by successive coupling of the branching trebler amidite derivative (C6XLT-phos) followed by the GalNAc amidite (ST23-phos). Attachement of (vp)-mU moiety was achieved by use of (vp)-mU-phos in the last synthesis cycle. The (vp)-mU-phos does not provide a hydroxy group suitable for further synthesis elongation and therefore, does not possess an DMT-group. Hence coupling of (vp)-mU-phos results in synthesis termination.

For the removal of the methyl esters masking the vinylphosphonate, the CPG carrying the fully assembled oligonucleotide was dried under reduced pressure and transferred into a 20 ml PP syringe reactor for solid phase peptide synthesis equipped with a disc frit (Carl Roth GmbH). The CPG was then brought into contact with a solution of 250 µL TMSBr and 177 µL pyridine in CH₂Cl₂ (0.5 ml/µmol solid support bound oligonucleotide) at room temperature and the reactor was sealed with a Luer cap. The reaction vessels were slightly agitated over a period of 2x15 min, the excess reagent discarded, and the residual CPG washed 2x with 10 ml acetonitrile. Further downstream processing did not alter from any other example compound.

The single strands were cleaved off the CPG by 40% aq. methylamine treatment (90 min, RT). The resulting crude oligonucleotide was purified by ion exchange chromatography (Resource Q, 6 ml, GE Healthcare) on a AKTA Pure HPLC System using a sodium chloride gradient. Product containing fractions were pooled, desalted on a size exclusion column (Zetadex, EMP Biotech) and lyophilized until further use.

All final single stranded products were analysed by AEX-HPLC to prove their purity. Identity of the respective single stranded products was proved by LC-MS analysis.

### Example 8 - double strand formation

Individual single strands were dissolved in a concentration of 60 OD/ml in H₂O. Both individual oligonucleotide solutions were added together in a reaction vessel. For easier reaction monitoring a titration was performed. The first strand was added in 25% excess over the second strand as determined by UV-absorption at 260 nm. The reaction mixture was heated to 80°C for 5 min and then slowly cooled to RT. Double strand formation was monitored by ion pairing reverse phase HPLC. From the UV-area of the residual single strand the needed amount of the second strand was calculated and added to the reaction mixture. The reaction was heated to 80°C again and slowly cooled to RT. This procedure was repeated until less than 10% of residual single strand was detected.

### Example 9

*In vivo* study showing knockdown of C3 mRNA in murine liver tissue and serum protein after single subcutaneous dosing of 1 or 5 mg/kg GalNAc conjugated siRNAs.

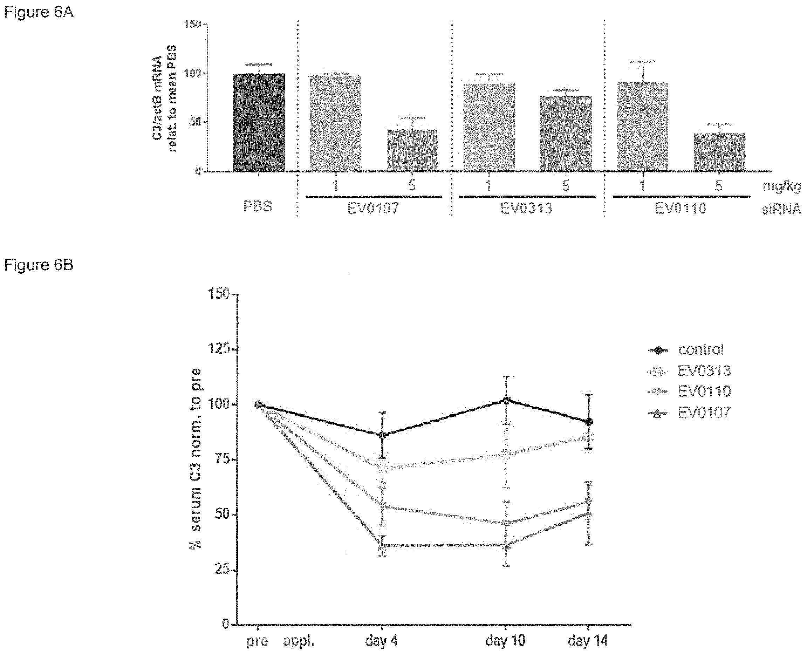

Female C57BL/6N mice with an age of 8 weeks were obtained from CHARLES RIVER, Sulzfeld, Germany. Animal experiments were performed according to ethical guidelines of the German Protection of Animals Act in its version of July 2013. Mice were randomized according to weight into groups of 4 mice. On day 0 of the study animals received a single subcutaneous dose of 1 or 5 mg/kg siRNA dissolved in phosphate buffered saline (PBS) or PBS only as control. The viability, body weight and behaviour of the mice was monitored during the study without pathological findings. Serum samples were taken before the application, at day 4, day 10 and day 14. At day 14 the study was terminated, animals were euthanized, and liver samples were snap frozen and stored at - 80°C until further analysis. For analysis, RNAs were isolated using the InviTrap Spin Tissue RNA Mini Kit from Stratec according to the manufacturer's protocol. QPCR was performed using C3 and Actin specific primer probe sets and Takyon™ One-Step Low Rox Probe 5X MasterMix dTTP on the QuantStudio6 device from Applied Biosystems in single-plex 384 well format. Expression differences were calculated using the delta delta Ct method and relative expression of C3 versus the house keeping gene actin normalized to the PBS control experiment was used for comparison of the different siRNAs. EV0107, EV0313 and EV0110 induced a dose dependent knockdown of liver C3 mRNA. The maximum achieved knockdown was observed using siRNA EV0107 (57%) and EV0110 (61%) using 5 mg/kg siRNA, respectively. Results are shown in Figure 6A. The figure shows relative C3 mRNA expression in % in murine liver 14 days after a single dosing of GalNAc conjugated siRNAs EV0107, EV0313 and EV0110. The identity of the single strands forming each of the siRNA duplexes as well as their sequences are to be found in the tables at the end of the description. Data is shown in bar charts as mean ± SD (n=4 per group).

Serum samples were analysed using commercially available C3 ELISA Kits. The analyses were carried out according to the manufacturer's protocol, and C3 serum levels were calculated relative to the respective pre dose levels. Results are shown in Figure 6B. The figure shows relative C3 protein serum levels in % from mouse serum samples taken before, at day 4, at day 10 and at day 14 of the study after dosing of 5mg/kg EV0313, EV0110 and EV0107 GalNAc conjugated siRNAs. Data is shown as means ± SD (n=3 or 4 per group).

### Example 10

*In vitro* study in primary mouse, human and cynomolgus hepatocytes showing C3 knockdown efficacy of tested siRNA-GalNAc conjugates at 1, 10 an 100nM.

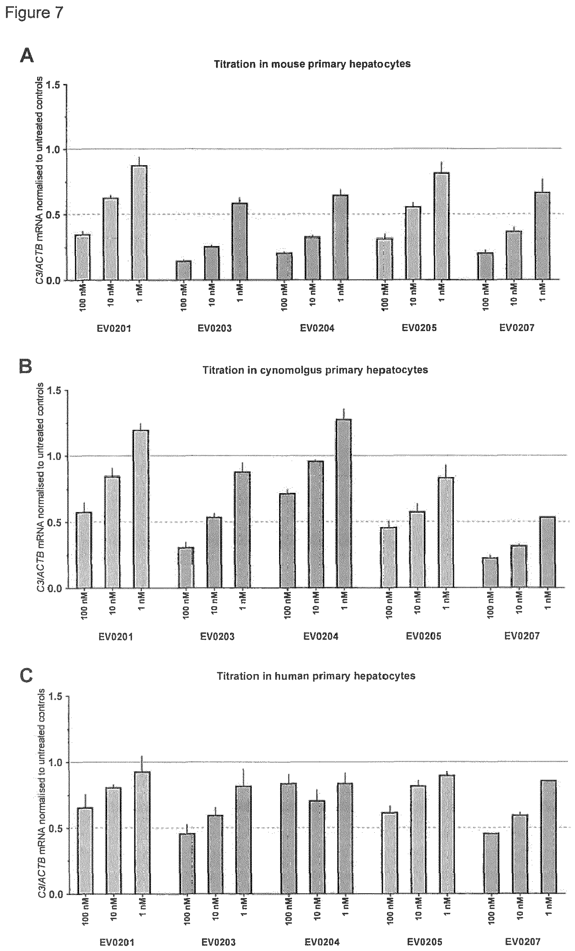

Expression of C3 mRNA after incubation with the GalNAc siRNA conjugates EV0201, EV0203, EV0204, EV0205 and EV0207 at 1, 10 and 100nM was measured (Figure 7). siRNA sequences and modifications are listed in Tables 3 and 5. The mRNA level of the house keeping gene ACTIN served as housekeeping control. Human and cynomolgus primary hepatocytes were seeded into collagen I-coated 96-well plates (Life Technologies) at a density of 40,000 cells per well. Mouse hepatocytes were seeded at a density of 25,000 cells per well. GalNAc-conjugated siRNAs were added immediately after plating in the previously defined media to final siRNA concentrations of 100, 10 and 1 nM. Plates were then incubated at 37 °C in a 5% CO2 atmosphere for 24 hours. Subsequently, cells were lysed and RNA was isolated using InviTrap RNA Cell HTS96 Kit/C (Stratec).

10 µl of RNA-solution was used for gene expression analysis by reverse transcription quantitative polymerase chain reaction (RT-qPCR) performed with amplicon sets/sequences for ACTB (Eurogentec) and C3 (BioTez GmbH, Berlin, Germany), respectively. The RT-qPCR reactions were carried out with an ABI StepOne Plus (Applied Biosystems, part of Thermo Fisher Scientific, Massachusetts, USA) using standard protocols for RT-PCR (48 °C 30 min, 95 °C 10 min, 40 cycles at 95 °C 15 s followed by 60 °C 1 min). The data were calculated by using the comparative CT method also known as the 2-deltadelta Ct method. SiRNAs EV0201, EV0203, EV0204, EV0205 and EV0207 show dose-dependent inhibition of C3 mRNA expression in primary hepatocytes.

### Example 11

*In vivo* study showing knockdown of C3 mRNA in murine liver tissue and serum protein after a single subcutaneous dosing of 5 or 10 mg/kg GalNAc conjugated modified siRNAs.

siRNA sequences and modifications are listed in Tables 3 and 5. The mRNA level of the house keeping gene ACTIN served as housekeeping control. Male C57BL/6N mice with an age of about 8 weeks were obtained from CHARLES RIVER, Sulzfeld, Germany. Animal experiments were conducted in compliance with the principles of the Hungarian Act 1998: XXVIII regulating animal protection (latest modified by Act 2011 CLVIII) and in Government Decree 40/2013 on animal experiments. Mice were assigned into groups of 4 mice. On day 0 of the study, the animals received a single subcutaneous dose of 5 or 10 mg/kg siRNA dissolved in phosphate buffered saline (PBS) or PBS only as control. The viability, body weight and behaviour of the mice was monitored during the study without pathological findings. Serum samples were taken before the application, at day 4, day 10, day 14, day 21, day 28, day 35 and day 42. At day 14 and at day 42 half of the groups, respectively, were terminated, the animals were euthanized, and liver samples were snap frozen and stored at - 80°C until further analysis.

For analysis, RNAs were isolated using the InviTrap Spin Tissue RNA Mini Kit from Stratec according to the manufacturer's protocol. RT-qPCR was performed using C3 and ACTIN specific primer probe sets and Takyon™ One-Step Low Rox Probe 5X MasterMix dTTP on the QuantStudio6 device from Applied Biosystems in single-plex 384 well format. Expression differences were calculated using the delta delta Ct method and relative expression of C3 versus the house keeping gene ACTIN normalized to the PBS group was used for comparison of the different siRNAs.

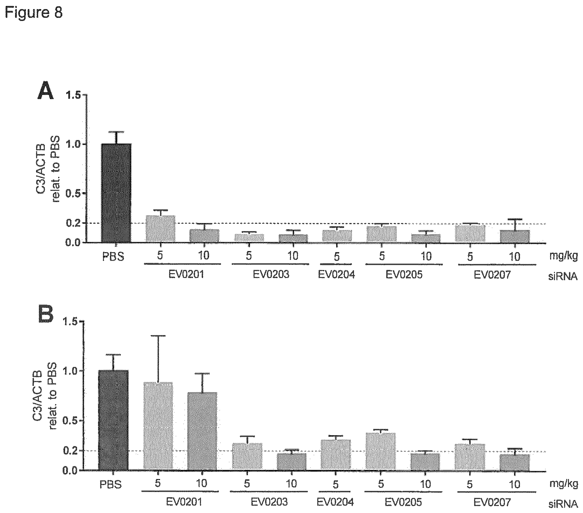

All tested siRNAs (EV0201, EV0203, EV0204, EV0205 and EV0207) inhibit C3 mRNA expression by more than 70 % after 14 days after a single dose of 5 or 10 mg/kg (Figure 8A). After 42 days, the inhibition of C3 expression by EV0203, EV0204, EV0205 and EV0207 was still more than 80 % knockdown with a 10 mg/kg siRNA dose (Figure 8B).

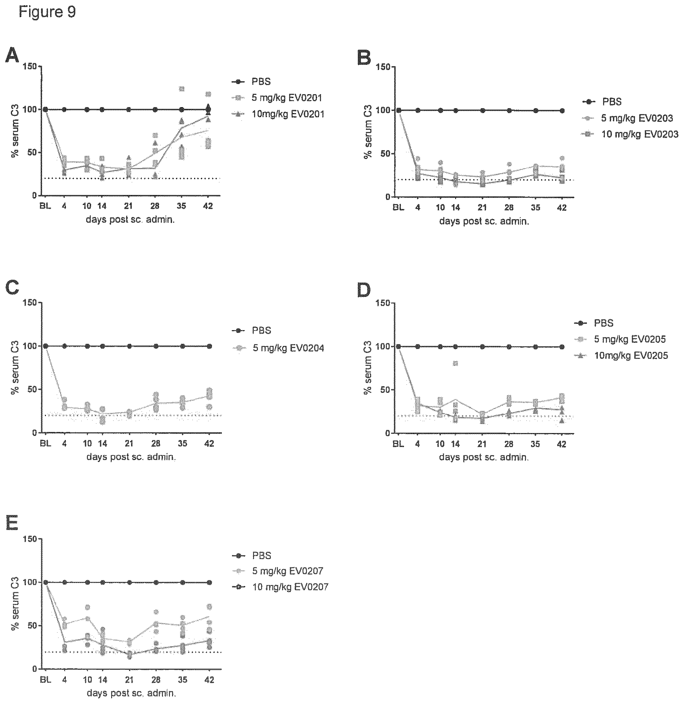

For C3 protein level analysis, serum samples were measured using commercially available C3 ELISA Kits. The analyses were carried out according the manufacturer's protocol, and % C3 serum levels were calculated relative to the group means at baseline/before the application and relative to the time matched PBS control group's means. The data for the C3 protein analyses mirror the results from the RNA analyses (Figure 9). EV0203, EV0204, EV0205 and EV0207 were able to induce a long lasting C3 serum decrease. A reduction of up to 80 % 42 days after a single application of 10 mg/kg was obtained for EV0203 (Figure 9B).

### Statements

The following statements represent aspects of the invention.
1. A double-stranded nucleic acid for inhibiting expression of complement component C3, wherein the nucleic acid comprises a first strand and a second strand, wherein the first strand sequence comprises a sequence of at least 15 nucleotides differing by no more than 3 nucleotides from any one of the sequences SEQ ID NO: 361, 95, 111, 125, 131, 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 97, 99, 101, 103, 105, 107, 109, 113, 115, 117, 119, 121, 123, 127, 129 or 133.
2. The nucleic acid of statement 1, wherein
(a) the first strand sequence comprises a sequence of at least 18 nucleotides differing by no more than 3 nucleotides from any one of the first strand sequences of Table 1 and optionally wherein the second strand sequence comprises a sequence of at least 18 nucleotides differing by no more than 3 nucleotides from the second strand sequence in the same line of the table;
(b) the first strand sequence comprises a sequence of at least 18 nucleotides differing by no more than 1 nucleotide from any one of the first strand sequences of Table 1 and optionally wherein the second strand sequence comprises a sequence of at least 18 nucleotides differing by no more than 1 nucleotide from the second strand sequence in the same line of the table;
(c) the first strand sequence comprises a sequence of at least 18 nucleotides of any one of the first strand sequences of Table 1 and optionally wherein the second strand sequence comprises a sequence of at least 18 nucleotides of the second strand sequence in the same line of the table; or
(d) the first strand sequence consists of any one of the first strand sequences of Table 1 and optionally wherein the second strand sequence consists of the sequence of the second strand sequence in the same line of the table;
wherein Table 1 is:

| **First strand sequence (SEQ ID NO:)** | **Second strand sequence (SEQ ID NO:)** |
|---|---|
| 361 | 112 |
| 95 | 96 |
| 111 | 112 |
| 125 | 126 |
| 131 | 132 |
| 1 | 2 |
| 3 | 4 |
| 5 | 6 |
| 7 | 8 |
| 9 | 10 |
| 11 | 12 |
| 13 | 14 |
| 15 | 16 |
| 17 | 18 |
| 19 | 20 |
| 21 | 22 |
| 23 | 24 |
| 25 | 26 |
| 27 | 28 |
| 29 | 30 |
| 31 | 32 |
| 33 | 34 |
| 35 | 36 |
| 37 | 38 |
| 39 | |
| 41 | 42 |
| 43 | 44 |
| 45 | 46 |
| 47 | 48 |
| 49 | 50 |
| 51 | 52 |
| 53 | 54 |
| 55 | 56 |
| 57 | 58 |
| 59 | 60 |
| 61 | 62 |
| 63 | 64 |
| 65 | 66 |
| 67 | 68 |
| 69 | 70 |
| 71 | 72 |
| 73 | 74 |
| 75 | 76 |
| 77 | 78 |
| 79 | 80 |
| 81 | 82 |
| 83 | 84 |
| 85 | 86 |
| 87 | 88 |
| 89 | 90 |
| 91 | 92 |
| 93 | 94 |
| 97 | 98 |
| 99 | 100 |
| 101 | 102 |
| 103 | 104 |
| 105 | 106 |
| 107 | 108 |
| 109 | 110 |
| 113 | 114 |
| 115 | 116 |
| 117 | 118 |
| 119 | 120 |
| 121 | 122 |
| 123 | 124 |
| 127 | 128 |
| 129 | 130 |
| 133 | 134 |

3. The nucleic acid of any of the preceding statements, wherein the first strand sequence comprises the sequence of SEQ ID NO: 361 and optionally wherein the second strand sequence comprises a sequence of at least 15 nucleotides of the sequence of SEQ ID NO: 112; or wherein the first strand sequence comprises the sequence of SEQ ID NO: 95 and optionally wherein the second strand sequence comprises a sequence of at least 15 nucleotides of the sequence of SEQ ID NO: 96; or wherein the first strand sequence comprises the sequence of SEQ ID NO: 125 and optionally wherein the second strand sequence comprises a sequence of at least 15 nucleotides of the sequence of SEQ ID NO: 126; or wherein the first strand sequence comprises the sequence of SEQ ID NO: 131 and optionally wherein the second strand sequence comprises a sequence of at least 15 nucleotides of the sequence of SEQ ID NO: 132.
4. A double-stranded nucleic acid that is capable of inhibiting expression of complement component C3 for use as a medicament.
5. The nucleic acid of any of the preceding statements, wherein the first strand and the second strand are separate strands and are each 18-25 nucleotides in length.
6. The nucleic acid of any of the preceding statements, wherein the first strand and the second strand form a duplex region from 17-25 nucleotides in length.
7. The nucleic acid of any of the preceding statements, wherein the duplex region consists of 17-25 consecutive nucleotide base pairs.
8. The nucleic acid of any of the preceding statements, wherein said nucleic acid:
a) is blunt ended at both ends;
b) has an overhang at one end and a blunt end at the other end; or
c) has an overhang at both ends.
9. The nucleic acid of any of the preceding statements, wherein the nucleic acid is a siRNA.
10. The nucleic acid of any of the preceding statements, wherein the nucleic acid mediates RNA interference.
11. The nucleic acid of any of the preceding statements, wherein at least one nucleotide of the first and/or second strand is a modified nucleotide.
12. The nucleic acid of any of the preceding statements, wherein at least nucleotides 2 and 14 of the first strand are modified by a first modification, the nucleotides being numbered consecutively starting with nucleotide number 1 at the 5' end of the first strand.
13 The nucleic acid of any of the preceding statements, wherein each of the even-numbered nucleotides of the first strand are modified by a first modification, the nucleotides being numbered consecutively starting with nucleotide number 1 at the 5' end of the first strand.
14. The nucleic acid of any of statements 12-13, wherein the odd-numbered nucleotides of the first strand are modified by a second modification, wherein the second modification is different from the first modification.
15. The nucleic acid of statements 12-14, wherein the nucleotides of the second strand in a position corresponding to an even-numbered nucleotide of the first strand are modified by a third modification, wherein the third modification is different from the first modification.
16. The nucleic acid of statements 12-15, wherein the nucleotides of the second strand in a position corresponding to an odd-numbered nucleotide of the first strand are modified by a fourth modification, wherein the fourth modification is different from the second modification and different from the third modification when a second and/or a third modification are present.
17. The nucleic acid of statements 12-14, wherein the nucleotide/nucleotides of the second strand in a position corresponding to nucleotide 11 or nucleotide 13 or nucleotides 11 and 13 or nucleotides 11-13 of the first strand is/are modified by a fourth modification and preferably wherein the nucleotides of the second strand that are not modified by a fourth modification are modified by a third modification.
18. The nucleic acid of statements 12-17, wherein the first modification is the same as the fourth modification if both modifications are present in the nucleic acid and preferably wherein the second modification is the same as the third modification if both modifications are present in the nucleic acid.
19. The nucleic acid of statements 12-18, wherein the first modification is a 2'-F modification; the second modification, if present in the nucleic acid, is preferably a 2'-OMe modification; the third modification, if present in the nucleic acid, is preferably a 2'-OMe modification; and the fourth modification, if present in the nucleic acid, is preferably a 2'-F modification.
20. The nucleic acid of any of the preceding statements, wherein each of the nucleotides of the first strand and of the second strand is a modified nucleotide.
21. The nucleic acid of any of the previous statements, wherein the first strand has a terminal 5' (E)-vinylphosphonate nucleotide at its 5' end and wherein the terminal 5' (E)-vinylphosphonate nucleotide is preferably linked to the second nucleotide in the first strand by a phosphodiester linkage.
22. The nucleic acid of any of the preceding statements, wherein the nucleic acid comprises a phosphorothioate linkage between the terminal two or three 3' nucleotides and/or 5' nucleotides of the first and/or the second strand and preferably wherein the linkages between the remaining nucleotides are phosphodiester linkages.
23. The nucleic acid of any of statements 1-21, comprising a phosphorodithioate linkage between each of the two, three or four terminal nucleotides at the 3' end of the first strand and/or comprising a phosphorodithioate linkage between each of the two, three or four terminal nucleotides at the 3' end of the second strand and/or a phosphorodithioate linkage between each of the two, three or four terminal nucleotides at the 5' end of the second strand and comprising a linkage other than a phosphorodithioate linkage between the two, three or four terminal nucleotides at the 5' end of the first strand.
24. The nucleic acid of statement 23, wherein the nucleic acid comprises a phosphorothioate linkage between each of the three terminal 3' nucleotides and/or between each of the three terminal 5' nucleotides on the first strand, and/or between each of the three terminal 3' nucleotides and/or between each of the three terminal 5' nucleotides of the second strand when there is no phosphorodithioate linkage present at that end.
25 The nucleic acid of statement 23, wherein all the linkages between the nucleotides of both strands other than the linkage between the two terminal nucleotides at the 3' end of the first strand and the linkages between the two terminal nucleotides at the 3' end and at the 5' end of the second strand are phosphodiester linkages.
26. The nucleic acid of any of the preceding statements, wherein the nucleic acid is conjugated to a ligand.
27. The nucleic acid of statement 26, wherein the ligand comprises (i) one or more N-acetyl galactosamine (GalNAc) moieties or derivatives thereof, and (ii) a linker, wherein the linker conjugates the at least one GalNAc moiety or derivative thereof to the nucleic acid.
28. The nucleic acid of any of statements 1-27, wherein the nucleic acid is conjugated to a ligand comprising a compound of formula (II):

[S-X¹-P-X²]₃-A-X³- (II)

wherein:
S represents a saccharide, preferably wherein the saccharide is N-acetyl galactosamine;
X¹ represents C₃-C₆ alkylene or (-CH₂-CH₂-O)ₘ(-CH₂)₂- wherein m is 1, 2, or 3;
P is a phosphate or modified phosphate, preferably a thiophosphate;
X² is alkylene or an alkylene ether of the formula (-CH₂)ₙ-O-CH₂- where n = 1- 6;
A is a branching unit;
X₃ represents a bridging unit;
wherein a nucleic acid as defined in any of statements 1 to 27 is conjugated to X³ via a phosphate or modified phosphate, preferably a thiophosphate.
29. The nucleic acid of any of statements 1-27, wherein the first strand of the nucleic acid is a compound of formula (V): 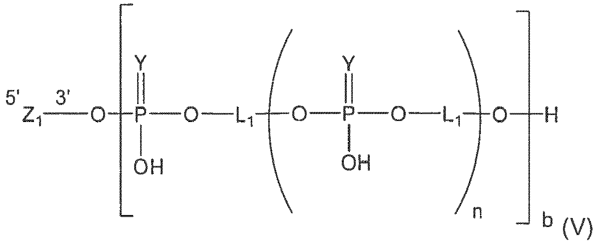 wherein b is 0 or 1; and
wherein the second strand is a compound of formula (VI): 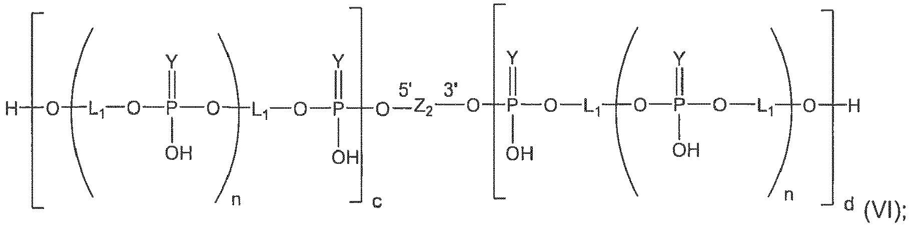 wherein:
c and d are independently 0 or 1;
Z₁ and Z₂ are respectively the first and second strand of the nucleic acid;
Y is independently O or S;
n is independently 0, 1, 2 or 3; and
L₁ is a linker to which a ligand is attached, wherein L₁ is the same or different in formulae (V) and (VI), and is the same or different within formulae (V) and (VI) when L₁ is present more than once within the same formula;
and wherein b + c + d is 2 or 3.
30. A composition comprising a nucleic acid of any of the previous statements and a delivery vehicle and/or a physiologically acceptable excipient and/or a carrier and/or a diluent and/or a buffer and/or a preservative.
31. A composition comprising a nucleic acid of any of statements 1-29 and a further therapeutic agent selected from the group comprising an oligonucleotide, a small molecule, a monoclonal antibody, a polyclonal antibody and a peptide.
32. A nucleic acid of any of statements 1-3 or 5-29 or a composition of any of statements 30-31 for use as a medicament.
33. A nucleic acid of any of statements 1-29 or a composition of any of statements 30-32 for use in the prevention, decrease of the risk of suffering from, or treatment of a disease, disorder or syndrome.
34. The nucleic acid or composition of statement 33, wherein the disease, disorder or syndrome is a complement-mediated disease, disorder or syndrome.
35. The nucleic acid or composition of any of statements 33-34, wherein the disease, disorder or syndrome is associated with aberrant activation or over-activation of the complement pathway and/or with over-expression or ectopic expression or localisation or accumulation of C3.
36. The nucleic acid or composition of any of statements 33-35, wherein the disease, disorder or syndrome is:
a) selected from the group comprising C3 Glomerulopathy (C3G), Paroxysmal Nocturnal Hemoglobinuria (PNH), atypical Hemolytic Uremic Syndrome (aHUS), Lupus nephritis, IgA nephropathy (IgA N), Primary Membranous Nephropathy, Immune Complex-mediated Glomerulonephritis (IC-mediated GN), post-Infectious Glomerulonephritis (PIGN), Systemic Lupus Erythematosus (SLE), Ischemia/reperfusion injury, age-related macular degeneration (AMD), Rheumatoid arthritis (RA), antineutrophil Cytoplasmic Autoantibodies-associated Vasculitis (ANCA-AV), dysbiotic periodontal Disease, Malarial Anaemia and sepsis;
b) selected from the group comprising C3 Glomerulopathy (C3G), Paroxysmal Nocturnal Hemoglobinuria (PNH), atypical Hemolytic Uremic Syndrome (aHUS), Lupus nephritis, IgA nephropathy (IgA N) and Primary Membranous Nephropathy; or
c) C3 Glomerulopathy (C3G).
37. Use of a nucleic acid of any of statements 1-29 or a composition of any of statements 30-31 in the prevention, decrease of the risk of suffering from, or treatment of a disease, disorder or syndrome, wherein the disease, disorder or syndrome is preferably C3 Glomerulopathy (C3G).
38. A method of preventing, decreasing the risk of suffering from, or treating a disease, disorder or syndrome comprising administering a pharmaceutically effective dose of a nucleic acid of any of statements 1-29 or 32-36 or a composition of any of statements 30-36 to an individual in need of treatment, preferably wherein the nucleic acid or composition is administered to the subject subcutaneously, intravenously or by oral, rectal or intraperitoneal administration.

### Summary tables

**Summary duplex table - Table 3**

| **Duplex** | **Single Strands** | **Duplex** | **Single Strands** | **Duplex** | **Single Strands** |
|---|---|---|---|---|---|
| EV0001 | EV0001A | EV0042 | EV0042A | EV0083 | EV0051A |
| | EV0001B | | EV0042B | | EV0083B |
| EV0002 | EV0002A | EV0043 | EV0043A | EV0084 | EV0052A |
| | EV0002B | | EV0043B | | EV0084B |
| EV0003 | EV0003A | EV0044 | EV0044A | EV0085 | EV0053A |
| | EV0003B | | EV0044B | | EV0085B |
| EV0004 | EV0004A | EV0045 | EV0045A | EV0086 | EV0054A |
| | EV0004B | | EV0045B | | EV0086B |
| EV0005 | EV0005A | EV0046 | EV0046A | EV0087 | EV0055A |
| | EV0005B | | EV0046B | | EV0087B |
| EV0006 | EV0006A | EV0047 | EV0047A | EV0088 | EV0056A |
| | EV0006B | | EV0047B | | EV0088B |
| EV0007 | EV0007A | EV0048 | EV0048A | EV0089 | EV0057A |
| | EV0007B | | EV0048B | | EV0089B |
| EV0008 | EV0008A | EV0049 | EV0049A | EV0090 | EV0058A |
| | EV0008B | | EV0049B | | EV0090B |
| EV0009 | EV0009A | EV0050 | EV0050A | EV0091 | EV0059A |
| | EV0009B | | EV0050B | | EV0091B |
| EV0010 | EV0010A | EV0051 | EV0051A | EV0092 | EV0060A |
| | EV0010B | | EV0051B | | EV0092B |
| EV0011 | EV0011A | EV0052 | EV0052A | EV0093 | EV0061A |
| | EV0011B | | EV0052B | | EV0093B |
| EV0012 | EV0012A | EV0053 | EV0053A | EV0094 | EV0062A |
| | EV0012B | | EV0053B | | EV0094B |
| EV0013 | EV0013A | EV0054 | EV0054A | EV0095 | EV0063A |
| | EV0013B | | EV0054B | | EV0095B |
| EV0014 | EV0014A | EV0055 | EV0055A | EV0096 | EV0064A |
| | EV0014B | | EV0055B | | EV0096B |
| EV0015 | EV0015A | EV0056 | EV0056A | EV0097 | EV0065A |
| | EV0015B | | EV0056B | | EV0097B |
| EV0016 | EV0016A | EV0057 | EV0057A | EV0098 | EV0066A |
| | EV0016B | | EV0057B | | EV0098B |
| EV0017 | EV0017A | EV0058 | EV0058A | EV0099 | EV0067A |
| | EV0017B | | EV0058B | | EV0099B |
| EV0018 | EV0018A | EV0059 | EV0059A | EV0100 | EV0068A |
| | EV0018B | | EV0059B | | EV0100B |
| EV0019 | EV0019A | EV0060 | EV0060A | EV0101 | EV0101A |
| | EV0019B | | EV0060B | | EV0101B |
| EV0020 | EV0020A | EV0061 | EV0061A | EV0102 | EV0102A |
| | EV0020B | | EV0061B | | EV0102B |
| EV0021 | EV0021A | EV0062 | EV0062A | EV0103 | EV0103A |
| | EV0021B | | EV0062B | | EV0103B |
| EV0022 | EV0022A | EV0063 | EV0063A | EV0104 | EV0104A |
| | EV0022B | | EV0063B | | EV0104B |
| EV0023 | EV0023A | EV0064 | EV0064A | EV0105 | EV0105A |
| | EV0023B | | EV0064B | | EV0105B |
| EV0024 | EV0024A | EV0065 | EV0065A | EV0106 | EV0106A |
| | EV0024B | | EV0065B | | EV0106B |
| EV0025 | EV0025A | EV0066 | EV0066A | EV0107 | EV0107A |
| | EV0025B | | EV0066B | | EV0107B |
| EV0026 | EV0026A | EV0067 | EV0067A | EV0108 | EV0108A |
| | EV0026B | | EV0067B | | EV0108B |
| EV0027 | EV0027A | EV0068 | EV0068A | EV0109 | EV0109A |
| | EV0027B | | EV0068B | | EV0109B |
| EV0028 | EV0028A | EV0069 | EV0037A | EV0110 | EV0110A |
| | EV0028B | | EV0069B | | EV0110B |
| EV0029 | EV0029A | EV0070 | EV0038A | EV0111 | EV0111A |
| | EV0029B | | EV0070B | | EV0111 B |
| EV0030 | EV0030A | EV0071 | EV0039A | EV0201 | EV0201A |
| | EV0030B | | EV0071B | | EV0201B |
| EV0031 | EV0031A | EV0072 | EV0040A | EV0202 | EV0201A |
| | EV0031B | | EV0072B | | EV0202B |
| EV0032 | EV0032A | EV0073 | EV0041A | EV0203 | EV0203A |
| | EV0032B | | EV0073B | | EV0201B |
| EV0033 | EV0033A | EV0074 | EV0042A | EV0204 | EV0203A |
| | EV0033B | | EV0074B | | EV0202B |
| EV0034 | EV0034A | EV0075 | EV0043A | EV0205 | EV0110A |
| | EV0034B | | EV0075B | | EV0205B |
| EV0035 | EV0035A | EV0076 | EV0044A | EV0206 | EV0110A |
| | EV0035B | | EV0076B | | EV0206B |
| EV0036 | EV0036A | EV0077 | EV0045A | EV0207 | EV0207A |
| | EV0036B | | EV0077B | | EV0205B |
| EV0037 | EV0037A | EV0078 | EV0046A | EV0208 | EV0207A |
| | EV0037B | | EV0078B | | EV0206B |
| EV0038 | EV0038A | EV0079 | EV0047A | EV0209 | EV0209A |
| | EV0038B | | EV0079B | | EV0205B |
| EV0039 | EV0039A | EV0080 | EV0048A | EV0312 | EV0312A |
| | EV0039B | | EV0080B | | EV0112B |
| EV0040 | EV0040A | EV0081 | EV0049A | EV0313 | EV0313A |
| | EV0040B | | EV0081B | | EV0313B |
| EV0041 | EV0041A | EV0082 | EV0050A | | |
| | EV0041B | | EV0082B | | |

**Summary abbreviations table - Table 4**

| **Abbreviation** | **Meaning** |
|---|---|
| mA, mU, mC, mG | 2'-O-Methyl RNA nucleotides |
| 2'-OMe | 2'-O-Methyl modification |
| fA, fU, fC, fG | 2'deoxy-2'-F RNA nucleotides |
| 2'-F | 2'-fluoro modification |
| (ps) | phosphorothioate |
| (vp) | Vinyl-(E)-phosphonate |
| (vp)-mU | 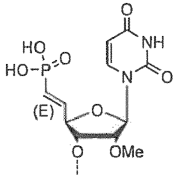 |
| (vp)-mU-phos | 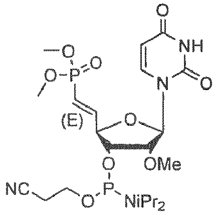 |
| ivA, ivC, ivU, ivG | inverted RNA (3'-3') nucleotides |
| ST23 | 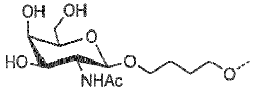 |
| ST23-phos | 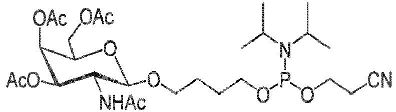 |
| ST43 (or C6XLT) | 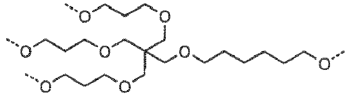 |
| ST43-phos (or C6XLT-phos) | 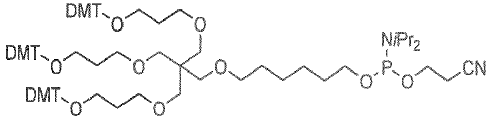 |
| Ser(GN) | 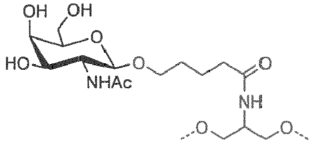 |

The abbreviations as shown in this abbreviation table may be used herein. The list of abbreviations may not be exhaustive and further abbreviations and their meaning may be found throughout this document.

**Summary sequence table - Table 5**

| **SEQ ID NO:** | **Name (A=1^{st} strand; B=2^{nd} strand)** | **Sequence 5'-3'** | **Unmodified sequence 5'-3' counterpart** |
|---|---|---|---|
| 1 | EV0001Aun | UUUCAUAGUAGGCUCGGAU | UUUCAUAGUAGGCUCGGAU |
| 2 | EV0001Bun | AUCCGAGCCUACUAUGAAA | AUCCGAGCCUACUAUGAAA |
| 3 | EV0002Aun | UUUCUCUGUAGGCUCCACU | UUUCUCUGUAGGCUCCACU |
| 4 | EV0002Bun | AGUGGAGCCUACAGAGAAA | AGUGGAGCCUACAGAGAAA |
| 5 | EV0003Aun | UUAUAGAUGUAGUAGAAUU | UUAUAGAUGUAGUAGAAUU |
| 6 | EV0003Bun | AAUUCUACUACAUCUAUAA | AAUUCUACUACAUCUAUAA |
| 7 | EV0004Aun | AUGACAAAGGCAGUUCCCU | AUGACAAAGGCAGUUCCCU |
| 8 | EV0004Bun | AGGGAACUGCCUUUGUCAU | AGGGAACUGCCUUUGUCAU |
| 9 | EV0005Aun | AUCUGGUAGGGAGAGGUCA | AUCUGGUAGGGAGAGGUCA |
| 10 | EV0005Bun | UGACCUCUCCCUACCAGAU | UGACCUCUCCCUACCAGAU |
| 11 | EV0006Aun | UGUGUGUUGAUGCUGAGUU | UGUGUGUUGAUGCUGAGUU |
| 12 | EV0006Bun | AACUCAGCAUCAACACACA | AACUCAGCAUCAACACACA |
| 13 | EV0007Aun | UAAUUGUUGGAGUUGCCCA | UAAUUGUUGGAGUUGCCCA |
| 14 | EV0007Bun | UGGGCAACUCCAACAAUUA | UGGGCAACUCCAACAAUUA |
| 15 | EV0008Aun | AGGAAGUUGACGUUGAGGG | AGGAAGUUGACGUUGAGGG |
| 16 | EV0008Bun | CCCUCAACGUCAACUUCCU | CCCUCAACGUCAACUUCCU |
| 17 | EV0009Aun | AUGAAGUCGGUGGUGAUGG | AUGAAGUCGGUGGUGAUGG |
| 18 | EV0009Bun | CCAUCACCACCGACUUCAU | CCAUCACCACCGACUUCAU |
| 19 | EV0010Aun | UUUACCACCAGCGAGCCCA | UUUACCACCAGCGAGCCCA |
| 20 | EV0010Bun | UGGGCUCGCUGGUGGUAAA | UGGGCUCGCUGGUGGUAAA |
| 21 | EV0011Aun | UCUAUCUUCAGGGUCAUCU | UCUAUCUUCAGGGUCAUCU |
| 22 | EV0011Bun | AGAUGACCCUGAAGAUAGA | AGAUGACCCUGAAGAUAGA |
| 23 | EV0012Aun | AUGUAGUUGCAGCAGUCCA | AUGUAGUUGCAGCAGUCCA |
| 24 | EV0012Bun | UGGACUGCUGCAACUACAU | UGGACUGCUGCAACUACAU |
| 25 | EV0013Aun | AAUAUAUUCAUGAGCUUCG | AAUAUAUUCAUGAGCUUCG |
| 26 | EV0013Bun | CGAAGCUCAUGAAUAUAUU | CGAAGCUCAUGAAUAUAUU |
| 27 | EV0014Aun | AAAUAUAUUCAUGAGCUUC | AAAUAUAUUCAUGAGCUUC |
| 28 | EV0014Bun | GAAGCUCAUGAAUAUAUUU | GAAGCUCAUGAAUAUAUUU |
| 29 | EV0015Aun | UCCUGCAUUACUGUGACCU | UCCUGCAUUACUGUGACCU |
| 30 | EV0015Bun | AGGUCACAGUAAUGCAGGA | AGGUCACAGUAAUGCAGGA |
| 31 | EV0016Aun | CAACAGAGUAGGGUAGCCG | CAACAGAGUAGGGUAGCCG |
| 32 | EV0016Bun | CGGCUACCCUACUCUGUUG | CGGCUACCCUACUCUGUUG |
| 33 | EV0017Aun | UCGAACAACAGAGUAGGGU | UCGAACAACAGAGUAGGGU |
| 34 | EV0017Bun | ACCCUACUCUGUUGUUCGA | ACCCUACUCUGUUGUUCGA |
| 35 | EV0018Aun | UUUCGAACAACAGAGUAGG | UUUCGAACAACAGAGUAGG |
| 36 | EV0018Bun | CCUACUCUGUUGUUCGAAA | CCUACUCUGUUGUUCGAAA |
| 37 | EV0019Aun | GUUUCAUCCAGGUAAUGCA | GUUUCAUCCAGGUAAUGCA |
| 38 | EV0019Bun | UGCAUUACCUGGAUGAAAC | UGCAUUACCUGGAUGAAAC |
| 39 | EV0020Aun | UUAUCUUUGGCUGUGGUCA | UUAUCUUUGGCUGUGGUCA |
| 40 | EV0020Bun | UGACCACAGCCAAAGAUAA | UGACCACAGCCAAAGAUAA |
| 41 | EV0021Aun | UGUUCAUUGAGCCAACGCA | UGUUCAUUGAGCCAACGCA |
| 42 | EV0021Bun | UGCGUUGGCUCAAUGAACA | UGCGUUGGCUCAAUGAACA |
| 43 | EV0023Aun | UUGGUAUUGAGCCAAGGCU | UUGGUAUUGAGCCAAGGCU |
| 44 | EV0023Bun | AGCCUUGGCUCAAUACCAA | AGCCUUGGCUCAAUACCAA |
| 45 | EV0024Aun | UUUAUUACAGGUGAGUUGA | UUUAUUACAGGUGAGUUGA |
| 46 | EV0024Bun | UCAACUCACCUGUAAUAAA | UCAACUCACCUGUAAUAAA |
| 47 | EV0025Aun | UUUCUGUUUCCGGUGCUGG | UUUCUGUUUCCGGUGCUGG |
| 48 | EV0025Bun | CCAGCACCGGAAACAGAAA | CCAGCACCGGAAACAGAAA |
| 49 | EV0026Aun | UCAAGGAUCAUAGUGUUCU | UCAAGGAUCAUAGUGUUCU |
| 50 | EV0026Bun | AGAACACUAUGAUCCUUGA | AGAACACUAUGAUCCUUGA |
| 51 | EV0027Aun | AUCUCAAGGAUCAUAGUGU | AUCUCAAGGAUCAUAGUGU |
| 52 | EV0027Bun | ACACUAUGAUCCUUGAGAU | ACACUAUGAUCCUUGAGAU |
| 53 | EV0028Aun | UCAUACUUGGAGAUGUAUC | UCAUACUUGGAGAUGUAUC |
| 54 | EV0028Bun | GAUACAUCUCCAAGUAUGA | GAUACAUCUCCAAGUAUGA |
| 55 | EV0029Aun | UAUCGGAGAAGGCUUUGUC | UAUCGGAGAAGGCUUUGUC |
| 56 | EV0029Bun | GACAAAGCCUUCUCCGAUA | GACAAAGCCUUCUCCGAUA |
| 57 | EV0030Aun | AUGAUGAGGGUGUUCCUAU | AUGAUGAGGGUGUUCCUAU |
| 58 | EV0030Bun | AUAGGAACACCCUCAUCAU | AUAGGAACACCCUCAUCAU |
| 59 | EV0031Aun | UAUUGGUGAACUUUGAAAG | UAUUGGUGAACUUUGAAAG |
| 60 | EV0031Bun | CUUUCAAAGUUCACCAAUA | CUUUCAAAGUUCACCAAUA |
| 61 | EV0032Aun | AGCUUGUUCAGCUUUCCAU | AGCUUGUUCAGCUUUCCAU |
| 62 | EV0032Bun | AUGGAAAGCUGAACAAGCU | AUGGAAAGCUGAACAAGCU |
| 63 | EV0033Aun | UUUGUAUGAAGCAAUUCUC | UUUGUAUGAAGCAAUUCUC |
| 64 | EV0033Bun | GAGAAUUGCUUCAUACAAA | GAGAAUUGCUUCAUACAAA |
| 65 | EV0034Aun | GUCUUGUACACAUAGUCCA | GUCUUGUACACAUAGUCCA |
| 66 | EV0034Bun | UGGACUAUGUGUACAAGAC | UGGACUAUGUGUACAAGAC |
| 67 | EV0035Aun | UGCUCAAUGGCCAUGAUGU | UGCUCAAUGGCCAUGAUGU |
| 68 | EV0035Bun | ACAUCAUGGCCAUUGAGCA | ACAUCAUGGCCAUUGAGCA |
| 69 | EV0036Aun | UUGGCAUUCGUCCUCCUCG | UUGGCAUUCGUCCUCCUCG |
| 70 | EV0036Bun | CGAGGAGGACGAAUGCCAA | CGAGGAGGACGAAUGCCAA |
| 71 | EV0037Aun | GUCUGGAUGAAGAGGUACC | GUCUGGAUGAAGAGGUACC |
| 72 | EV0037Bun | GGUACCUCUUCAUCCAGAC | GGUACCUCUUCAUCCAGAC |
| 73 | EV0038Aun | UGUCUGGAUGAAGAGGUAC | UGUCUGGAUGAAGAGGUAC |
| 74 | EV0038Bun | GUACCUCUUCAUCCAGACA | GUACCUCUUCAUCCAGACA |
| 75 | EV0039Aun | UCUGUCUGGAUGAAGAGGU | UCUGUCUGGAUGAAGAGGU |
| 76 | EV0039Bun | ACCUCUUCAUCCAGACAGA | ACCUCUUCAUCCAGACAGA |
| 77 | EV0040Aun | CUUGUCUGUCUGGAUGAAG | CUUGUCUGUCUGGAUGAAG |
| 78 | EV0040Bun | CUUCAUCCAGACAGACAAG | CUUCAUCCAGACAGACAAG |
| 79 | EV0041Aun | AUGGUCUUGUCUGUCUGGA | AUGGUCUUGUCUGUCUGGA |
| 80 | EV0041Bun | UCCAGACAGACAAGACCAU | UCCAGACAGACAAGACCAU |
| 81 | EV0042Aun | AGAUGGUCUUGUCUGUCUG | AGAUGGUCUUGUCUGUCUG |
| 82 | EV0042Bun | CAGACAGACAAGACCAUCU | CAGACAGACAAGACCAUCU |
| 83 | EV0043Aun | GUAGAUGGUCUUGUCUGUC | GUAGAUGGUCUUGUCUGUC |
| 84 | EV0043Bun | GACAGACAAGACCAUCUAC | GACAGACAAGACCAUCUAC |
| 85 | EV0044Aun | GUGUAGAUGGUCUUGUCUG | GUGUAGAUGGUCUUGUCUG |
| 86 | EV0044Bun | CAGACAAGACCAUCUACAC | CAGACAAGACCAUCUACAC |
| 87 | EV0045Aun | GGGGUGUAGAUGGUCUUGU | GGGGUGUAGAUGGUCUUGU |
| 88 | EV0045Bun | ACAAGACCAUCUACACCCC | ACAAGACCAUCUACACCCC |
| 89 | EV0046Aun | UAGGCUCGGAUCUUCCACU | UAGGCUCGGAUCUUCCACU |
| 90 | EV0046Bun | AGUGGAAGAUCCGAGCCUA | AGUGGAAGAUCCGAGCCUA |
| 91 | EV0047Aun | CUCGAAACUGGGCAGCACG | CUCGAAACUGGGCAGCACG |
| 92 | EV0047Bun | CGUGCUGCCCAGUUUCGAG | CGUGCUGCCCAGUUUCGAG |
| 93 | EV0048Aun | UUGGUGAAGUGGAUCUGGU | UUGGUGAAGUGGAUCUGGU |
| 94 | EV0048Bun | ACCAGAUCCACUUCACCAA | ACCAGAUCCACUUCACCAA |
| 95 | EV0049Aun | UCUUGGUGAAGUGGAUCUG | UCUUGGUGAAGUGGAUCUG |
| 96 | EV0049Bun | CAGAUCCACUUCACCAAGA | CAGAUCCACUUCACCAAGA |
| 97 | EV0050Aun | GUCUUGGUGAAGUGGAUCU | GUCUUGGUGAAGUGGAUCU |
| 98 | EV0050Bun | AGAUCCACUUCACCAAGAC | AGAUCCACUUCACCAAGAC |
| 99 | EV0051Aun | GGUGUCUUGGUGAAGUGGA | GGUGUCUUGGUGAAGUGGA |
| 100 | EV0051Bun | UCCACUUCACCAAGACACC | UCCACUUCACCAAGACACC |
| 101 | EV0052Aun | AACACCAUGAGGUCAAAGG | AACACCAUGAGGUCAAAGG |
| 102 | EV0052Bun | CCUUUGACCUCAUGGUGUU | CCUUUGACCUCAUGGUGUU |
| 103 | EV0053Aun | GAACACCAUGAGGUCAAAG | GAACACCAUGAGGUCAAAG |
| 104 | EV0053Bun | CUUUGACCUCAUGGUGUUC | CUUUGACCUCAUGGUGUUC |
| 105 | EV0054Aun | GUCACGAACACCAUGAGGU | GUCACGAACACCAUGAGGU |
| 106 | EV0054Bun | ACCUCAUGGUGUUCGUGAC | ACCUCAUGGUGUUCGUGAC |
| 107 | EV0055Aun | ACACAGAUCCCUUUCUUGU | ACACAGAUCCCUUUCUUGU |
| 108 | EV0055Bun | ACAAGAAAGGGAUCUGUGU | ACAAGAAAGGGAUCUGUGU |
| 109 | EV0058Aun | CCUUGCAGGAGAAUUCUGG | CCUUGCAGGAGAAUUCUGG |
| 110 | EV0058Bun | CCAGAAUUCUCCUGCAAGG | CCAGAAUUCUCCUGCAAGG |
| 111 | EV0059Aun | AGAGAGAAGACCUUGACCA | AGAGAGAAGACCUUGACCA |
| 112 | EV0059Bun | UGGUCAAGGUCUUCUCUCU | UGGUCAAGGUCUUCUCUCU |
| 113 | EV0060Aun | GAGUCGAUGGCGAUGAGGU | GAGUCGAUGGCGAUGAGGU |
| 114 | EV0060Bun | ACCUCAUCGCCAUCGACUC | ACCUCAUCGCCAUCGACUC |
| 115 | EV0061Aun | GCUUGGAACACCAUGAAGG | GCUUGGAACACCAUGAAGG |
| 116 | EV0061Bun | CCUUCAUGGUGUUCCAAGC | CCUUCAUGGUGUUCCAAGC |
| 117 | EV0062Aun | UCAUUUUCCUUGGUCUCUU | UCAUUUUCCUUGGUCUCUU |
| 118 | EV0062Bun | AAGAGACCAAGGAAAAUGA | AAGAGACCAAGGAAAAUGA |
| 119 | EV0063Aun | UGUGUCUGGAGCAAAGCCA | UGUGUCUGGAGCAAAGCCA |
| 120 | EV0063Bun | UGGCUUUGCUCCAGACACA | UGGCUUUGCUCCAGACACA |
| 121 | EV0064Aun | AGGUAGAUGAUGAGGGUGU | AGGUAGAUGAUGAGGGUGU |
| 122 | EV0064Bun | ACACCCUCAUCAUCUACCU | ACACCCUCAUCAUCUACCU |
| 123 | EV0065Aun | UUGUAAUAGGCGUAGACCU | UUGUAAUAGGCGUAGACCU |
| 124 | EV0065Bun | AGGUCUACGCCUAUUACAA | AGGUCUACGCCUAUUACAA |
| 125 | EV0066Aun | GUUGUAAUAGGCGUAGACC | GUUGUAAUAGGCGUAGACC |
| 126 | EV0066Bun | GGUCUACGCCUAUUACAAC | GGUCUACGCCUAUUACAAC |
| 127 | EV0067Aun | GGGUCUUGUACACAUAGUC | GGGUCUUGUACACAUAGUC |
| 128 | EV0067Bun | GACUAUGUGUACAAGACCC | GACUAUGUGUACAAGACCC |
| 129 | EV0068Aun | CACUUGAUGGGGCUGAUGA | CACUUGAUGGGGCUGAUGA |
| 130 | EV0068Bun | UCAUCAGCCCCAUCAAGUG | UCAUCAGCCCCAUCAAGUG |
| 131 | EV0312Aun | AUUGUAAUAGGCGUAGACC | AUUGUAAUAGGCGUAGACC |
| 132 | EV0112Bun | GGUCUACGCCUAUUACAAU | GGUCUACGCCUAUUACAAU |
| 133 | EV0313Aun | AUGUAGAUGGUCUUGUCUG | AUGUAGAUGGUCUUGUCUG |
| 134 | EV0313Bun | CAGACAAGACCAUCUACAU | CAGACAAGACCAUCUACAU |
| 135 | EV0069Bun | GGUACCUCUUCAUCCAGAA | GGUACCUCUUCAUCCAGAA |
| 136 | EV0072Bun | CUUCAUCCAGACAGACAAA | CUUCAUCCAGACAGACAAA |
| 137 | EV0073Bun | UCCAGACAGACAAGACCAA | UCCAGACAGACAAGACCAA |
| 138 | EV0074Bun | CAGACAGACAAGACCAUCA | CAGACAGACAAGACCAUCA |
| 139 | EV0075Bun | GACAGACAAGACCAUCUAA | GACAGACAAGACCAUCUAA |
| 140 | EV0076Bun | CAGACAAGACCAUCUACAA | CAGACAAGACCAUCUACAA |
| 141 | EV0077Bun | ACAAGACCAUCUACACCCA | ACAAGACCAUCUACACCCA |
| 142 | EV0079Bun | CGUGCUGCCCAGUUUCGAA | CGUGCUGCCCAGUUUCGAA |
| 143 | EV0082Bun | AGAUCCACUUCACCAAGAA | AGAUCCACUUCACCAAGAA |
| 144 | EV0083Bun | UCCACUUCACCAAGACACA | UCCACUUCACCAAGACACA |
| 145 | EV0084Bun | CCUUUGACCUCAUGGUGUA | CCUUUGACCUCAUGGUGUA |
| 146 | EV0085Bun | CUUUGACCUCAUGGUGUUA | CUUUGACCUCAUGGUGUUA |
| 147 | EV0086Bun | ACCUCAUGGUGUUCGUGAA | ACCUCAUGGUGUUCGUGAA |
| 148 | EV0087Bun | ACAAGAAAGGGAUCUGUGA | ACAAGAAAGGGAUCUGUGA |
| 149 | EV0088Bun | AGGGAUCUGUGUGGCAGAA | AGGGAUCUGUGUGGCAGAA |
| 150 | EV0089Bun | AAUGCAGGACUUCUUCAUA | AAUGCAGGACUUCUUCAUA |
| 151 | EV0090Bun | CCAGAAUUCUCCUGCAAGA | CCAGAAUUCUCCUGCAAGA |
| 152 | EV0091Bun | UGGUCAAGGUCUUCUCUCA | UGGUCAAGGUCUUCUCUCA |
| 153 | EV0092Bun | ACCUCAUCGCCAUCGACUA | ACCUCAUCGCCAUCGACUA |
| 154 | EV0093Bun | CCUUCAUGGUGUUCCAAGA | CCUUCAUGGUGUUCCAAGA |
| 155 | EV0096Bun | ACACCCUCAUCAUCUACCA | ACACCCUCAUCAUCUACCA |
| 156 | EV0098Bun | GGUCUACGCCUAUUACAAA | GGUCUACGCCUAUUACAAA |
| 157 | EV0099Bun | GACUAUGUGUACAAGACCA | GACUAUGUGUACAAGACCA |
| 158 | EV0100Bun | UCAUCAGCCCCAUCAAGUA | UCAUCAGCCCCAUCAAGUA |
| 159 | EV0001 A | mU fU mU fC mA fU mA fG mU fA mG fG mC fU mC fG mG fA mU | UUUCAUAGUAGGCUCGGAU |
| 160 | EV0001B | fA mU fC mC fG mA fG mC fC mU fA mC fU mA fU mG fA mA fA | AUCCGAGCCUACUAUGAAA |
| 161 | EV0002A | mU fU mU fC mU fC mU fG mU fA mG fG mC fU mC fC mA fC mU | UUUCUCUGUAGGCUCCACU |
| 162 | EV0002B | fA mG fU mG fG mA fG mC fC mU fA mC fA mG fA mG fA mA fA | AGUGGAGCCUACAGAGAAA |
| 163 | EV0003A | mU fU mA fU mA fG mA fU mG fU mA fG mU fA mG fA mA fU mU | UUAUAGAUGUAGUAGAAUU |
| 164 | EV0003B | fA mA fU mU fC mU fA mC fU mA fC mA fU mC fU mA fU mA fA | AAUUCUACUACAUCUAUAA |
| 165 | EV0004A | mA fU mG fA mC fA mA fA mG fG mC fA mG fU mU fC mC fC mU | AUGACAAAGGCAGUUCCCU |
| 166 | EV0004B | fA mG fG mG fA mA fC mU fG mC fC mU fU mU fG mU fC mA fU | AGGGAACUGCCUUUGUCAU |
| 167 | EV0005A | mA fU mC fU mG fG mU fA mG fG mG fA mG fA mG fG mU fC mA | AUCUGGUAGGGAGAGGUCA |
| 168 | EV0005B | fU mG fA mC fC mU fC mU fC mC fC mU fA mC fC mA fG mA fU | UGACCUCUCCCUACCAGAU |
| 169 | EV0006A | mU fG mU fG mU fG mU fU mG fA mU fG mC fU mG fA mG fU mU | UGUGUGUUGAUGCUGAGUU |
| 170 | EV0006B | fA mA fC mU fC mA fG mC fA mU fC mA fA mC fA mC fA mC fA | AACUCAGCAUCAACACACA |
| 171 | EV0007A | mU fA mA fU mU fG mU fU mG fG mA fG mU fU mG fC mC fC mA | UAAUUGUUGGAGUUGCCCA |
| 172 | EV0007B | fU mG fG mG fC mA fA mC fU mC fC mA fA mC fA mA fU mU fA | UGGGCAACUCCAACAAUUA |
| 173 | EV0008A | mA fG mG fA mA fG mU fU mG fA mC fG mU fU mG fA mG fG mG | AGGAAGUUGACGUUGAGGG |
| 174 | EV0008B | fC mC fC mU fC mA fA mC fG mU fC mA fA mC fU mU fC mC fU | CCCUCAACGUCAACUUCCU |
| 175 | EV0009A | mA fU mG fA mA fG mU fC mG fG mU fG mG fU mG fA mU fG mG | AUGAAGUCGGUGGUGAUGG |
| 176 | EV0009B | fC mC fA mU fC mA fC mC fA mC fC mG fA mC fU mU fC mA fU | CCAUCACCACCGACUUCAU |
| 177 | EV0010A | mU fU mU fA mC fC mA fC mC fA mG fC mG fA mG fC mC fC mA | UUUACCACCAGCGAGCCCA |
| 178 | EV0010B | fU mG fG mG fC mU fC mG fC mU fG mG fU mG fG mU fA mA fA | UGGGCUCGCUGGUGGUAAA |
| 179 | EV0011A | mU fC mU fA mU fC mU fU mC fA mG fG mG fU mC fA mU fC mU | UCUAUCUUCAGGGUCAUCU |
| 180 | EV0011B | fA mG fA mU fG mA fC mC fC mU fG mA fA mG fA mU fA mG fA | AGAUGACCCUGAAGAUAGA |
| 181 | EV0012A | mA fU mG fU mA fG mU fU mG fC mA fG mC fA mG fU mC fC mA | AUGUAGUUGCAGCAGUCCA |
| 182 | EV0012B | fU mG fG mA fC mU fG mC fU mG fC mA fA mC fU mA fC mA fU | UGGACUGCUGCAACUACAU |
| 183 | EV0013A | mA fA mU fA mU fA mU fU mC fA mU fG mA fG mC fU mU fC mG | AAUAUAUUCAUGAGCUUCG |
| 184 | EV0013B | fC mG fA mA fG mC fU mC fA mU fG mA fA mU fA mU fA mU fU | CGAAGCUCAUGAAUAUAUU |
| 185 | EV0014A | mA fA mA fU mA fU mA fU mU fC mA fU mG fA mG fC mU fU mC | AAAUAUAUUCAUGAGCUUC |
| 186 | EV0014B | fG mA fA mG fC mU fC mA fU mG fA mA fU mA fU mA fU mU fU | GAAGCUCAUGAAUAUAUUU |
| 187 | EV0015A | mU fC mC fU mG fC mA fU mU fA mC fU mG fU mG fA mC fC mU | UCCUGCAUUACUGUGACCU |
| 188 | EV0015B | fA mG fG mU fC mA fC mA fG mU fA mA fU mG fC mA fG mG fA | AGGUCACAGUAAUGCAGGA |
| 189 | EV0016A | mC fA mA fC mA fG mA fG mU fA mG fG mG fU mA fG mC fC mG | CAACAGAGUAGGGUAGCCG |
| 190 | EV0016B | fC mG fG mC fU mA fC mC fC mU fA mC fU mC fU mG fU mU fG | CGGCUACCCUACUCUGUUG |
| 191 | EV0017A | mU fC mG fA mA fC mA fA mC fA mG fA mG fU mA fG mG fG mU | UCGAACAACAGAGUAGGGU |
| 192 | EV0017B | fA mC fC mC fU mA fC mU fC mU fG mU fU mG fU mU fC mG fA | ACCCUACUCUGUUGUUCGA |
| 193 | EV0018A | mU fU mU fC mG fA mA fC mA fA mC fA mG fA mG fU mA fG mG | UUUCGAACAACAGAGUAGG |
| 194 | EV0018B | fC mC fU mA fC mU fC mU fG mU fU mG fU mU fC mG fA mA fA | CCUACUCUGUUGUUCGAAA |
| 195 | EV0019A | mG fU mU fU mC fA mU fC mC fA mG fG mU fA mA fU mG fC mA | GUUUCAUCCAGGUAAUGCA |
| 196 | EV0019B | fU mG fC mA fU mU fA mC fC mU fG mG fA mU fG mA fA mA fC | UGCAUUACCUGGAUGAAAC |
| 197 | EV0020A | mU fU mA fU mC fU mU fU mG fG mC fU mG fU mG fG mU fC mA | UUAUCUUUGGCUGUGGUCA |
| 198 | EV0020B | fU mG fA mC fC mA fC mA fG mC fC mA fA mA fG mA fU mA fA | UGACCACAGCCAAAGAUAA |
| 199 | EV0021 A | mU fG mU fU mC fA mU fU mG fA mG fC mC fA mA fC mG fC mA | UGUUCAUUGAGCCAACGCA |
| 200 | EV0021B | fU mG fC mG fU mU fG mG fC mU fC mA fA mU fG mA fA mC fA | UGCGUUGGCUCAAUGAACA |
| 201 | EV0022A | mA fA mC fA mC fC mA fU mG fA mA fG mG fU mG fG mC fC mU | AACACCAUGAAGGUGGCCU |
| 202 | EV0022B | fA mG fG mC fC mA fC mC fU mU fC mA fU mG fG mU fG mU fU | AGGCCACCUUCAUGGUGUU |
| 203 | EV0023A | mU fU mG fG mU fA mU fU mG fA mG fC mC fA mA fG mG fC mU | UUGGUAUUGAGCCAAGGCU |
| 204 | EV0023B | fA mG fC mC fU mU fG mG fC mU fC mA fA mU fA mC fC mA fA | AGCCUUGGCUCAAUACCAA |
| 205 | EV0024A | mU fU mU fA mU fU mA fC mA fG mG fU mG fA mG fU mU fG mA | UUUAUUACAGGUGAGUUGA |
| 206 | EV0024B | fU mC fA mA fC mU fC mA fC mC fU mG fU mA fA mU fA mA fA | UCAACUCACCUGUAAUAAA |
| 207 | EV0025A | mU fU mU fC mU fG mU fU mU fC mC fG mG fU mG fC mU fG mG | UUUCUGUUUCCGGUGCUGG |
| 208 | EV0025B | fC mC fA mG fC mA fC mC fG mG fA mA fA mC fA mG fA mA fA | CCAGCACCGGAAACAGAAA |
| 209 | EV0026A | mU fC mA fA mG fG mA fU mC fA mU fA mG fU mG fU mU fC mU | UCAAGGAUCAUAGUGUUCU |
| 210 | EV0026B | fA mG fA mA fC mA fC mU fA mU fG mA fU mC fC mU fU mG fA | AGAACACUAUGAUCCUUGA |
| 211 | EV0027A | mA fU mC fU mC fA mA fG mG fA mU fC mA fU mA fG mU fG mU | AUCUCAAGGAUCAUAGUGU |
| 212 | EV0027B | fA mC fA mC fU mA fU mG fA mU fC mC fU mU fG mA fG mA fU | ACACUAUGAUCCUUGAGAU |
| 213 | EV0028A | mU fC mA fU mA fC mU fU mG fG mA fG mA fU mG fU mA fU mC | UCAUACUUGGAGAUGUAUC |
| 214 | EV0028B | fG mA fU mA fC mA fU mC fU mC fC mA fA mG fU mA fU mG fA | GAUACAUCUCCAAGUAUGA |
| 215 | EV0029A | mU fA mU fC mG fG mA fG mA fA mG fG mC fU mU fU mG fU mC | UAUCGGAGAAGGCUUUGUC |
| 216 | EV0029B | fG mA fC mA fA mA fG mC fC mU fU mC fU mC fC mG fA mU fA | GACAAAGCCUUCUCCGAUA |
| 217 | EV0030A | mA fU mG fA mU fG mA fG mG fG mU fG mU fU mC fC mU fA mU | AUGAUGAGGGUGUUCCUAU |
| 218 | EV0030B | fA mU fA mG fG mA fA mC fA mC fC mC fU mC fA mU fC mA fU | AUAGGAACACCCUCAUCAU |
| 219 | EV0031A | mU fA mU fU mG fG mU fG mA fA mC fU mU fU mG fA mA fA mG | UAUUGGUGAACUUUGAAAG |
| 220 | EV0031B | fC mU fU mU fC mA fA mA fG mU fU mC fA mC fC mA fA mU fA | CUUUCAAAGUUCACCAAUA |
| 221 | EV0032A | mA fG mC fU mU fG mU fU mC fA mG fC mU fU mU fC mC fA mU | AGCUUGUUCAGCUUUCCAU |
| 222 | EV0032B | fA mU fG mG fA mA fA mG fC mU fG mA fA mC fA mA fG mC fU | AUGGAAAGCUGAACAAGCU |
| 223 | EV0033A | mU fU mU fG mU fA mU fG mA fA mG fC mA fA mU fU mC fU mC | UUUGUAUGAAGCAAUUCUC |
| 224 | EV0033B | fG mA fG mA fA mU fU mG fC mU fU mC fA mU fA mC fA mA fA | GAGAAUUGCUUCAUACAAA |
| 225 | EV0034A | mG fU mC fU mU fG mU fA mC fA mC fA mU fA mG fU mC fC mA | GUCUUGUACACAUAGUCCA |
| 226 | EV0034B | fU mG fG mA fC mU fA mU fG mU fG mU fA mC fA mA fG mA fC | UGGACUAUGUGUACAAGAC |
| 227 | EV0035A | mU fG mC fU mC fA mA fU mG fG mC fC mA fU mG fA mU fG mU | UGCUCAAUGGCCAUGAUGU |
| 228 | EV0035B | fA mC fA mU fC mA fU mG fG mC fC mA fU mU fG mA fG mC fA | ACAUCAUGGCCAUUGAGCA |
| 229 | EV0036A | mU fU mG fG mC fA mU fU mC fG mU fC mC fU mC fC mU fC mG | UUGGCAUUCGUCCUCCUCG |
| 230 | EV0036B | fC mG fA mG fG mA fG mG fA mC fG mA fA mU fG mC fC mA fA | CGAGGAGGACGAAUGCCAA |
| 231 | EV0037A | mG fU mC fU mG fG mA fU mG fA mA fG mA fG mG fU mA fC mC | GUCUGGAUGAAGAGGUACC |
| 232 | EV0037B | fG mG fU mA fC mC fU mC fU mU fC mA fU mC fC mA fG mA fC | GGUACCUCUUCAUCCAGAC |
| 233 | EV0038A | mU fG mU fC mU fG mG fA mU fG mA fA mG fA mG fG mU fA mC | UGUCUGGAUGAAGAGGUAC |
| 234 | EV0038B | fG mU fA mC fC mU fC mU fU mC fA mU fC mC fA mG fA mC fA | GUACCUCUUCAUCCAGACA |
| 235 | EV0039A | mU fC mU fG mU fC mU fG mG fA mU fG mA fA mG fA mG fG mU | UCUGUCUGGAUGAAGAGGU |
| 236 | EV0039B | fA mC fC mU fC mU fU mC fA mU fC mC fA mG fA mC fA mG fA | ACCUCUUCAUCCAGACAGA |
| 237 | EV0040A | mC fU mU fG mU fC mU fG mU fC mU fG mG fA mU fG mA fA mG | CUUGUCUGUCUGGAUGAAG |
| 238 | EV0040B | fC mU fU mC fA mU fC mC fA mG fA mC fA mG fA mC fA mA fG | CUUCAUCCAGACAGACAAG |
| 239 | EV0041 A | mA fU mG fG mU fC mU fU mG fU mC fU mG fU mC fU mG fG mA | AUGGUCUUGUCUGUCUGGA |
| 240 | EV0041B | fU mC fC mA fG mA fC mA fG mA fC mA fA mG fA mC fC mA fU | UCCAGACAGACAAGACCAU |
| 241 | EV0042A | mA fG mA fU mG fG mU fC mU fU mG fU mC fU mG fU mC fU mG | AGAUGGUCUUGUCUGUCUG |
| 242 | EV0042B | fC mA fG mA fC mA fG mA fC mA fA mG fA mC fC mA fU mC fU | CAGACAGACAAGACCAUCU |
| 243 | EV0043A | mG fU mA fG mA fU mG fG mU fC mU fU mG fU mC fU mG fU mC | GUAGAUGGUCUUGUCUGUC |
| 244 | EV0043B | fG mA fC mA fG mA fC mA fA mG fA mC fC mA fU mC fU mA fC | GACAGACAAGACCAUCUAC |
| 245 | EV0044A | mG fU mG fU mA fG mA fU mG fG mU fC mU fU mG fU mC fU mG | GUGUAGAUGGUCUUGUCUG |
| 246 | EV0044B | fC mA fG mA fC mA fA mG fA mC fC mA fU mC fU mA fC mA fC | CAGACAAGACCAUCUACAC |
| 247 | EV0045A | mG fG mG fG mU fG mU fA mG fA mU fG mG fU mC fU mU fG mU | GGGGUGUAGAUGGUCUUGU |
| 248 | EV0045B | fA mC fA mA fG mA fC mC fA mU fC mU fA mC fA mC fC mC fC | ACAAGACCAUCUACACCCC |
| 249 | EV0046A | mU fA mG fG mC fU mC fG mG fA mU fC mU fU mC fC mA fC mU | UAGGCUCGGAUCUUCCACU |
| 250 | EV0046B | fA mG fU mG fG mA fA mG fA mU fC mC fG mA fG mC fC mU fA | AGUGGAAGAUCCGAGCCUA |
| 251 | EV0047A | mC fU mC fG mA fA mA fC mU fG mG fG mC fA mG fC mA fC mG | CUCGAAACUGGGCAGCACG |
| 252 | EV0047B | fC mG fU mG fC mU fG mC fC mC fA mG fU mU fU mC fG mA fG | CGUGCUGCCCAGUUUCGAG |
| 253 | EV0048A | mU fU mG fG mU fG mA fA mG fU mG fG mA fU mC fU mG fG mU | UUGGUGAAGUGGAUCUGGU |
| 254 | EV0048B | fA mC fC mA fG mA fU mC fC mA fC mU fU mC fA mC fC mA fA | ACCAGAUCCACUUCACCAA |
| 255 | EV0049A | mU fC mU fU mG fG mU fG mA fA mG fU mG fG mA fU mC fU mG | UCUUGGUGAAGUGGAUCUG |
| 256 | EV0049B | fC mA fG mA fU mC fC mA fC mU fU mC fA mC fC mA fA mG fA | CAGAUCCACUUCACCAAGA |
| 257 | EV0050A | mG fU mC fU mU fG mG fU mG fA mA fG mU fG mG fA mU fC mU | GUCUUGGUGAAGUGGAUCU |
| 258 | EV0050B | fA mG fA mU fC mC fA mC fU mU fC mA fC mC fA mA fG mA fC | AGAUCCACUUCACCAAGAC |
| 259 | EV0051 A | mG fG mU fG mU fC mU fU mG fG mU fG mA fA mG fU mG fG mA | GGUGUCUUGGUGAAGUGGA |
| 260 | EV0051B | fU mC fC mA fC mU fU mC fA mC fC mA fA mG fA mC fA mC fC | UCCACUUCACCAAGACACC |
| 261 | EV0052A | mA fA mC fA mC fC mA fU mG fA mG fG mU fC mA fA mA fG mG | AACACCAUGAGGUCAAAGG |
| 262 | EV0052B | fC mC fU mU fU mG fA mC fC mU fC mA fU mG fG mU fG mU fU | CCUUUGACCUCAUGGUGUU |
| 263 | EV0053A | mG fA mA fC mA fC mC fA mU fG mA fG mG fU mC fA mA fA mG | GAACACCAUGAGGUCAAAG |
| 264 | EV0053B | fC mU fU mU fG mA fC mC fU mC fA mU fG mG fU mG fU mU fC | CUUUGACCUCAUGGUGUUC |
| 265 | EV0054A | mG fU mC fA mC fG mA fA mC fA mC fC mA fU mG fA mG fG mU | GUCACGAACACCAUGAGGU |
| 266 | EV0054B | fA mC fC mU fC mA fU mG fG mU fG mU fU mC fG mU fG mA fC | ACCUCAUGGUGUUCGUGAC |
| 267 | EV0055A | mA fC mA fC mA fG mA fU mC fC mC fU mU fU mC fU mU fG mU | ACACAGAUCCCUUUCUUGU |
| 268 | EV0055B | fA mC fA mA fG mA fA mA fG mG fG mA fU mC fU mG fU mG fU | ACAAGAAAGGGAUCUGUGU |
| 269 | EV0056A | mG fU mC fU mG fC mC fA mC fA mC fA mG fA mU fC mC fC mU | GUCUGCCACACAGAUCCCU |
| 270 | EV0056B | fA mG fG mG fA mU fC mU fG mU fG mU fG mG fC mA fG mA fC | AGGGAUCUGUGUGGCAGAC |
| 271 | EV0057A | mG fA mU fG mA fA mG fA mA fG mU fC mC fU mG fC mA fU mU | GAUGAAGAAGUCCUGCAUU |
| 272 | EV0057B | fA mA fU mG fC mA fG mG fA mC fU mU fC mU fU mC fA mU fC | AAUGCAGGACUUCUUCAUC |
| 273 | EV0058A | mC fC mU fU mG fC mA fG mG fA mG fA mA fU mU fC mU fG mG | CCUUGCAGGAGAAUUCUGG |
| 274 | EV0058B | fC mC fA mG fA mA fU mU fC mU fC mC fU mG fC mA fA mG fG | CCAGAAUUCUCCUGCAAGG |
| 275 | EV0059A | mA fG mA fG mA fG mA fA mG fA mC fC mU fU mG fA mC fC mA | AGAGAGAAGACCUUGACCA |
| 276 | EV0059B | fU mG fG mU fC mA fA mG fG mU fC mU fU mC fU mC fU mC fU | UGGUCAAGGUCUUCUCUCU |
| 277 | EV0060A | mG fA mG fU mC fG mA fU mG fG mC fG mA fU mG fA mG fG mU | GAGUCGAUGGCGAUGAGGU |
| 278 | EV0060B | fA mC fC mU fC mA fU mC fG mC fC mA fU mC fG mA fC mU fC | ACCUCAUCGCCAUCGACUC |
| 279 | EV0061 A | mG fC mU fU mG fG mA fA mC fA mC fC mA fU mG fA mA fG mG | GCUUGGAACACCAUGAAGG |
| 280 | EV0061B | fC mC fU mU fC mA fU mG fG mU fG mU fU mC fC mA fA mG fC | CCUUCAUGGUGUUCCAAGC |
| 281 | EV0062A | mU fC mA fU mU fU mU fC mC fU mU fG mG fU mC fU mC fU mU | UCAUUUUCCUUGGUCUCUU |
| 282 | EV0062B | fA mA fG mA fG mA fC mC fA mA fG mG fA mA fA mA fU mG fA | AAGAGACCAAGGAAAAUGA |
| 283 | EV0063A | mU fG mU fG mU fC mU fG mG fA mG fC mA fA mA fG mC fC mA | UGUGUCUGGAGCAAAGCCA |
| 284 | EV0063B | fU mG fG mC fU mU fU mG fC mU fC mC fA mG fA mC fA mC fA | UGGCUUUGCUCCAGACACA |
| 285 | EV0064A | mA fG mG fU mA fG mA fU mG fA mU fG mA fG mG fG mU fG mU | AGGUAGAUGAUGAGGGUGU |
| 286 | EV0064B | fA mC fA mC fC mC fU mC fA mU fC mA fU mC fU mA fC mC fU | ACACCCUCAUCAUCUACCU |
| 287 | EV0065A | mU fU mG fU mA fA mU fA mG fG mC fG mU fA mG fA mC fC mU | UUGUAAUAGGCGUAGACCU |
| 288 | EV0065B | fA mG fG mU fC mU fA mC fG mC fC mU fA mU fU mA fC mA fA | AGGUCUACGCCUAUUACAA |
| 289 | EV0066A | mG fU mU fG mU fA mA fU mA fG mG fC mG fU mA fG mA fC mC | GUUGUAAUAGGCGUAGACC |
| 290 | EV0066B | fG mG fU mC fU mA fC mG fC mC fU mA fU mU fA mC fA mA fC | GGUCUACGCCUAUUACAAC |
| 291 | EV0067A | mG fG mG fU mC fU mU fG mU fA mC fA mC fA mU fA mG fU mC | GGGUCUUGUACACAUAGUC |
| 292 | EV0067B | fG mA fC mU fA mU fG mU fG mU fA mC fA mA fG mA fC mC fC | GACUAUGUGUACAAGACCC |
| 293 | EV0068A | mC fA mC fU mU fG mA fU mG fG mG fG mC fU mG fA mU fG mA | CACUUGAUGGGGCUGAUGA |
| 294 | EV0068B | fU mC fA mU fC mA fG mC fC mC fC mA fU mC fA mA fG mU fG | UCAUCAGCCCCAUCAAGUG |
| 295 | EV0069B | mG mG mU mA mC mC fU fC fU mU mC mA mU mC mC mA mG mA irA | GGUACCUCUUCAUCCAGAA |
| 296 | EV0070B | mG mU mA mC mC mU fC fU fU mC mA mU mC mC mA mG mA mC irA | GUACCUCUUCAUCCAGACA |
| 297 | EV0071B | mA mC mC mU mC mU fU fC fA mU mC mC mA mG mA mC mA mG irA | ACCUCUUCAUCCAGACAGA |
| 298 | EV0072B | mC mU mU mC mA mU fC fC fA mG mA mC mA mG mA mC mA mA irA | CUUCAUCCAGACAGACAAA |
| 299 | EV0073B | mU mC mC mA mG mA fC fA fG mA mC mA mA mG mA mC mC mA irA | UCCAGACAGACAAGACCAA |
| 300 | EV0074B | mC mA mG mA mC mA fG fA fC mA mA mG mA mC mC mA mU mC irA | CAGACAGACAAGACCAUCA |
| 301 | EV0075B | mG mA mC mA mG mA fC fA fA mG mA mC mC mA mU mC mU mA irA | GACAGACAAGACCAUCUAA |
| 302 | EV0076B | mC mA mG mA mC mA fA fG fA mC mC mA mU mC mU mA mC mA irA | CAGACAAGACCAUCUACAA |
| 303 | EV0077B | mA mC mA mA mG mA fC fC fA mU mC mU mA mC mA mC mC mC irA | ACAAGACCAUCUACACCCA |
| 304 | EV0078B | mA mG mU mG mG mA fA fG fA mU mC mC mG mA mG mC mC mU irA | AGUGGAAGAUCCGAGCCUA |
| 305 | EV0079B | mC mG mU mG mC mU fG fC fC mC mA mG mU mU mU mC mG mA irA | CGUGCUGCCCAGUUUCGAA |
| 306 | EV0080B | mA mC mC mA mG mA fU fC fC mA mC mU mU mC mA mC mC mA irA | ACCAGAUCCACUUCACCAA |
| 307 | EV0081B | mC mA mG mA mU mC fC fA fC mU mU mC mA mC mC mA mA mG irA | CAGAUCCACUUCACCAAGA |
| 308 | EV0082B | mA mG mA mU mC mC fA fC fU mU mC mA mC mC mA mA mG mA irA | AGAUCCACUUCACCAAGAA |
| 309 | EV0083B | mU mC mC mA mC mU fU fC fA mC mC mA mA mG mA mC mA mC irA | UCCACUUCACCAAGACACA |
| 310 | EV0084B | mC mC mU mU mU mG fA fC fC mU mC mA mU mG mG mU mG mU irA | CCUUUGACCUCAUGGUGUA |
| 311 | EV0085B | mC mU mU mU mG mA fC fC fU mC mA mU mG mG mU mG mU mU irA | CUUUGACCUCAUGGUGUUA |
| 312 | EV0086B | mA mC mC mU mC mA fU fG fG mU mG mU mU mC mG mU mG mA irA | ACCUCAUGGUGUUCGUGAA |
| 313 | EV0087B | mA mC mA mA mG mA fA fA fG mG mG mA mU mC mU mG mU mG irA | ACAAGAAAGGGAUCUGUGA |
| 314 | EV0088B | mA mG mG mG mA mU fC fU fG mU mG mU mG mG mC mA mG mA irA | AGGGAUCUGUGUGGCAGAA |
| 315 | EV0089B | mA mA mU mG mC mA fG fG fA mC mU mU mC mU mU mC mA mU irA | AAUGCAGGACUUCUUCAUA |
| 316 | EV0090B | mC mC mA mG mA mA fU fU fC mU mC mC mU mG mC mA mA mG irA | CCAGAAUUCUCCUGCAAGA |
| 317 | EV0091B | mU mG mG mU mC mA fA fG fG mU mC mU mU mC mU mC mU mC irA | UGGUCAAGGUCUUCUCUCA |
| 318 | EV0092B | mA mC mC mU mC mA fU fC fG mC mC mA mU mC mG mA mC mU irA | ACCUCAUCGCCAUCGACUA |
| 319 | EV0093B | mC mC mU mU mC mA fU fG fG mU mG mU mU mC mC mA mA mG irA | CCUUCAUGGUGUUCCAAGA |
| 320 | EV0094B | mA mA mG mA mG mA fC fC fA mA mG mG mA mA mA mA mU mG irA | AAGAGACCAAGGAAAAUGA |
| 321 | EV0095B | mU mG mG mC mU mU fU fG fC mU mC mC mA mG mA mC mA mC irA | UGGCUUUGCUCCAGACACA |
| 322 | EV0096B | mA mC mA mC mC mC fU fC fA mU mC mA mU mC mU mA mC mC irA | ACACCCUCAUCAUCUACCA |
| 323 | EV0097B | mA mG mG mU mC mU fA fC fG mC mC mU mA mU mU mA mC mA irA | AGGUCUACGCCUAUUACAA |
| 324 | EV0098B | mG mG mU mC mU mA fC fG fC mC mU mA mU mU mA mC mA mA irA | GGUCUACGCCUAUUACAAA |
| 325 | EV0099B | mG mA mC mU mA mU fG fU fG mU mA mC mA mA mG mA mC mC irA | GACUAUGUGUACAAGACCA |
| 326 | EV0100B | mU mC mA mU mC mA fG fC fC mC mC mA mU mC mA mA mG mU irA | UCAUCAGCCCCAUCAAGUA |
| 327 | EV0101A | mA (ps) fG (ps) mG fA mA fG mU fU mG fA mC fG mU fU mG fA mG (ps) fG (ps) mG | AGGAAGUUGACGUUGAGGG |
| 328 | EV0101 B | [ST23(ps)]3 ST43(ps) fC mC fC mU fC mA fA mC fG mU fC mA fA mC fU mU fC (ps) mC (ps) fU | CCCUCAACGUCAACUUCCU |
| 329 | EV0102A | mA (ps) fA (ps) mU fA mU fA mU fU mC fA mU fG mA fG mC fU mU (ps) fC (ps) mG | AAUAUAUUCAUGAGCUUCG |
| 330 | EV0102B | [ST23(ps)]3 ST43(ps) fC mG fA mA fG mC fU mC fA mU fG mA fA mU fA mU fA (ps) mU (ps) fU | CGAAGCUCAUGAAUAUAUU |
| 331 | EV0103A | mA (ps) fU (ps) mG fA mU fG mA fG mG fG mU fG mU fU mC fC mU (ps) fA (ps) mU | AUGAUGAGGGUGUUCCUAU |
| 332 | EV0103B | [ST23(ps)]3 ST43(ps) fA mU fA mG fG mA fA mC fA mC fC mC fU mC fA mU fC (ps) mA (ps) fU | AUAGGAACACCCUCAUCAU |
| 333 | EV0104A | mU (ps) fC (ps) mU fG mU fC mU fG mG fA mU fG mA fA mG fA mG (ps) fG (ps) mU | UCUGUCUGGAUGAAGAGGU |
| 334 | EV0104B | [ST23(ps)]3 ST43(ps) fA mC fC mU fC mU fU mC fA mU fC mC fA mG fA mC fA (ps) mG (ps) fA | ACCUCUUCAUCCAGACAGA |
| 335 | EV0105A | mG (ps) fU (ps) mA fG mA fU mG fG mU fC mU fU mG fU mC fU mG (ps) fU (ps) mC | GUAGAUGGUCUUGUCUGUC |
| 336 | EV0105B | [ST23(ps)]3 ST43(ps) fG mA fC mA fG mA fC mA fA mG fA mC fC mA fU mC fU (ps) mA (ps) fC | GACAGACAAGACCAUCUAC |
| 337 | EV0106A | mG (ps) fA (ps) mA fC mA fC mC fA mU fG mA fG mG fU mC fA mA (ps) fA (ps) mG | GAACACCAUGAGGUCAAAG |
| 338 | EV0106B | [ST23(ps)]3 ST43(ps) fC mU fU mU fG mA fC mC fU mC fA mU fG mG fU mG fU (ps) mU (ps) fC | CUUUGACCUCAUGGUGUUC |
| 339 | EV0107A | mA (ps) fG (ps) mA fG mA fG mA fA mG fA mC fC mU fU mG fA mC (ps) fC (ps) mA | AGAGAGAAGACCUUGACCA |
| 340 | EV0107B | [ST23(ps)]3 ST43(ps) mU fG mG fU mC fA mA fG mG fU mC fU mU fC mU fC mU (ps) fC (ps) mU | UGGUCAAGGUCUUCUCUCU |
| 341 | EV0108A | mC (ps) fU (ps) mU fG mU fC mU fG mU fC mU fG mG fA mU fG mA (ps) fA (ps) mG | CUUGUCUGUCUGGAUGAAG |
| 342 | EV0108B | [ST23(ps)]3 ST43(ps) mC mU mU mC mA mU fC fC fA mG mA mC mA mG mA mC mA mA irA | CUUCAUCCAGACAGACAAA |
| 343 | EV0109A | mG (ps) fU (ps) mA fG mA fU mG fG mU fC mU fU mG fU mC fU mG (ps) fU (ps) mC | GUAGAUGGUCUUGUCUGUC |
| 344 | EV0109B | [ST23(ps)]3 ST43(ps) mG mA mC mA mG mA fC fA fA mG mA mC mC mA mU mC mU mA irA | GACAGACAAGACCAUCUAA |
| 345 | EV0110A | mU (ps) fC (ps) mU fU mG fG mU fG mA fA mG fU mG fG mA fU mC (ps) fU (ps) mG | UCUUGGUGAAGUGGAUCUG |
| 346 | EV0110B | [ST23(ps)]3 ST43(ps) mC mA mG mA mU mC fC fA fC mU mU mC mA mC mC mA mA mG irA | CAGAUCCACUUCACCAAGA |
| 347 | EV0111A | mG (ps) fU (ps) mU fG mU fA mA fU mA fG mG fC mG fU mA fG mA (ps) fC (ps) mC | GUUGUAAUAGGCGUAGACC |
| 348 | EV0111B | [ST23(ps)]3 ST43(ps) mG mG mU mC mU mA fC fG fC mC mU mA mU mU mA mC mA mA irA | GGUCUACGCCUAUUACAAA |
| 349 | EV0312A | mA (ps) fU (ps) mU fG mU fA mA fU mA fG mG fC mG fU mA fG mA (ps) fC (ps) mC | AUUGUAAUAGGCGUAGACC |
| 350 | EV0112B | [ST23(ps)]3 ST43(ps) fG mG fU mC fU mA fC mG fC mC fU mA fU mU fA mC fA (ps) mA (ps) fU | GGUCUACGCCUAUUACAAU |
| 351 | EV0313A | mA (ps) fU (ps) mG fU mA fG mA fU mG fG mU fC mU fU mG fU mC (ps) fU (ps) mG | AUGUAGAUGGUCUUGUCUG |
| 352 | EV0313B | [ST23(ps)]3 ST43(ps) fC mA fG mA fC mA fA mG fA mC fC mA fU mC fU mA fC (ps) mA (ps) fU | CAGACAAGACCAUCUACAU |
| 353 | EV0201 A | mU (ps) fG (ps) mA fG mA fG mA fA mG fA mC fC mU fU mG fA mC (ps) fC (ps) mA | UGAGAGAAGACCUUGACCA |
| 354 | EV0201B | [ST23(ps)]3 ST43(ps) mU mG mG mU mC mA fA fG fG mU mC mU mU mC mU mC mU (ps) mC (ps) mU | UGGUCAAGGUCUUCUCUCU |
| 355 | EV0202B | Ser(GN) (ps) mU (ps) mG (ps) mG mU mC mA fA fG fG mU mC mU mU mC mU mC mU (ps) mC (ps) mU (ps) Ser(GN) | UGGUCAAGGUCUUCUCUCU |
| 356 | EV0203A | (vp)-mU fG mA fG mA fG mA fA mG fA mC fC mU fU mG fA mC (ps) fC (ps) mA | UGAGAGAAGACCUUGACCA |
| 357 | EV0205B | [ST23(ps)]3 ST43(ps) mC mA mG mA mU mC fC fA fC mU mU mC mA mC mC mA mA (ps) mG (ps) mA | CAGAUCCACUUCACCAAGA |
| 358 | EV0206B | Ser(GN) (ps) mC (ps) mA (ps) mG mA mU mC fC fA fC mU mU mC mA mC mC mA mA (ps) mG (ps) mA (ps) Ser(GN) | CAGAUCCACUUCACCAAGA |
| 359 | EV0207A | (vp)-mU (ps) fC (ps) mU fU mG fG mU fG mA fA mG fU mG fG mA fU mC (ps) fU (ps) mG | UCUUGGUGAAGUGGAUCUG |
| 360 | EV0209A | (vp)-mU fC mU fU mG fG mU fG mA fA mG fU mG fG mA fU mC (ps) fU (ps) mG | UCUUGGUGAAGUGGAUCUG |
| 361 | EV0201Aun | UGAGAGAAGACCUUGACCA | UGAGAGAAGACCUUGACCA |

## Claims

1. A double-stranded nucleic acid for inhibiting expression of complement component C3, wherein the nucleic acid comprises a first strand and a second strand, wherein the first strand sequence comprises a sequence of at least 15 nucleotides differing by no more than 3 nucleotides from any one of the sequences SEQ ID NO: 361, 95, 111, 125, 131, 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 97, 99, 101, 103, 105, 107, 109, 113, 115, 117, 119, 121, 123, 127, 129 or 133.

2. A double-stranded nucleic acid that is capable of inhibiting expression of complement component C3 for use as a medicament.

3. The nucleic acid of any of the preceding claims, wherein the first strand and the second strand form a duplex region from 17-25 nucleotides in length.

4. The nucleic acid of any of the preceding claims, wherein the nucleic acid mediates RNA interference.

5. The nucleic acid of any of the preceding claims, wherein at least one nucleotide of the first and/or second strand is a modified nucleotide, preferably a non-naturally occurring nucleotide such as a 2'-F modified nucleotide.

6. The nucleic acid of any of the preceding claims, wherein at least nucleotides 2 and 14 of the first strand are modified by a first modification, the nucleotides being numbered consecutively starting with nucleotide number 1 at the 5' end of the first strand.

7. The nucleic acid of any of the previous claims, wherein the first strand has a terminal 5' (E)-vinylphosphonate nucleotide at its 5' end.

8. The nucleic acid of any of the preceding claims, wherein the nucleic acid comprises a phosphorothioate linkage between the terminal two or three 3' nucleotides and/or 5' nucleotides of the first and/or the second strand and preferably wherein the linkages between the remaining nucleotides are phosphodiester linkages.

9. The nucleic acid of any of the preceding claims, comprising a phosphorodithioate linkage between each of the two, three or four terminal nucleotides at the 3' end of the first strand and/or comprising a phosphorodithioate linkage between each of the two, three or four terminal nucleotides at the 3' end of the second strand and/or a phosphorodithioate linkage between each of the two, three or four terminal nucleotides at the 5' end of the second strand and comprising a linkage other than a phosphorodithioate linkage between the two, three or four terminal nucleotides at the 5' end of the first strand.

10. The nucleic acid of any of the preceding claims, wherein the nucleic acid is conjugated to a ligand.

11. The nucleic acid of claim 10, wherein the ligand comprises (i) one or more N-acetyl galactosamine (GalNAc) moieties or derivatives thereof, and (ii) a linker, wherein the linker conjugates the at least one GalNAc moiety or derivative thereof to the nucleic acid.

12. A composition comprising a nucleic acid of any of the previous claims and a delivery vehicle and/or a physiologically acceptable excipient and/or a carrier and/or a diluent and/or a buffer and/or a preservative and/or a further therapeutic agent selected from the group comprising an oligonucleotide, a small molecule, a monoclonal antibody, a polyclonal antibody and a peptide.

13. A nucleic acid of any of claims 1 and 3-11 or a composition of claim 12 for use as a medicament.

14. A nucleic acid of any of claims 1 and 3-11 or a composition of claim 12 for use in the prevention, decrease of the risk of suffering from, or treatment of a disease, disorder or syndrome, wherein the disease, disorder or syndrome is preferably a complement-mediated disease, disorder or syndrome.

15. Use of a nucleic acid of any of claims 1 and 3-11 or a composition of claim 12 in the prevention, decrease of the risk of suffering from, or treatment of a disease, disorder or syndrome, wherein the disease, disorder or syndrome is preferably C3 Glomerulopathy (C3G).
